# EUROPEAN PATENT APPLICATION

(11) **EP 4 062 942 A1**
(43) Date of publication of application: **28.09.2022**
(21) Application number: 21163944.8
(22) Date of filing: 22.03.2021
(51) Int. Cl.: A61K 47/54, A61K 47/55, C07D 275/04, A61P 35/00, A61P 37/00

(54) **DISULFIDE-BASED PRODRUG COMPOUNDS**

(71) Applicant: Ludwig-Maximilians-Universität München, 80539 München (DE)
(72) Inventor: Thorn-Seshold, Oliver, 81371 München (DE); Thorn-Seshold, Julia, 81371 München (DE); Felber, Jan, 80337 München (DE); Zeisel, Lukas, 81373 München (DE)
(74) Representative: Vossius & Partner Patentanwälte Rechtsanwälte mbB

(57) **Abstract**

The present application relates to a compound having the formula (I)

A-L-B (I)

wherein
A is represented by 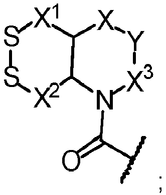 L is a bond or a self-immolative spacer;
B is represented by

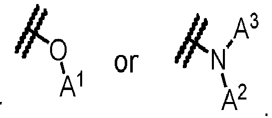

The compound is capable of releasing molecular cargo in the presence of a reductase and is thus suitable for treating, ameliorating, preventing or diagnosing a disorder selected from a neoplastic disorder; atherosclerosis; an autoimmune disorder; an inflammatory disease; a chronic inflammatory autoimmune disease; ischaemia; and reperfusion injury.

## Description

### Technical Field

The present application relates to a compound having the formula (I). The compound having the formula (I) is capable of releasing a molecular cargo in the presence of a reductant and is thus suitable for treating, ameliorating, preventing or diagnosing a disorder selected from a neoplastic disorder; atherosclerosis; an autoimmune disorder; an inflammatory disease; a chronic inflammatory autoimmune disease; ischaemia; and reperfusion injury.

In particular, the molecular cargo can be a therapeutic or diagnostic agent. In particular, the reductant can be a biological disulfide reductase or its effector disulfide protein which may be catalytically active. The invention further relates to a pharmaceutical or diagnostic composition comprising the same.

### Prior Art

Dithiol/disulfide-exchange redox reactions are critical in a great number of biological pathways. Often, these are coordinated through conserved, specialised networks of oxidoreductases that perform redox reactions upon or using disulfides and/or thiols. The thioredoxin reductase - thioredoxin (TrxR-Trx) system, and the glutathione reductase - glutathione - glutaredoxin (GR-GSH-Grx) system, are two of the central redox systems; other key reductive enzymes/proteins include but are not limited to peroxiredoxins, glutathione peroxidases, glutathione-S-transferases, methionine sulfoxide reductase, and protein disulfide isomerases (PDIs) (Lee, S. et al. Antioxid Redox Signal 2013, 18, 1165-1207. https://doi.org/10.1089/ars.2011.4322). Such redox systems drive reactions vital to cellular metabolism, and can also regulate protein activity, protein-protein interactions, and protein localisation by reversible dithiol/disulfide-type reactions (Jones, D. P. et al. Antioxidants & Redox Signaling 2015, 23, 734-746. https://doi.org/10.1089/ars.2015.6247).

To better investigate and understand biological processes, it is of great interest to develop specific methods to image or quantify the activity of biological species, or otherwise to respond to their activity. Therefore it was an object of the present invention to provide compounds which have high selectivity for an oxidoreductase or its redox effector protein and which can be reduced by the disulfide reductive activity of that enzyme and thus release a molecular cargo. This has been a longstanding goal of research in the field of cellular dithiol/disulfide-exchange redox reactions. In this field, a significant challenge for chemical probe development is to ensure that a probe is specific for being reduced by the targeted species, and is not significantly reduced by any other reducing species in the cell. Therefore, to selectively probe one of the species TrxR, GR, Trx, or Grx, a compound must not be activated by any of the others or by GSH. This is difficult because these reductants can perform similar chemical reactions, and because the most active of these reductants have the lowest cellular concentrations (TrxR and GR have nM cellular concentrations; Trx and Grx have µM concentrations) while the least active reductant GSH is the most concentrated (mM).

These reducing species all act upon disulfides, so most reduction-activated chemical probes in the prior art have used disulfides as reduction-sensing motifs. Probes based on linear disulfides (such as E1) have been extensively reported, but it has been shown since decades that these disulfides are nonspecifically reduced by thiols including by GSH (Lim, C. S. et al. J. Am. Chem. Soc. 2011, 133, 11132-11135. https://doi.org/10.1021/ja205081s; Butora, G. et al. Angewandte Chemie International Edition 2014, 53, 14046-14050. https://doi.org/10.1002/anie.201407130). Therefore, linear disulfides are not suitable as selective probes in a cellular context.

Recently, there has been limited development of probes using cyclic disulfides as reduction-sensing motifs. For example, Fang and coworkers have developed several so-called "TRFS" probes and prodrugs (such as **E2;** Zhang, L. et al. J. Am. Chem. Soc. 2014, 136, 226-233. https://doi.org/10.1021/ja408792k) based on a cyclic five-membered disulfide motif; most of these release a cargo after reduction of the disulfide. However, it has been shown that this type of disulfide too is nonspecifically reduced by monothiols including by GSH, and therefore that five-membered cyclic disulfides are not suitable as the basis for oxidoreductase-selective probes in a cellular context (Felber, J. et al. ChemRxiv 2020. https://doi.org/10.26434/chemrxiv. 13483155.v1).

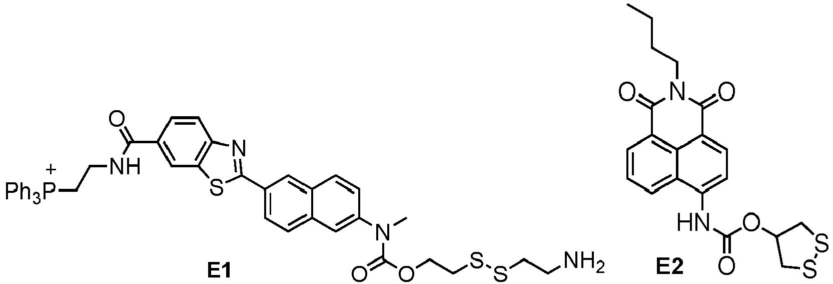

As far as we are aware, only three concepts have been disclosed of probe or prodrug designs based on cyclic six-membered disulfides that are intended to release a general molecular cargo after reduction inside cells.

Firstly, in 2014, Butora disclosed a six-membered cyclic disulfide **E3** capable of releasing a phosphate molecular cargo (R'O)(RO)P(O)OH after reduction. **E3** was applied to cells, and the report stated that release was effected by intracellular GSH (Butora, G. et al. Angewandte Chemie International Edition 2014, 53, 14046-14050. https://doi.org/10.1002/anie.201407130; WO 2014/088923). The approach has since been used by e.g. Hayashi in 2017 (Hayashi, J. et al. Bioorganic & Medicinal Chemistry Letters 2017, 27, 3135-3138. https://doi.org/10.1016/j.bmcl.2017.05.031).

Secondly, in 2017, Kong et al. reported a six-membered cyclic disulfide **E4** intended to release an aniline molecular cargo after reduction. **E4** was applied to cells, and the report stated that release was effected by intracellular H₂Se (Kong, F. et al. Anal. Chem. 2017, 89, 688-693. https://doi.org/10.1021/acs.analchem.6b03136). A similar concept was used by Li et al. in 2019 in a six-membered cyclic disulfide probe intended to release a different aniline cargo (molecule TRFS7 in their report) but the report stated that "the 1,2-dithianes cannot be reduced by either TrxR or GSH"; a related six-membered cyclic disulfide probe (molecule TRFS8 in their report) featuring a urea linkage was not intended to release a molecular cargo and indeed did not release it (Li, X. et al. Nature Communications 2019, 10, 2745. https://doi.org/10.1038/s41467-019-10807-8).

Thirdly, in 2017, Ziv disclosed prodrugs where a 4-amino-1,2-dithiane is linked to a phenolic molecular cargo as the carbamate, so as to release the phenol following reduction which was ascribed to occur through the "ambient reductive environment" of the cell although no cellular reductive species were demonstrated. An example of this is shown as molecule **E5** (WO 2017,017669; US 9,687,556). Somewhat relatedly, Kohn has worked on six-membered cyclic disulfide derivatives, although not intended to release a cargo. In 2004, Kohn published the molecule **E6** (Lee, S. H. et al. J. Am. Chem. Soc. 2004, 126, 4281-4292. https://doi.org/10.1021/ja030577r). In this molecule three of the atoms in the piperazine-like ring that is annelated to the cyclic disulfide are sp²-configured, which rigidifies the system and prevents it from adopting a decalin-like geometry that would stabilise the disulfide. Notably, **E6** was reported to be transthiolated by "an intracellular thiol or albumin" (i.e. by a monothiol) then to subsequently undergo a cyclisation that results in the attached mitomycin moiety becoming reactive. In 2005, Lee et al. published a related mitomycin derivative containing a six-membered cyclic disulfide that likewise did not release a molecular cargo after activation (Lee, S. H. et al. Org. Biomol. Chem. 2005, 3, 471-482. https://doi.org/10.1039/B414806A).

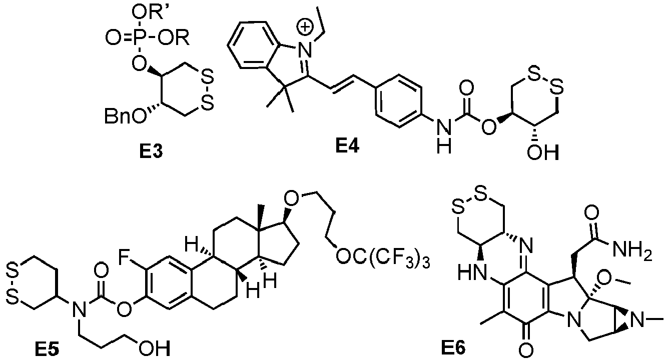

Therefore the relevant prior art in disulfides capable of releasing a molecular cargo after reduction contains (i) linear and cyclic five-membered disulfide designs that are unstable to monothiols such as GSH and therefore in a cellular context cannot be selective for a particular reductant; and (ii) monocyclic six-membered disulfide designs such as **E3, E4,** and **E5,** for which the literature is contradictory about what the relevant cellular reductant is (suggesting it is respectively GSH, H₂Se, or an undefined ambient reductive environment, or alternatively stating that GSH cannot reduce the cyclic six-membered disulfide). The example of the geometrically strained six-membered disulfide **E6,** or the strained cyclic five-membered disulfides such as **E2** (discussed in Felber, J. et al. ChemRxiv 2020. https://doi.org/10.26434/chemrxiv.13483155.v1) shows that destabilisation of the disulfide enhances its reactivity to reductants, which makes the disulfides sensitive to monothiols including GSH (and therefore in a cellular context they cannot be selective for a particular reductant).

Therefore there remain numerous unsolved challenges, including to: (i) design cyclic disulfide-based probe or prodrug systems that release a molecular cargo and that feature optimised disulfide ring geometries that stabilise the disulfide; and to (ii) design disulfide-based probe or prodrug systems that release a molecular cargo and that are selective for a particular cellular reductant, i.e. are shown to resist reduction by other major cellular reductants (e.g. GSH, TrxR, Trx, Grx, GR etc as appropriate).

Under pathological conditions, homeostasis in these redox systems and in their target proteins is significantly dysregulated. This has been particularly shown in diseases such as cancer, inflammatory diseases such as autoimmune disorders, under acute stress such as reperfusion injury, and under other chronic conditions such as cardiovascular disease (Mohammadi, F. et al. Cancer Chemotherapy and Pharmacology 2019. https://doi.org/10.1007/s00280-019-03912-4, Whayne, T. F. et al. Can. J. Physiol. Pharmacol. 2015, 93, 903-911. https://doi.org/10.1139/cjpp-2015-0105). Such dysregulation has also been studied in the context of disease-associated biomarkers, such as hypoxia. Hypoxia is a pathology-associated feature, prevalent in cancer and inflammation, that is tightly correlated with significant biochemical changes including oxidoreductase dysregulation, many of which rely on the hypoxia-dependent transcription factor HIF-1. Oxidoreductase dysregulation may be reflected in a combination of changes to their expression level, enzymatic activity, redox poise (the ratio of oxidised to reduced form of the oxidoreductase), localisation, and/or other parameters. For example, under pathological conditions, the TrxR-Trx and GR-GSH-Grx systems act as repair systems, counteracting acute cellular damage by oxidants, and re-normalising redox imbalances e.g. from oxygen depletion (hypoxia) or from the switching of metabolic pathways due to cellular stress (e.g. the Warburg Effect in cancer; Arnér, E. S. J. et al. Seminars in Cancer Biology 2006, 16, 420-426. https://doi.org/10.1016/j.semcancer.2006.10.009, Karlenius, T. C. et al. Cancers 2010, 2, 209-232. https://doi.org/10.3390/cancers2020209); therefore cells affected by pathologies that require such repair and renormalisation are often found to upregulate expression levels and activity of these oxidoreductase systems, and shift their redox poise (Jiang, J. et al. Nanoscale 2014, 6, 12104-12110. https://doi.org/10.1039/C4NR01263A). This dysregulation has been particularly studied in the context of cancer, where for the example of Trx it has been shown that cycling hypoxia, which is a hallmark of most tumors, upregulates Trx expression levels (Karlenius, T. C. et al. Biochemical and Biophysical Research Communications 2012, 419, 350-355. https://doi.org/10.1016/j.bbrc.2012.02.027); that Trx overexpression contributes to hallmarks of cancer including increased proliferation and angiogenesis, and evasion of apoptosis (Powis, G. et al. Current Opinion in Pharmacology 2007, 7, 392-397. https://doi.org/10.1016/j.coph.2007.04.003); and that Trx overexpression in tumors contributes to chemoresistance and is associated to poor patient survival (Biaglow, J. E. et al. Cancer Biology & Therapy 2005, 4, 13-20. https://doi.org/10.4161/cbt.4.1.1434). Dysregulated function is also particularly studied in the context of inflammatory diseases, since many central players in inflammation (NFκB, TNF-α, Keap1, Nrf2) are regulated by these oxidoreductases, and processes from inflammasome activation to immune cell chemotaxis have also been shown to depend on these oxidoreductases (Bertini, R. et al. J Exp Med 1999, 189, 1783-1789. https:Hdoi.org/10.1084/jem.189.11.1783, Yoshihara, E. et al. Front Immunol 2014, 4, 514-514. https://doi.org/10.3389/fimmu.2013.00514).

The disease-correlated nature of these dysregulated states of oxidoreductases - particularly of Trx, TrxR, and PDIs - makes them promising targets for diagnostic or therapeutic applications using diagnostic or therapeutic drugs that target these oxidoreductases. For example, the TrxR-inhibiting drug auranofin is FDA-approved for use in the inflammatory disease, rheumatoid arthritis (Lee, S. et al. Antioxid Redox Signal 2013, 18, 1165-1207. https://doi.org/10.1089/ars.2011.4322; Arnér, E. S. J. et al. Seminars in Cancer Biology 2006, 16, 420-426. https://doi.org/10.1016/j.semcancer.2006.10.009). In this context, a probe or prodrug system to release a molecular cargo, that is selectively triggered by one of these oxidoreductases, would be very valuable. Similarly, substantial efforts have been made to use the disease-correlated feature, hypoxia, both as a diagnostic marker and for therapeutic exploitation, for example by using diagnostic and therapeutic drugs that target hypoxia, which has been extensively reviewed (Airley, R. et al. The Pharmaceutical Journal 2000, 264, 666-673; Phillips, R. M. Cancer Chemotherapy and Pharmacology 2016, 77, 441-457. https://doi.org/10.1007/s00280-015-2920-7).

Therefore it was a further object of the present invention to provide compounds which may have high selectivity for a oxidoreductase in a pathologically dysregulated state, and can be reduced by the disulfide reductive activity of that oxidoreductase or its effector proteins and thus release a molecular cargo after reduction. It was a further object of the present invention to provide compounds which may have high selectivity for pathologically dysregulated redox states and/or for cells in hypoxia and/or cells with an activated hypoxic response pathway.

It was a further object of the present invention to provide compounds which are also useful, e.g. in a diagnostic or therapeutic capacity, in that they are efficiently activated upon delivery into cells so releasing a molecular cargo, in which case the delivery into the cell is intended to be the process that gives selectivity for the targeted cells or tissues.

In conclusion therefore, the objects of the present invention were provide compounds which are capable of releasing a molecular cargo after reductive activation, and which
(A) are suitable for diagnosing, quantifying, or responding to disulfide reductive activity; and/or
(B) are suitable for diagnosing, treating, ameliorating or preventing a disorder associated with disulfide reductive activity; and/or
(C) are suitable for diagnosing, treating, ameliorating or preventing a disorder selected from a neoplastic disorder; atherosclerosis; an autoimmune disorder; an inflammatory disease; a chronic inflammatory autoimmune disease; ischaemia; and reperfusion injury, particularly cancer; and/or
(D) are suitable for diagnosing, treating, ameliorating or preventing a disorder associated with a dysregulated redox state and/or with hypoxia and/or with activation of the hypoxic response pathway; and/or
(E) are useful because they are efficiently activated upon delivery into cells.
Note that it is possible, and in many cases it is desirable, that one or more of these objectives should be fulfilled by the same compound; for example, a single compound of the invention may fulfil objectives A and E simultaneously; while another compound of the invention may fulfil objectives A, B, C and D simultaneously, etc.

### Summary of the Invention

The present invention relates to the following items:
1. A compound having the formula (I)

   A-L-B (I)

   wherein
   A is represented by 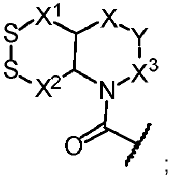 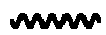 denotes the attachment point of A to L;
   L is a bond or a self-immolative spacer;
   B is represented by 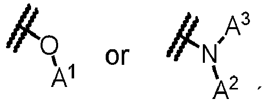 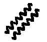 denotes the attachment point of B to L;
   A¹ is selected such that A¹-OH is a therapeutic, diagnostic or theranostic agent which contains an -OH group that is attached to a 5- or 6-membered aromatic or heteroaromatic ring;
   A² and A³ are independently selected such that A²-NH-A³ is a therapeutic, diagnostic or theranostic agent which contains an -NH₂ or -NH- moiety;
   X is selected from -N(R^{a})-, -N(R^{b})- and -CR^{c}₂-;
   X¹ is -(CR^{d}₂)ₘ-;
   X² is -(CR^{e}₂)ₙ-;
   X³ is -CR^{f}₂-;
   Y is -(CR^{g}₂)ₚ-;
   W is independently selected from -OH, -N(R^{h})(Rⁱ), and a heterocyclic group selected from azetidinyl, pyrrolidinyl, piperidinyl or morpholino, wherein the heterocyclic group is attached to the -C₁₋₄-alkyl or -(C₂₋₄-alkylene)- group via the N atom;
   R^{a} is selected from -H, -C₁₋₄-alkyl, -C(O)-C₁₋₄-alkyl, -C(O)-O-C₁₋₄-alkyl, -C(O)-N(C₁₋₄-alkyl)₂, -S(O)₂-C₁₋₄-alkyl, and -(C₂₋₄-alkylene)-O-(C₁₋₄-alkyl), wherein -C₁₋₄-alkyl or -(C₂₋₄-alkylene)- can be optionally substituted by W;
   R^{b} is an acyl group of a monopeptide selected from -proteinogenic amino acids attached via a carboxy group;
   R^{c} groups are independently selected from -H and -C₁₋₄-alkyl;
   R^{d} groups are independently selected from -H and -C₁₋₄-alkyl;
   R^{e} groups are independently selected from -H and -C₁₋₄-alkyl;
   R^{f} groups are independently selected from -H and -C₁₋₄-alkyl;
   R^{g} groups are independently selected from -H and -C₁₋₄-alkyl;
   R^{h} is independently selected from -H, -C₁₋₄-alkyl and -CH₂CH₂OH;
   Rⁱ is independently selected from -H, -C₁₋₄-alkyl and -CH₂CH₂OH;
   m is 0, 1 or 2;
   n is 1 or 2, provided that m+n is 2 or 3;
   p is 0, 1, or 2, provided that when X = -N(R^{a})- or -N(R^{b})- then p = 1 or 2; or any pharmaceutically acceptable salt, solvate or ester thereof.
2. The compound according to item 1, wherein L is a bond.
3. The compound according to item 1, wherein L is a self-immolative spacer selected from 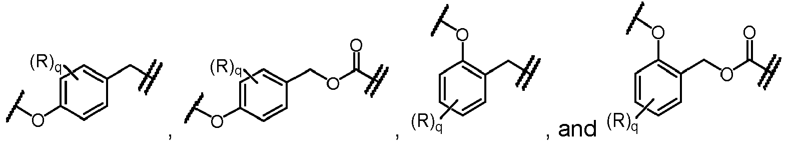 wherein
   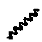 denotes the attachment point to A;
   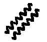 denotes the attachment point to B;
   R is independently selected from halogen, -O(R^{r}), -N(R^{s})(R^{t}), -NO₂, -CN, and a heterocyclic group selected from azetidinyl, pyrrolidinyl, piperidinyl or morpholino, wherein the heterocyclic group is attached to the phenyl ring *via* the N atom;
   q is 0, 1, 2, 3 or 4;
   R^{r} is independently selected from -H, -C₁₋₄-alkyl and -(C₂₋₄-alkylene)-O-(C₁₋₄-alkyl), wherein -C₁₋₄-alkyl or -(C₂₋₄-alkylene)- can be optionally substituted by W;
   R^{s} is independently selected from -H, -C(O)-C₁₋₄-alkyl and -C₁₋₄-alkyl; and
   R^{t} is independently selected from -H, -C(O)-C₁₋₄-alkyl and -C₁₋₄-alkyl.
4. The compound according to item 3, wherein L is a self-immolative spacer selected from 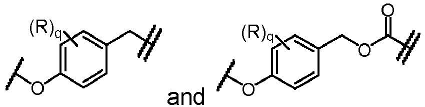 wherein R and q are as defined in item 3.
5. The compound according to any one of items 1 to 4, wherein X¹ and X² are -CH₂-.
6. The compound according to any one of items 1 to 5, wherein A¹-OH or A²-NH-A³ is selected from a diagnostically acceptable dye, a therapeutically acceptable DNA-alkylating agent, a therapeutically acceptable tubulin-inhibiting agent, and a therapeutically acceptable DNA-intercalating agent.
7. The compound according to any one of items 1 to 6, wherein A¹-OH or A²-NH-A³ is selected from 10-hydroxycamptothecin, 10-hydroxybelotecan, 10-hydroxygimatecan, 10-hydroxy-CKD-602, 10-hydroxy-BNP-1350, 10-hydroxy-sinotecan, topotecan, 7-ethyl-10-hydroxy-camptothecin (SN-38), 10-hydroxy-20-acetoxy-camptothecin, pyrrolobenzodiazepine, methotrexate, duocarmycin, CC-1065, doxorubicin, epirubicin, daunorubicin, pirarubicin, carminomycin, doxorubicin-*N*,*O*-acetal, 4-(bis(2-chloroethyl)amino)phenol, 4-(bis(2-bromoethyl)amino)phenol, 4-(bis(2-mesylethyl)amino)phenol, 4-((2-chloroethyl-2'-mesylethyl)amino)phenol, 5-hydroxy-seco-cyclopropabenzaindoles, 5-hydroxy-seco-(2-methyl-cyclopropa)benzaindoles, 5-hydroxy-seco-cyclopropamethoxybenzaindoles, 5-amino-seco-cyclopropabenzaindoles, etoposide, teniposide, GL331, NPF, TOP53, NK611, tubulysin A, tubulysin B, tubulysin C, tubulysin G, tubulysin I, monomethyl auristatin E, monomethyl auristatin F, dolastatin 10, dolastatin 15, symplostastin 1, symplostastin 3, narciclasine, pancratistatin, 2-epi-narciclasine, narciprimine, calicheamicin α1, calicheamicin β1, calicheamicin γ1, calicheamicin δ1, calicheamicin ε, calicheamicin θ, calicheamicin T, diclofenac, aceclofenac, mefenamic acid, clonixin, piroxicam, meloxicam, tenoxicam, lornoxicam, baricitinib, filgotinib, tofacitinib, upadacitinib, ruxolitinib, peficitinib, decemotinib, solcitinib, itacitinib, fostamatinib, SHR0302, leuco-methylene blue, leuco-methyl methylene blue, leuco-dimethyl methylene blue, leuco-toluidine blue, leuco-Azure A, leuco-Azure B, leuco-Azure C, leuco-Thionin, leuco-methylene violet, leuco-new methylene blue, leuco-Nile blue A, leuco-brilliant cresyl blue, firefly luciferin (D-Luciferin), umbelliferone, 4-trifluoromethylumbelliferone, 6,8-difluoro-4-methylumbelliferone, 7-hydroxycoumarin-3-carboxylic acid, 6,8-difluoro-7-hydroxy-5-methylcoumarin (DiFMU), 7-amino-4-methylcoumarin, 7-amino-4-chloromethylcoumarin, 3-*O*-methylfluorescein, 3-*O*-ethyl-5-carboxyfluorescein, 2,7-difluoro-3-*O*-methylfluorescein, 3-*N*-acetyl-rhodamine, 3-*N*-acetyl-dimethylsilarhodamine, 2,7-dibromo-3-*N*-acetyl-dimethylcarborhodamine, 3-*N*-acetyl-6-carboxyrhodamine, 2,7-difluoro-3-*N*-acetylrhodol, 3-O-(*N*,*N*-dimethyl-2-aminoethyl)-6-carboxyfluorescein, 2,7-dichloro-3-*O*-(*N*,*N*-dimethyl-2-aminoethyl)fluorescein, blackberry quencher (BBQ), black hole quencher 3 (BHQ3), 2-(2-hydroxyphenyl)quinazolin-4-one, 6-chloro-2-(5-chloro-2-hydroxyphenyl)quinazolin-4-one, and 6-bromo-2-(5-bromo-2-hydroxyphenyl)quinazolin-4-one.
8. A pharmaceutical or diagnostic composition comprising the compound according to any one of items 1 to 7, or a pharmaceutically acceptable salt, solvate, or ester thereof, and optionally a pharmaceutically acceptable carrier or excipient.
9. The pharmaceutical or diagnostic composition according to item 8, further comprising a second pharmaceutically active agent selected from a vascular disrupting agent, a cytotoxic chemotherapeutic agent and an immunomodulator.
10. The pharmaceutical or diagnostic composition according to item 9, wherein the second pharmaceutically active agent is selected from combretastatin A-4 (CA4), 3'-aminocombretastatin A-4, BNC105, ABT-751, ZD6126, combretastatin A-1, or prodrugs of the same (which includes but is not limited to combretastatin A-4 phosphate (CA4P), 3'-aminocombretastatin A-4 3'-serinamide (ombrabulin), combretastatin A-1 bisphosphate (CA1P), and BNC105 phosphate (BNC105P)), and pharmaceutically acceptable salts, solvates or esters of the same.
11. A compound according to any one of items 1 to 7, or a pharmaceutically acceptable salt, solvate, or ester thereof, for use in medicine.
12. A compound according to any one of items 1 to 7, or a pharmaceutically acceptable salt, solvate, or ester thereof, for use in the treatment, amelioration, prevention or diagnosis of a disorder selected from a neoplastic disorder; atherosclerosis; an autoimmune disorder; an inflammatory disease; a chronic inflammatory autoimmune disease; ischaemia; and reperfusion injury.
13. The compound for use according to item 12, wherein the neoplastic disorder is cancer which is preferably selected from acoustic neuroma, adenocarcinoma, angiosarcoma, basal cell carcinoma, bile duct carcinoma, bladder carcinoma, breast cancer, bronchogenic carcinoma, cervical cancer, chondrosarcoma, chordoma, choriocarcinoma, craniopharyngioma, cystadenocarcinoma, embryonal carcinoma, endotheliosarcoma, ependymoma, epithelial carcinoma, Ewing's tumor, fibrosarcoma, hemangioblastoma, leiomyosarcoma, liposarcoma, Merkel cell carcinoma, melanoma, mesothelioma, myelodysplastic syndrome, myxosarcoma, oligodendroglioma, osteogenic sarcoma, ovarian cancer, pancreatic cancer, papillary adenocarcinomas, papillary carcinoma, pinealoma, prostate cancer, renal cell carcinoma, retinoblastoma, rhabdomyosarcoma, sebaceous gland carcinoma, seminoma, squamous cell carcinoma, sweat gland carcinoma, synovioma, testicular tumor, Wilms' tumor, adrenocortical carcinoma, urothelial carcinoma, gallbladder cancer, parathyroid cancer, Kaposi sarcoma, colon carcinoma, gastrointestinal stromal tumor, anal cancer, rectal cancer, small intestine cancer, brain tumor, glioma, glioblastoma, astrocytoma, neuroblastoma, medullary carcinoma, medulloblastoma, meningioma, leukemias including acute myeloid leukemia, multiple myeloma, acute lymphoblastic leukemia, liver cancer including hepatoma and hepatocellular carcinoma, lung carcinoma, non-small-cell lung cancer, small cell lung carcinoma, lymphangioendotheliosarcoma, lymphangiosarcoma, primary CNS lymphoma, non-Hodgkin lymphoma, and classical Hodgkin's lymphoma, preferably colon cancer, rectal cancer, small intestine cancer, brain tumor, leukemia, liver cancer, lung cancer, lymphoma, basal cell carcinoma, breast cancer, cervical cancer, melanoma, ovarian cancer, pancreatic cancer, and squamous cell carcinoma.
14. Use of a compound according to any one of items 1 to 7, or a pharmaceutically acceptable salt, solvate, or ester thereof, for the manufacture of a medicament for the treatment, amelioration, prevention or diagnosis of a disorder selected from a neoplastic disorder; atherosclerosis; an autoimmune disorder; an inflammatory disease; a chronic inflammatory autoimmune disease; ischaemia; and reperfusion injury.
15. Use of a compound according to item 14, or a pharmaceutically acceptable salt, solvate, or ester thereof, wherein the neoplastic disorder is cancer which is preferably selected from acoustic neuroma, adenocarcinoma, angiosarcoma, basal cell carcinoma, bile duct carcinoma, bladder carcinoma, breast cancer, bronchogenic carcinoma, cervical cancer, chondrosarcoma, chordoma, choriocarcinoma, craniopharyngioma, cystadenocarcinoma, embryonal carcinoma, endotheliosarcoma, ependymoma, epithelial carcinoma, Ewing's tumor, fibrosarcoma, hemangioblastoma, leiomyosarcoma, liposarcoma, Merkel cell carcinoma, melanoma, mesothelioma, myelodysplastic syndrome, myxosarcoma, oligodendroglioma, osteogenic sarcoma, ovarian cancer, pancreatic cancer, papillary adenocarcinomas, papillary carcinoma, pinealoma, prostate cancer, renal cell carcinoma, retinoblastoma, rhabdomyosarcoma, sebaceous gland carcinoma, seminoma, squamous cell carcinoma, sweat gland carcinoma, synovioma, testicular tumor, Wilms' tumor, adrenocortical carcinoma, urothelial carcinoma, gallbladder cancer, parathyroid cancer, Kaposi sarcoma, colon carcinoma, gastrointestinal stromal tumor, anal cancer, rectal cancer, small intestine cancer, brain tumor, glioma, glioblastoma, astrocytoma, neuroblastoma, medullary carcinoma, medulloblastoma, meningioma, leukemias including acute myeloid leukemia, multiple myeloma, acute lymphoblastic leukemia, liver cancer including hepatoma and hepatocellular carcinoma, lung carcinoma, non-small-cell lung cancer, small cell lung carcinoma, lymphangioendotheliosarcoma, lymphangiosarcoma, primary CNS lymphoma, non-Hodgkin lymphoma, and classical Hodgkin's lymphoma, preferably colon cancer, rectal cancer, small intestine cancer, brain tumor, leukemia, liver cancer, lung cancer, lymphoma, basal cell carcinoma, breast cancer, cervical cancer, melanoma, ovarian cancer, pancreatic cancer, and squamous cell carcinoma.
16. A method of treating, ameliorating, preventing or diagnosing a disorder selected from a neoplastic disorder; atherosclerosis; an autoimmune disorder; an inflammatory disease; a chronic inflammatory autoimmune disease; ischaemia; and reperfusion injury, wherein an effective amount of a compound according to any one of items 1 to 7, or a pharmaceutically acceptable salt, solvate, or ester thereof, is administered to a patient in need thereof.
17. A method according to item 16, wherein the neoplastic disorder is cancer which is preferably is selected from acoustic neuroma, adenocarcinoma, angiosarcoma, basal cell carcinoma, bile duct carcinoma, bladder carcinoma, breast cancer, bronchogenic carcinoma, cervical cancer, chondrosarcoma, chordoma, choriocarcinoma, craniopharyngioma, cystadenocarcinoma, embryonal carcinoma, endotheliosarcoma, ependymoma, epithelial carcinoma, Ewing's tumor, fibrosarcoma, hemangioblastoma, leiomyosarcoma, liposarcoma, Merkel cell carcinoma, melanoma, mesothelioma, myelodysplastic syndrome, myxosarcoma, oligodendroglioma, osteogenic sarcoma, ovarian cancer, pancreatic cancer, papillary adenocarcinomas, papillary carcinoma, pinealoma, prostate cancer, renal cell carcinoma, retinoblastoma, rhabdomyosarcoma, sebaceous gland carcinoma, seminoma, squamous cell carcinoma, sweat gland carcinoma, synovioma, testicular tumor, Wilms' tumor, adrenocortical carcinoma, urothelial carcinoma, gallbladder cancer, parathyroid cancer, Kaposi sarcoma, colon carcinoma, gastrointestinal stromal tumor, anal cancer, rectal cancer, small intestine cancer, brain tumor, glioma, glioblastoma, astrocytoma, neuroblastoma, medullary carcinoma, medulloblastoma, meningioma, leukemias including acute myeloid leukemia, multiple myeloma, acute lymphoblastic leukemia, liver cancer including hepatoma and hepatocellular carcinoma, lung carcinoma, non-small-cell lung cancer, small cell lung carcinoma, lymphangioendotheliosarcoma, lymphangiosarcoma, primary CNS lymphoma, non-Hodgkin lymphoma, and classical Hodgkin's lymphoma, preferably colon cancer, rectal cancer, small intestine cancer, brain tumor, leukemia, liver cancer, lung cancer, lymphoma, basal cell carcinoma, breast cancer, cervical cancer, melanoma, ovarian cancer, pancreatic cancer, and squamous cell carcinoma.
18. A method of predicting the suitability of a compound having the formula (I) as defined in any one of items 1 to 7, or a pharmaceutically acceptable salt, solvate, or ester thereof, for treating a patient who is suffering from a disorder selected from a neoplastic disorder; atherosclerosis; an autoimmune disorder; an inflammatory disease; a chronic inflammatory autoimmune disease; ischaemia; and reperfusion injury, wherein the method comprises:
   (i) obtaining a sample from the patient;
   (ii) contacting the sample with a compound having the formula (I) as defined in any one of items 1 to 7, or a pharmaceutically acceptable salt, solvate, or ester thereof, wherein A¹ is selected such that A¹-OH is a diagnostic or theranostic agent or A² and A³ are independently selected such that A²-NH-A³ is a diagnostic or theranostic agent; and
   (iii) detecting the presence or absence of A¹-OH or A²-NH-A³.

### Description of the figures

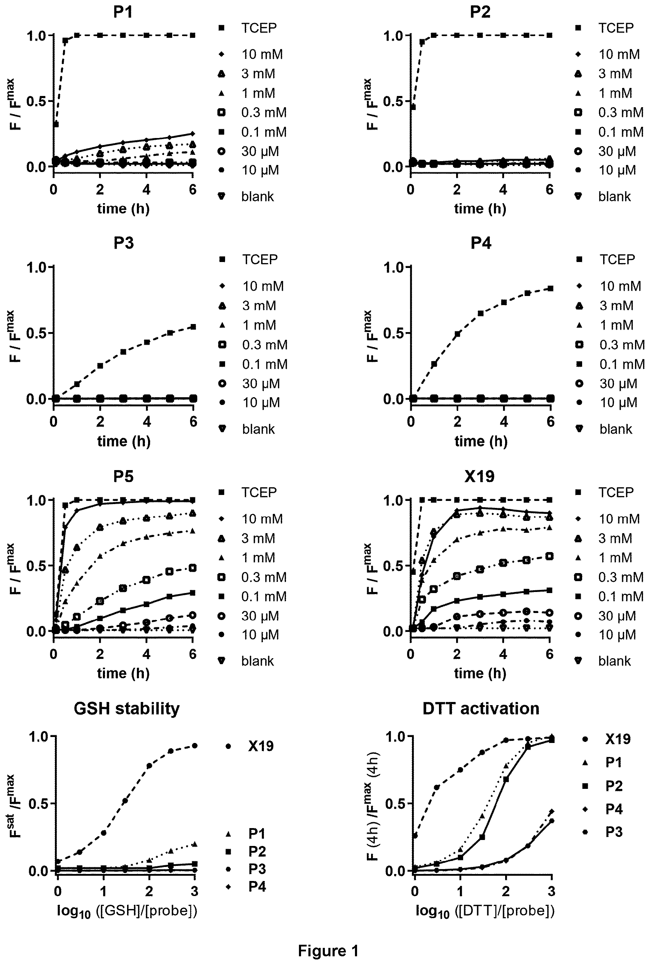

**Figure 1** shows how the compounds of the invention resist releasing their cargos when challenged with non-enzymatic reductants, evaluated in cell-free assays. The timecourse graphs show how different concentrations of the monothiol reductant glutathione (GSH) time-dependently trigger the release of fluorescent cargos **(PQ-OH** or **MF-OH),** as measured by fluorescence signal. The concentration dependence of this timecourse data is summarised in the GSH stability plot, which shows the signal generation by those probes, depending on the ratio of GSH to probe concentration (expressed as a fraction of the maximal signal F^{max}). Similar timecourse assay data for the dithiol reductant dithiothreitol (DTT), is summarised in the DTT activation plot. The results of Figure 1 show that compounds of the general formula (I) **(P1, P2, P3** and **P4)** can be stable to physiological monothiol reductants (GSH), yet can be efficiently activated by dithiol-type reductants, while probes based on prior art disulfides **(X19)** are already unstable to the monothiol GSH.

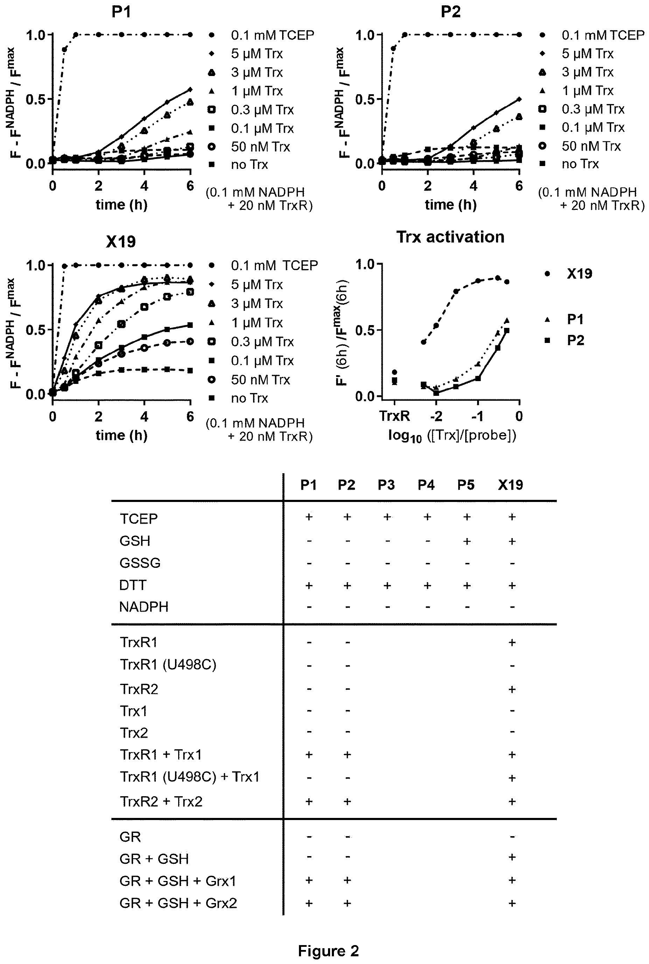

**Figure 2** shows the redox enzyme activation of compounds of the invention that release the fluorescent cargo **PQ-OH,** evaluated in cell-free assays. The timecourse graphs show that compounds of the general formula (I) **(P1** and **P2)** can release their cargo with selectivity preference for the complete protein reductant system (in this case NADPH/TrxR/Trx), in this case with high redox effector protein concentrations that may mimic upregulated expression levels, whereas prior art disulfides (as in **X19)** release their cargo with low redox effector protein concentrations and without specificity for the intact reductant systems (in this case, NADPH/TrxR). The concentration dependence of this timecourse data is summarised in the Trx activation plot, which shows the signal generation by those probes, depending on the ratio of Trx to probe concentration (expressed as a fraction of the maximal signal F^{max}). The table summarises fluorescence assay signal generation (+) or absence of signal generation (-) by various disulfide-based reduction-sensing compounds with different triggers when incubated with diverse non-enzymatic reductants and disulfide reductase systems, showing again that the compounds of the general formula (I) **(P1** and **P2)** can release their cargo with selectivity preference for complete protein reductant systems.

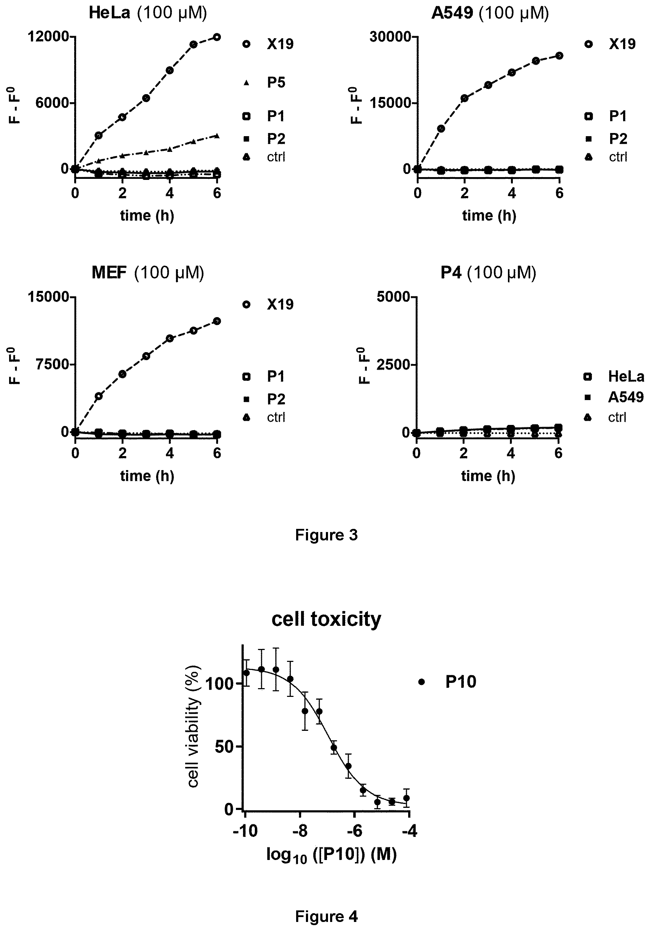

**Figure 3** shows the fluorescence signal generation by compounds of the invention that release the fluorescent cargos **PQ-OH** and **MF-OH** upon reduction, when administered to various cell lines (HeLa, A549 and MEF). The results show that compounds of the general formula (I) can be resistant against cargo release in cells growing under ordinary conditions **(P1,** and **P2),** offering the possibility that they may give selective cargo release in cells growing under pathological conditions, while probes based on prior art disulfides **(X19)** are activated to a large extent and with similar kinetics inside all cell types growing under ordinary conditions. The results also show that other compounds of the general formula (I) can be active for cargo release in cells growing under ordinary conditions **(P5).**
**Figure 4** shows results of antiproliferation assays in HeLa cells incubated with **P10,** a therapeutic compound of the general formula (I) with the same disulfide trigger motif as **P2** and **P4,** but that releases a DNA alkylator (CBI derivative) following reduction. The IC₅₀ expected for a fully-released phenolic CBI-based molecular cargo is ca. 40 pM (Jin, W. et al. J Am Chem Soc 2007, 129, 15391-15397. https://doi.org/10.1021/ja075398e), whereas **P10** has IC₅₀ ca. 200 nM, indicating that in cells under normal growth conditions **P10** only releases a small amount of the of active DNA-alkylating agent. Therefore, if the stabilised disulfide system in **P10** is activated under increasingly reductive conditions (such as by overexpression or overactivation of enzymatic reductants in redox-dysregulated tissues), its toxicity in those tissues may be substantially and selectively increased, compared to tissues with normal conditions.

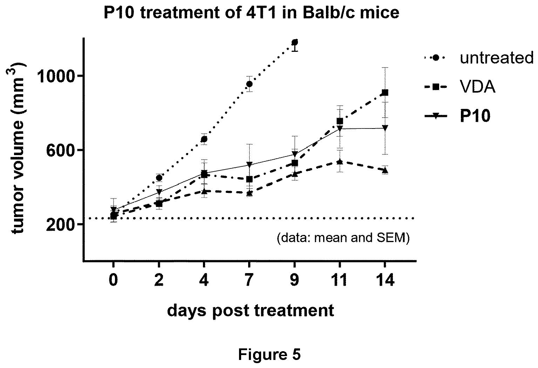

**Figure 5** shows the results of a mouse tumor treatment assay using **P10,** a therapeutic compound of the general formula (I), administered both as monotherapy and in a combination with the vascular disrupting agent (VDA) combretastatin A-4 phosphate. This shows that compounds of the general formula (I) can deliver a therapeutic effect in a disease model where dysregulated metabolism and redox conditions are expected, and that this therapeutic effect is enhanced by combination treatment designed to increase disease-localised hypoxia before the compound of the general formula (I) is applied, in order to improve the activation of the compound of the general formula (I) by pathologically-localised reductive processes.

### Definitions

Unless stated otherwise the following definitions apply.

The term **"alkyl"** refers to a saturated straight or branched hydrocarbon chain. In any of the below definitions, C₁₋₄-alkyl is not particularly limited, and refers to a saturated straight or branched hydrocarbon chain, which has 1 to 4 carbon atoms, preferably 1 to 3 carbon atoms, more preferably 1 or 2 carbon atoms, even more preferably 1 carbon atom. Preferably C₁₋₄-alkyl can be methyl, ethyl, *n*-propyl, or *i*-propyl, more preferably it can be methyl or ethyl, most preferably it can be methyl.

In any of the below definitions, C₂₋₄-**alkylene** is not particularly limited, and refers to a saturated straight or branched hydrocarbon chain, which has 2 to 4 carbon atoms, preferably 2 or 3 carbon atoms, more preferably 2 carbon atoms. Preferably C₂₋₄-alkylene can be ethylene, or n-propylene, more preferably it can be ethylene.

An **aromatic ring** is any not particularly limited and refers to any aromatic carbocyclic ring, which has 5 to 7 carbon atoms, preferably 5 or 6 carbon atoms, more preferably the aromatic ring is phenyl. The aromatic ring can optionally be annulated to one or more carbocyclic or heterocyclic rings. Examples of annulated rings include but are not limited to naphthyl, quinolyl, isoquinolyl, quinoxalyl, benzopyranonyl, benzo[1,3]dioxolyl, benzothiazolyl, 2,3-dihydro-1*H-*benzo[e]indole, xanthenyl or indolyl, preferably naphthyl, quinolyl or xanthenyl.

The **heteroaromatic ring** is any not particularly limited and refers to any aromatic heterocyclic ring, which has 5 to 7 ring atoms, preferably 5 or 6 ring atoms. The heteroaromatic ring can optionally be annulated to one more carbocyclic or heterocyclic rings. Examples of the heteroaromatic ring include thiophenyl, pyridinyl, pyrazolyl, pyrimidinyl, purinyl, pyrrolo, furanyl, oxazolyl, thiazolyl, imidazolyl, benzofuranyl, benzothiophenyl, benzothiazolyl, benzoxazolyl or naphthyridinyl, preferably pyrrolo, pyridinyl, thiophenyl or furanyl.

An **aliphatic moiety** is a saturated or unsaturated, linear, branched or cyclic moiety containing between 1 to 50 carbon atoms and optionally 0 to 19 heteroatoms, preferably 0 to 15 heteroatoms, wherein the heteroatoms are typically chosen from O, N, S, Se, Si, Hal, B or P, preferably chosen from O, N, Hal, S, Si or P, more preferably O, N, Hal, S, Si, even more preferably chosen from O, N or Hal.

A **carbocyclic ring** is a cyclic structure containing from 4 to 50 carbon atoms. Examples of the carbocyclic ring include cyclobutane, cyclopentane, cyclohexane, benzene, cycloheptane, naphthalene or anthracene, preferably cyclohexane, cyclopentane, benzene or naphthalene, more preferably benzene or naphthalene.

A **heterocyclic ring** is a cyclic structure containing at least one heteroatom selected from N, O, Si, S, B, P, and Se, and containing at least one carbon atom. Examples of the heterocyclic ring include thiophene, pyridine, pyrazole, pyrimidine, pyrrole, furan, oxazole, thiazole, imidazole, quinoline, isoquinoline, benzofuran, benzothiophene, benzothiazole, benzoxazole, naphthyridine, piperidine, piperazine, pyrrolidine, tetrahydrothiophene, tetrahydrofuran or pyran, preferably pyridine, furan, thioazole, quinoline, isoquinoline, benzothiazole, piperidine, piperazine or pyran, more preferably pyridine, quinoline, piperidine or piperazine.

**Halogen** refers to -F, -CI, -Br or -I, preferably -F or -CI.

If a moiety is referred to as being **"optionally substituted"** by a substituent it can in each instance include one or more of the indicated substituents.

**Disulfide reductase** (also referred to as disulfide oxidoreductase) refers to an enzyme capable of reducing disulfides to the corresponding thiols. Examples thereof include thioredoxin reductase 1 (TrxR1), TrxR2, thioredoxin glutathione reductase (TrxR3/TGR), endoplasmic reticulum resident protein 18 (ERp18), ERp44, ERp57, ERp72, ERdj5, methionine-R-sulfoxide reductase (MsrB1), protein disulfide isomerase A 1 (PDIA1), PDIA2, PDIA3, PDIA4, PDIA5, PDIA6, EndoPDI, PDIp, disulfide bond formation protein A (DsbA), DsbB, DsbC, or glutathione disulfide reductase (GR). A **disulfide effector protein** (or redox effector protein or simply effector protein) is a redox active protein, that bears a reducing thioredoxin-like domain and that is itself reductively enabled by an upstream disulfide oxidoreductase enzyme. Examples thereof include thioredoxin 1 (Trx1), Trx2, thioredoxin-related protein of 14 kDa (TRP14), thioredoxin-related transmembrane protein 1 (TMX1), TMX2, TMX3, TMX4, thioredoxin-like protein 1 (TXNL1), human macrothioredoxin (hMTHr), anterior gradient protein 2 (AGR2), AGR3, glutaredoxin 1 (Grx1), Grx2, Grx3, Grx4, Grx5, GrxA or Grx6.

The term **"cancer",** as used herein, refers to a group of proliferative diseases characterized by uncontrolled division of abnormal cells in a subject. Non-limiting examples of cancers include acoustic neuroma, adenocarcinoma, angiosarcoma, basal cell carcinoma, bile duct carcinoma, bladder carcinoma, breast cancer, bronchogenic carcinoma, cervical cancer, chondrosarcoma, chordoma, choriocarcinoma, craniopharyngioma, cystadenocarcinoma, embryonal carcinoma, endotheliosarcoma, ependymoma, epithelial carcinoma, Ewing's tumor, fibrosarcoma, hemangioblastoma, leiomyosarcoma, liposarcoma, Merkel cell carcinoma, melanoma, mesothelioma, myelodysplastic syndrome, myxosarcoma, oligodendroglioma, osteogenic sarcoma, ovarian cancer, pancreatic cancer, papillary adenocarcinomas, papillary carcinoma, pinealoma, prostate cancer, renal cell carcinoma, retinoblastoma, rhabdomyosarcoma, sebaceous gland carcinoma, seminoma, squamous cell carcinoma, sweat gland carcinoma, synovioma, testicular tumor, Wilms' tumor, adrenocortical carcinoma, urothelial carcinoma, gallbladder cancer, parathyroid cancer, Kaposi sarcoma, colon carcinoma, gastrointestinal stromal tumor, anal cancer, rectal cancer, small intestine cancer, brain tumor, glioma, glioblastoma, astrocytoma, neuroblastoma, medullary carcinoma, medulloblastoma, meningioma, leukemias including acute myeloid leukemia, multiple myeloma, acute lymphoblastic leukemia, liver cancer including hepatoma and hepatocellular carcinoma, lung carcinoma, non-small-cell lung cancer, small cell lung carcinoma, lymphangioendotheliosarcoma, lymphangiosarcoma, primary CNS lymphoma, non-Hodgkin lymphoma, and classical Hodgkin's lymphoma.

The term **"cargo",** as used herein, represents a therapeutic, diagnostic or theranostic agent to be delivered to a site of enzymatic activity or disease (e.g. a tumour, a site of inflammatory or autoimmune disease). Cargos used in the compounds of the invention may be small molecules. Examples include agents for treating, ameliorating, preventing or diagnosing a neoplastic disorder, an inflammatory disorder (e.g. a non-steroidal anti-inflammatory drug (NSAID), a disease-modifying anti-rheumatic drug (DMARD), or mesalamine), atherosclerosis; an autoimmune disorder; a chronic inflammatory autoimmune disorder; ischaemia; and reperfusion injury as well as kinase inhibitors.. Examples also include diagnostically acceptable dyes, therapeutically acceptable DNA-alkylating agents, therapeutically acceptable DNA-intercalating agents or therapeutically acceptable tubulin-inhibiting agents.

Non-limiting examples of **antineoplastic** agents include 10-hydroxycamptothecin, 10-hydroxybelotecan, 10-hydroxygimatecan, 10-hydroxy-CKD-602, 10-hydroxy-BNP-1350, 10-hydroxy-sinotecan, topotecan, 7-ethyl-10-hydroxy-camptothecin (SN-38), 10-hydroxy-20-acetoxy-camptothecin, pyrrolobenzodiazepine, methotrexate, duocarmycin, CC-1065, doxorubicin, epirubicin, daunorubicin, pirarubicin, carminomycin, doxorubicin-*N*,*O*-acetal, 4-(bis(2-chloroethyl)amino)phenol, 4-(bis(2-bromoethyl)amino)phenol, 4-(bis(2-mesylethyl)amino)phenol, 4-((2-chloroethyl-2'-mesylethyl)amino)phenol, 5-hydroxy-seco-cyclopropabenzaindoles, 5-hydroxy-seco-(2-methyl-cyclopropa)benzaindoles, 5-hydroxy-seco-cyclopropamethoxybenzaindoles, 5-amino-seco-cyclopropabenzaindoles, etoposide, teniposide, GL331, NPF, TOP53, NK611, tubulysin A, tubulysin B, tubulysin C, tubulysin G, tubulysin I, monomethyl auristatin E, monomethyl auristatin F, dolastatin 10, dolastatin 15, symplostastin 1, symplostastin 3, narciclasine, pancratistatin, 2-epi-narciclasine, narciprimine, calicheamicin α1, calicheamicin β1, calicheamicin y1, calicheamicin δ1, calicheamicin ε, calicheamicin θ, or calicheamicin T.

Non-limiting examples of **NSAIDs** include diclofenac, aceclofenac, mefenamic acid, clonixin, piroxicam, meloxicam, tenoxicam, or lornoxicam.

Non-limiting examples of **DMARDs** include methotrexate.

Non-limiting examples of **kinase inhibitors** include baricitinib, filgotinib, tofacitinib, upadacitinib, ruxolitinib, peficitinib, decemotinib, solcitinib, itacitinib, fostamatinib, or SHR0302. Particularly of interest are inhibitors of the Janus Kinase (JAK) family of kinases, such as of JAK1 or JAK3.

Cargos used in the compounds of the invention may be diagnostic agents (e.g., a fluorescent agent or a radiotracer agent), that may be used to detect and/or determine the stage of a targeted pathology such as a tumor, or to measure or localise redox activity. Non-limiting examples of fluorescent diagnostic agents include leuco-methylene blue, leuco-methyl methylene blue, leuco-dimethyl methylene blue, leuco toluidine blue, leuco-Azure A, leuco-Azure B, leuco-Azure C, leuco-Thionin, leuco-methylene violet, leuco-new methylene blue, leuco-Nile blue A, leuco-brilliant cresyl blue, firefly luciferin (D-Luciferin), umbelliferone, 4-trifluoromethylumbelliferone, 6,8-difluoro-4-methylumbelliferone, 7-hydroxycoumarin-3-carboxylic acid, 6,8-difluoro-7-hydroxy-5-methylcoumarin (DiFMU), 7-amino-4-methylcoumarin, 7-amino-4-chloromethylcoumarin, 3-*O*-methylfluorescein, 3-*O*-ethyl-5-carboxyfluorescein, 2,7-difluoro-3-*O*-methylfluorescein, 3-*N*-acetyl-rhodamine, 3-*N*-acetyl-dimethylsilarhodamine, 2,7-dibromo-3-*N*-acetyl-dimethylcarborhodamine, 3-*N*-acetyl-6-carboxyrhodamine, 2,7-difluoro-3-*N*-acetylrhodol, 3-*O*-(*N*,*N*-dimethyl-2-aminoethyl)-6-carboxyfluorescein, 2,7-dichloro-3-*O*-(*N*,*N*-dimethyl-2-aminoethyl)fluorescein, blackberry quencher (BBQ), black hole quencher 3 (BHQ3), 2-(2-hydroxyphenyl)quinazolin-4-one, 6-chloro-2-(5-chloro-2-hydroxyphenyl)quinazolin-4-one, or 6-bromo-2-(5-bromo-2-hydroxyphenyl)quinazolin-4-one.

Radiotracer agents are agents useful for imaging which incorporate a radioactive isotope, non-limiting examples of which are radioactive isotopes of carbon, cobalt, fluorine, gallium, iodine, or technetium, preferably carbon, fluorine or iodine. Compounds of the present invention which have been labelled with a radioactive isotope can be employed as diagnostic applications.

The term **"pharmaceutically acceptable salt"** refers to a salt of a compound of the present invention. Suitable pharmaceutically acceptable salts include acid addition salts which may, for example, be formed by mixing a solution of compounds of the present invention with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compound carries an acidic moiety, suitable pharmaceutically acceptable salts thereof may include alkali metal salts (e.g., sodium or potassium salts); alkaline earth metal salts (e.g., calcium or magnesium salts); and salts formed with suitable organic ligands (e.g., ammonium, quaternary ammonium and amine cations formed using counteranions such as halide, hydroxide, carboxylate, sulfate, phosphate, nitrate, alkyl sulfonate and aryl sulfonate). Illustrative examples of pharmaceutically acceptable salts include, but are not limited to, acetate, adipate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, clavulanate, cyclopentanepropionate, digluconate, dihydrochloride, dodecylsulfate, edetate, edisylate, estolate, esylate, ethanesulfonate, formate, fumarate, gluceptate, glucoheptonate, gluconate, glutamate, glycerophosphate, hemisulfate, heptanoate, hexanoate, hydrabamine, hydrobromide, hydrochloride, hydroiodide, 2-hydroxy-ethanesulfonate, hydroxynaphthoate, iodide, isothionate, lactate, lactobionate, laurate, lauryl sulfate, malate, maleate, malonate, mandelate, mesylate, methanesulfonate, methylsulfate, mucate, 2-naphthalenesulfonate, napsylate, nicotinate, nitrate, *N*-methylglucamine ammonium salt, oleate, oxalate, pamoate (embonate), palmitate, pantothenate, pectinate, persulfate, 3-phenylpropionate, phosphate/diphosphate, picrate, pivalate, polygalacturonate, propionate, salicylate, stearate, sulfate, succinate, tannate, tartrate, teoclate, tosylate, triethiodide, undecanoate, valerate, and the like (see, for example, S. M. Berge et al.; J. Pharm. Sci. 1977, 66, 1-19).

When the compounds of the present invention are provided in crystalline form, the structure can contain solvent molecules. The solvents are typically pharmaceutically acceptable solvents and include, among others, water (hydrates) or organic solvents. Examples of possible **solvates** include hydrates, ethanolates and iso-propanolates.

The term **"pharmaceutically acceptable ester"** refers to an ester of a compound of the present invention. Suitable pharmaceutically acceptable esters include acetyl, butyryl, and acetoxymethyl esters.

The term **"protecting group",** as used herein (represented in schemes as moieties -PG), represents a group intended to protect a hydroxy or an amino group from participating in one or more undesirable reactions during chemical synthesis. The term **"*O*-protecting group",** as used herein, represents a group intended to protect a hydroxy or carbonyl group from participating in one or more undesirable reactions during chemical synthesis. The term **"*N*-protecting group",** as used herein, represents a group intended to protect a nitrogen containing (e.g., an amino or hydrazine) group from participating in one or more undesirable reactions during chemical synthesis. Exemplary *O*- and *N*-protecting groups include, but are not limited to: alkanoyl, aryloyl, or carbamyl groups such as formyl, acetyl, propionyl, pivaloyl, *tert*-butylacetyl, 2-chloroacetyl, 2-bromoacetyl, trifluoroacetyl, trichloroacetyl, phthalyl, o-nitrophenoxyacetyl, *α*-chlorobutyryl, benzoyl, 4-chlorobenzoyl, 4-bromobenzoyl, triiso-propylsilyloxymethyl, 4,4'-dimethoxytrityl, isobutyryl, phenoxyacetyl, 4-isopropylpehenoxyacetyl, dimethylformamidino, and 4-nitrobenzoyl. Other *O*-protecting groups include, but are not limited to: substituted alkyl, aryl, and aryl-alkyl ethers (e.g., trityl; methylthiomethyl; methoxymethyl; benzyloxymethyl; siloxymethyl; 2,2,2,-trichloroethoxymethyl; tetrahydropyranyl; tetrahydrofuranyl; ethoxyethyl; 1-[2-(trimethylsilyl)ethoxy]ethyl; 2-trimethylsilylethyl; *tert*-butyl ether; *p*-chlorophenyl, p-methoxyphenyl, p-nitrophenyl, benzyl, p-methoxybenzyl, and nitrobenzyl); silyl ethers (e.g., trimethylsilyl; triethylsilyl; triisopropylsilyl; dimethylisopropylsilyl; *tert*-butyldimethylsilyl; *tert*-butyldiphenylsilyl; tribenzylsilyl; triphenylsilyl; and diphenymethylsilyl); carbonates (e.g., methyl, methoxymethyl, 9-fluorenylmethyl; ethyl; 2,2,2-trichloroethyl; 2-(trimethylsilyl)ethyl; vinyl, allyl, nitrophenyl; benzyl; methoxybenzyl; 3,4-dimethoxybenzyl; and nitrobenzyl). Other *N*-protecting groups include, but are not limited to, chiral auxiliaries such as protected or unprotected, *L*- or D-amino acids such as alanine, leucine, phenylalanine, and the like; sulfonyl-containing groups such as benzenesulfonyl, p-toluenesulfonyl, and the like; carbamate forming groups such as benzyloxycarbonyl, *p*-chlorobenzyloxycarbonyl, p-methoxybenzyloxycarbonyl, *p*-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyl oxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1 -(*p*-biphenylyl)-Imethylethoxycarbonyl, a,a-dimethyl-3,5-dimethoxybenzyloxycarbonyl, benzhydryloxy carbonyl, tbutyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxy carbonyl, fluorenyl-9-methoxycarbonyl, cyclopentyloxycarbonyl, adamantyloxycarbonyl, cyclohexyloxycarbonyl, phenylthiocarbonyl, and the like, aryl-alkyl groups such as benzyl, triphenylmethyl, benzyloxymethyl, and the like and silyl groups such as trimethylsilyl, and the like. Useful *N*-protecting groups are formyl, acetyl, benzoyl, pivaloyl, *tert*-butylacetyl, alanyl, phenylsulfonyl, benzyl, *tert*-butyloxycarbonyl (Boc), and benzyloxycarbonyl (Cbz).

The term **"proteinogenic amino acids"** herein comprises the 20 principal naturally-occurring *L*-amino acids (glycine, alanine, valine, leucine, isoleucine, phenylalanine, proline, methionine, serine, threonine, cysteine, tyrosine, tryptophan, asparagic acid, glutamic acid, asparagine, arginine, histidine, lysine, asparagine and glutamine) which are to be attached, as the monopeptide, via their carboxyl terminus to the specified amine nitrogen of the compound, creating amides. Preferably the proteinogenic amino acid is selected from leucine, serine and lysine.

The term **"self-immolative spacer",** also called "traceless linker" or "self-immolative linker", as used herein, represents a multivalent (e.g., divalent) group covalently linking a disulfide-containing group which will be further defined below (as the moiety A), to a cargo B, in such a way that reductive cleavage of the disulfide bond in the moiety A is followed by a sequence of reactions which result in the cleavage of a bond between the self-immolative linker group and the cargo B, thereby releasing the cargo. Illustrative examples thereof include:

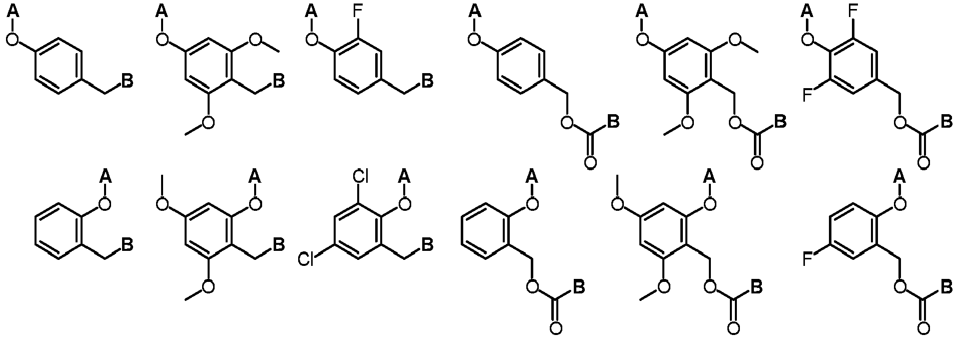

The stereochemistry at the ring atoms bridging the two rings of the bicyclic disulfide structure of the compounds of formula (I) of the invention is not particularly limited; any absolute configuration of the two bridging ring tertiary carbon atoms is within the scope of the compounds of formula (I). Either carbon can be separately either R, or S, or a mixture of R and S in any proportions (including racemic and undefined proportions); therefore the disulfide ring may be considered to be cis-fused to the carbamate-containing ring with any given stereochemistry, or *trans*-fused to the carbamate-containing ring with any given stereochemistry, or present as a mixture in any proportions of cis-fused and *trans*-fused with any given stereochemistries (including racemic and undefined proportions). Preferably, the disulfide ring is either cis-fused or *trans*-fused.

The term **"vascular disrupting agent"** refers to a compound that reduces tumor blood flow upon administration. Such agents are typically microtubule-depolymerising agents, and are well represented in the literature (Gill, Pharmacology & Therapeutics 2019, 202, 18-31); as known to those skilled in the art, these include combretastatin A-4 (CA4), 3'-aminocombretastatin A-4, BNC105, ABT-751, ZD6126, combretastatin A-1, and prodrugs of the same, including but not limited to combretastatin A-4 phosphate (CA4P), 3'-aminocombretastatin A-4 3'-serinamide (ombrabulin), combretastatin A-1 bisphosphate (CA1P), and BNC105 phosphate (BNC105P).

The term **"cytotoxic chemotherapeutic agent"** refers to compounds that have antineoplastic and/or antitumoral efficacy and which are typically useful in the treatment of cancer, optionally as part of combination therapies. Such agents include paclitaxel, docetaxel, nab-paclitaxel, epothilone, mertansine, irinotecan, etoposide, teniposide, mitoxantrone, doxorubicin, daunorubicin, epirubicin, cisplatin, carboplatin, oxaliplatin, melphalan, chlorambucil, busulfan, methotrexate, permetrexed, cyclophosphamide, 5-fluorouracil, capecitabine, gemcitabine, cytarabine, fludarabine, mercaptopurine, vinolrebine, vinblastine, vincristine, dolastatin, monomethyl auristatin E, monomethyl auristatin F, bleomycin, dacarbazine, pyrrolobenzodiazepine, temozolomide, carmustine, mitomycin and procarbazine.

In the context of the invention the term **"immunomodulator"** refers to therapeutically active compounds that interact with the immune system, which may for example be useful in the treatment of cancer or autoimmune diseases, optionally as part of combination therapies. Such agents include anti-PD-1 antibody such as pembrolizumab and nivolumab, anti-PD-L1 antibody such as avelumab, anti-CTLA4 antibody such as ipilimumab, cytokine agonists such as proleukin and interferon alfa-2a/interferon alfa-2b, mesalamine, baricitinib, filgotinib, tofacitinib, upadacitinib, ruxolitinib, peficitinib, decemotinib, solcitinib, itacitinib, fostamatinib, and SHR0302.

### Detailed description of the invention

### Compound having the formula (I)

The present invention provides a compound having the formula (I)

A-L-B (I)

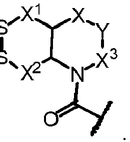

A is represented by
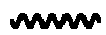 denotes the attachment point from A to L.
X is selected from -N(R^{a})-, -N(R^{b})- and -CR^{c}₂-, preferably -N(R^{a})- or -CR^{c}₂.
X¹ is -(CR^{d}₂)ₘ-, preferably -(CH₂)-.
X² is -(CR^{e}₂)ₙ-, preferably -(CH₂)-.
X³ is -CR^{f}₂-, preferably -(CH₂)-.
Y is -(CR^{g}₂)ₚ-, preferably -(CH₂)- or -(CH₂)-(CH₂)-, more preferably -(CH₂)-.
W is selected from -OH, -N(R^{h})(Rⁱ), or a heterocyclic group selected from azetidinyl, pyrrolidinyl, piperidinyl or morpholino, wherein the heterocyclic group is attached to the -C₁₋₄-alkyl or -(C₂₋₄-alkylene)- group via the N atom. Preferably, W is selected from -OH, -N(CH₃)₂, -azetidinyl or-morpholino. More preferably, W is selected from -OH and -N(CH₃)₂.
R^{a} is selected from -H, -C₁₋₄-alkyl, -C(O)-C₁₋₄-alkyl, -C(O)-O-C₁₋₄-alkyl, -C(O)-N(C₁₋₄-alkyl)₂, -S(O)₂-C₁₋₄-alkyl, and -(C₂₋₄-alkylene)-O-(C₁₋₄-alkyl), wherein -(C₂₋₄-alkylene) or -(C₁₋₄-alkyl) can be optionally substituted by W. Preferably R^{a} is selected from -H and -C₁₋₄-alkyl, wherein -(C₁₋₄-alkyl) can be optionally substituted by W. More preferably, R^{a} is selected from -H, -CH₃, -C₂H₅, and -CH₂CH₂W.
R^{b} is an acyl group of a monopeptide selected from proteinogenic amino acids attached via a carboxy group. The attachment point is not particularly limited, the proteinogenic amino acid can be connected via the carboxy group at the α-carbon or via a carboxy group of the side chain forming an amide group with the ring nitrogen atom, preferably the proteinogenic amino acid is connected via the carboxy group at the α-carbon. The proteinogenic amino acid is not particularly limited and can be any amino acid as defined above; preferably leucine, serine or lysine; more preferably serine. In the case of serine, for example, R^{b} is -C(O)-C(H)(NH₂)-CH₂OH.
R^{c} groups are independently selected from -H and -C₁₋₄-alkyl, preferably H.
R^{d} groups are independently selected from -H and -C₁₋₄-alkyl, preferably H.
R^{e} groups are independently selected from -H and -C₁₋₄-alkyl, preferably H.
R^{f} groups are independently selected from -H and -C₁₋₄-alkyl, preferably H.
R^{g} groups are independently selected from -H and -C₁₋₄-alkyl, preferably H.
R^{h} is selected from -H, -C₁₋₄-alkyl and -CH₂CH₂OH; preferably, from -H, -CH₃ and -CH₂CH₂OH.
Rⁱ is selected from -H, -C₁₋₄-alkyl and -CH₂CH₂OH; preferably, from -H, -CH₃ and -CH₂CH₂OH.
m is 0, 1 or 2 and n is 1 or 2, provided that m+n is 2 or 3. Preferably m is 1 and n is 1.
p is 0, 1, or 2, provided that when X = -N(R^{a})- or -N(R^{b})- then p = 1 or 2. Preferably p is 1 or 2. More preferably p is 1.
L is a bond or a self-immolative spacer. In one embodiment, L is a bond. In another embodiment, L is a self-immolative spacer. A self-immolative spacer is any linking group which is capable of covalently linking A and B and which can release the cargo H-B when the disulfide bond in A is cleaved and the resulting dithiol species cyclizes *via* nucleophilic attack at the carbonyl group of A. The self-immolative spacer is not particularly limited and can be chosen from any known self-immolative spacer. Examples are to be found in e.g. Blencowe, C. A. et al. Polym. Chem. 2011, 2, 773-790. https://doi.org/10.1039/C0PY00324G. Preferred examples thereof include, but are not limited to, the moieties shown in the Definitions section, and, in particular, para-hydroxybenzylic 1,4-elimination self-immolative spacers and *para-*hydroxybenzylic 1,6-elimination self-immolative spacers:

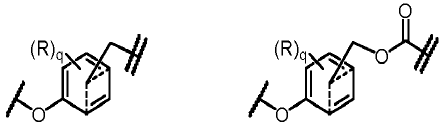

In these formulae (R)_{q}- can be attached at any available position on the benzene ring. 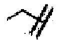 and 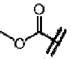 can be attached at either of the carbon atoms which are indicated by the dashed lines.

More preferably the self-immolative spacer is a para-hydroxybenzylic 1,6-elimination spacer:

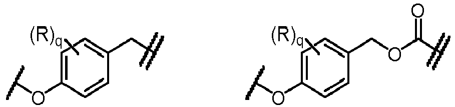

In these formulae 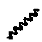 denotes the attachment from A to L. 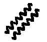 denotes the attachment point from L to B.

If L is a bond then the cargo B, which is a therapeutic, diagnostic or theranostic agent, can contain an -OH group that is attached to a 5- or 6-membered aromatic or heteroaromatic ring, preferably an -OH group that is attached to a 6-membered aromatic ring, more preferably an -OH group that is attached to phenyl ring. The 5- or 6-membered aromatic or heteroaromatic ring or the phenyl ring can be part of a larger structure as defined below. In these embodiments L is suitable for binding to A *via* the O atom, giving a carbamate.

If L is 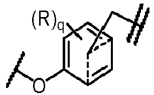 then the cargo, which may be a therapeutic, diagnostic or theranostic agent (such as an aniline or amine) that contains an -NH₂ or -NH- moiety; or an -OH group that is attached to a 5- or 6-membered aromatic or heteroaromatic ring that can be part of a larger structure as defined below; preferably, an -OH group that is attached to a 5- or 6-membered aromatic ring that can be part of a larger structure as defined below; more preferably, an -OH group that is attached to a phenyl ring that can be part of a larger structure as defined below.

In these embodiments the -NH₂ or -NH- moiety or the OH group is suitable for binding to L *via* the respective N or O atom.

If L is 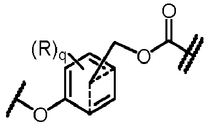 then the cargo, which is a therapeutic, diagnostic or theranostic agent, can contain an -NH₂ or -NH- moiety; or an -OH group that is attached to a 5- or 6-membered aromatic or heteroaromatic ring; preferably an -NH₂ or -NH- moiety which is attached to an aromatic ring that can be part of a larger structure as defined below, or an -OH group that is attached to a 5- or 6-membered aromatic ring that can be part of a larger structure as defined below; more preferably an -OH group that is attached to a phenyl ring that can be part of a larger structure as defined below.

In these embodiments the -NH₂ or -NH- moiety or the OH group is suitable for binding to L *via* the respective N or O atom.

q is 0, 1, 2, 3 or 4, preferably q is 0 or 1.

Each group R is independently selected from -halogen -O(R^{r}), -N(R^{s})(R^{t}), -NO₂, -CN, and a heterocyclic group selected from azetidinyl, pyrrolidinyl, piperidinyl or morpholino, wherein the heterocyclic group is attached to the aromatic ring *via* the N atom. Preferably, group R is a halogen (such as F or Cl) in *ortho* position relative to the bond to A; also preferably, group R is -O(R^{r}) in *ortho* position relative to the bond to B.

R^{r} is selected from -H and -C₁₋₄-alkyl, preferably -C₁₋₄-alkyl, more preferably R^{r} is methyl.

R^{s} is selected from -H, -C(O)-C₁₋₄-alkyl and -C₁₋₄-alkyl, preferably -H or -C₁₋₄-alkyl, more preferably -H or -CH₃.

R^{t} is selected from -H, -C(O)-C₁₋₄-alkyl and -C₁₋₄-alkyl, preferably -H or -C₁₋₄-alkyl, more preferably -H or -CH₃.

Most preferred examples of self-immolative spacers that link groups A and B are

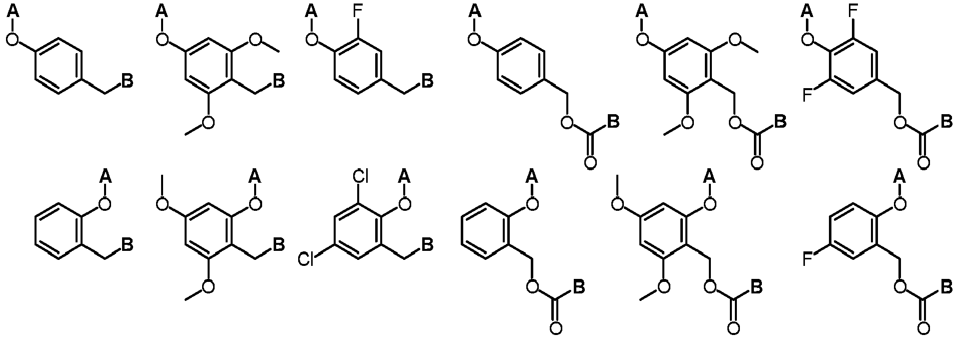

B is 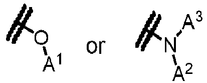 in which 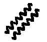 denotes the attachment point from L to B.

A¹ is selected such that A¹-OH is a therapeutic, diagnostic or theranostic agent which contains an -OH group that is attached to a 5- or 6-membered aromatic or heteroaromatic ring, preferably an -OH group that is attached to a 5- or 6-membered aromatic ring, more preferably an -OH group that is attached to a phenyl ring. The bond attaching L to that O atom replaces its bond to hydrogen that is present in the agent A¹-OH. The 5- or 6-membered aromatic or heteroaromatic ring can be part of a larger structure which forms the therapeutic, diagnostic or theranostic agent A¹-OH. In particular, the 5- or 6-membered aromatic or heteroaromatic ring can be substituted, or can be fused to a further ring or rings such as aromatic or heteroaromatic rings, preferably phenyl, pyridinyl, naphthyl, quinolyl, isoquinolyl, indolyl, pyrrolyl, thiophenyl, pyridinyl, pyrazolyl, pyrimidinyl, furanyl, oxazolyl, thiazolyl, imidazolyl, purinyl, pyrrolo, benzofuranyl, benzothiophenyl, benzothiazolyl, benzoxazolyl or naphthyridinyl, quinoxalyl, benzopyranonyl, benzo[1,3]dioxolyl, benzothiazolyl, 2,3-dihydro-1*H*-benzo[e]indole, xanthenyl or indolyl, more preferably phenyl, pyridinyl, benzopyranonyl, naphthyl, benzo[1,3]dioxolyl, 2,3-dihydro-1*H*-benzo[e]indole, quinolyl or xanthenyl.

A¹ can be any hydrocarbon moiety which contains a 5- or 6-membered aromatic or heteroaromatic ring, wherein the hydrocarbon moiety has from 3 to 50 carbon atoms, preferably from 5 to 50 carbon atoms, more preferably 6 to 50 carbon atoms. The hydrocarbon moiety can optionally contain 1 or more heteroatoms, preferably 1 to 20 heteroatoms, more preferably 1 to 15 heteroatoms, wherein the heteroatoms can be selected from O, N, S, Se, Si, Hal, B or P, preferably selected from O, N, Hal, S, Si or P, more preferably selected from O, N, Hal, S, Si, even more preferably selected from O, N or Hal.

A² and A³ are independently selected such that A²-NH-A³ is a therapeutic, diagnostic or theranostic agent which contains an -NH₂ or -NH- moiety. A² and A³ can be independently selected from 5- or 6-membered aromatic or heteroaromatic rings and aliphatic moieties, and A² may optionally be H. In one embodiment, A² and A³ may optionally be connected forming a carbocyclic or heterocyclic ring that can be saturated, unsaturated or aromatic and which has from 5 to 7 ring atoms. Each 5- or 6-membered aromatic, heteroaromatic, carbocyclic or heterocyclic ring can be part of a larger structure which forms the therapeutic, diagnostic or theranostic agent A²-NH-A³. In particular, the 5- or 6-membered aromatic or heteroaromatic ring or the carbocyclic or heterocyclic ring can be substituted or can be fused to a further ring or rings such as aromatic or heteroaromatic rings, preferably phenyl, pyridinyl, naphthyl, quinolyl, isoquinolyl, indolyl, pyrrolyl, thiophenyl, pyridinyl, pyrazolyl, pyrimidinyl, furanyl, oxazolyl, thiazolyl, imidazolyl, purinyl, pyrrolo, benzofuranyl, benzothiophenyl, benzothiazolyl, benzoxazolyl or naphthyridinyl, quinoxalyl, benzopyranonyl, benzothiazolyl, xanthenyl or indolyl. The aliphatic moiety can be substituted.

A² can be hydrogen or any hydrocarbon moiety which has from 1 to 50 carbon atoms, preferably from 1 to 20 carbon atoms, more preferably 1 to 10 carbon atoms, even more preferably 1 to 4 carbon atoms. The hydrocarbon moiety can optionally contain 1 or more heteroatoms, preferably 1 to 10 heteroatoms, more preferably 1 to 5 heteroatoms, wherein the heteroatoms can be selected from O, N, S, Se, Si, Hal, B or P, preferably selected from O, N, Hal, S, Si or P, more preferably selected from O, N, Hal, S, Si, even more preferably selected from O, N or Hal. In one embodiment A² is a C₁₋₄ alkyl, such as methyl.

A³ can be any hydrocarbon moiety which contains from 3 to 50 carbon atoms, preferably from 5 to 50 carbon atoms, more preferably 6 to 50 carbon atoms. The hydrocarbon moiety can optionally contain 1 or more heteroatoms, preferably 1 to 20 heteroatoms, more preferably 1 to 15 heteroatoms, wherein the heteroatoms can be selected from O, N, S, Se, Si, Hal, B or P, preferably selected from O, N, Hal, S, Si or P, more preferably selected from O, N, Hal, S, Si, even more preferably selected from O, N or Hal.

If A² and A³ are bound together to form a carbocyclic or heterocyclic ring, the carbocyclic or heterocyclic ring can contain from 3 to 50 carbon atoms, preferably from 5 to 50 carbon atoms, more preferably 6 to 50 carbon atoms. The hydrocarbon moiety can optionally contain 1 or more heteroatoms, preferably 1 to 20 heteroatoms, more preferably 1 to 15 heteroatoms, wherein the heteroatoms can be selected from O, N, S, Se, Si, Hal, B or P, preferably selected from O, N, Hal, S, Si or P, more preferably selected from O, N, Hal, S, Si, even more preferably selected from O, N or Hal.

The nature of the therapeutic agent, diagnostic agent or theranostic agent is not particularly limited, as long as it contains an -OH, -NH₂ or -NH- moiety which after removal of the H is suitable for binding to the self-immolative spacer L, or, if L is a bond, to A, via the O or N atom. As can be seen from the schemes below, the O or N atom partakes in the reaction when the moiety A of a compound having the formula (I) is cleaved by a disulfide reductase or its effector disulfide protein. The rest of the therapeutic agent, diagnostic agent or theranostic agent is not involved in this reaction, so that a wide variety of therapeutic agents, diagnostic agents and theranostic agents are suitable for use in the present invention.

Examples of suitable therapeutic agents, diagnostic agents and theranostic agents include, but are not limited to diagnostically acceptable dyes, therapeutically acceptable DNA-alkylating agents, therapeutically acceptable DNA-intercalating agents or therapeutically acceptable tubulin-inhibiting agents. In one embodiment, diagnostically acceptable dyes are preferred, which includes compounds or structurally and functionally related derivatives selected from, but not limited to the following classes of dyes: xanthenes including e.g. alkyl-fluoresceins, alkyl-carbofluoresceins, alkyl-silarhodamines, phenylquinazolinones, coumarins, firefly-luciferins, black hole quencher dyes, or any compound derived by substitution of the same. How acceptable compounds may be derived by substitution will be clarified in the subsequent examples. In another embodiment, therapeutically acceptable agents are preferred, which includes theranostic agents that include structurally and functionally related derivatives of the leuco-methylene blue class; DNA-alkylating agents that include compounds or structurally and functionally related derivatives selected from, but not limited to the following classes: nitrogen mustards or seco-cyclopropabenzaindoles; DNA-intercalating agents that include compounds or structurally and functionally related derivatives selected from, but not limited to the following classes of compounds: anthracyclins or camptothecins; antiproliferative agents that include compounds or structurally and functionally related derivatives selected from, but not limited to the following classes: monomethyl auristatins, the Amaryllidaceae alkaloids; or any compounds derived by substitution of the same.

Examples of therapeutic agents, diagnostic agents and theranostic agents include, but are not limited to, agents for treating, ameliorating, preventing or diagnosing a neoplastic disorder, an inflammatory disorder (e.g. a non-steroidal anti-inflammatory drug (NSAID), a disease-modifying anti-rheumatic drug (DMARD), or mesalamine), atherosclerosis; an autoimmune disorder; a chronic inflammatory autoimmune disorder; ischaemia; and reperfusion injury as well as kinase inhibitors.

Preferred are agents for which the conjugation of the active agent H-B into the compound A-L-B results in a substantial reduction of diagnostic signal or therapeutic potency; particularly preferred are therapeutically acceptable agents for which the conjugation of the active agent H-B into the compound A-L-B totally or near-totally prevents the compound from exhibiting the diagnostic signal or therapeutic potency that is associated with the free agent H-B. This preferred total or near-total prevention of the activity of agent H-B when conjugated as A-L-B is possible in several ways, non-limiting examples of which are given below in the section

### Utility, Diseases, and Examples.

How suitable diagnostic, therapeutic or theranostic agents may be derived by substitution of specific agents will be understood by reference to the following non-limiting examples:
A¹-OH or A²-NH-A³ can be a diagnostic agent of the xanthene class: 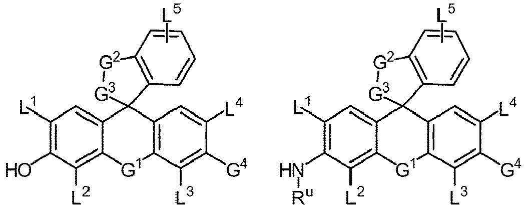
L¹, L², L³ or L⁴ are independently selected from -H, -halogen, -NO₂, -CN, -OMe, -O-CF₃.
L⁵ is attached at any free position on the indicated benzene ring and is selected from -H, -C₁₋₄-alkyl, -halogen, -C(O)-C₁₋₄-alkyl, -C(O)-O-C₁₋₄-alkyl, -C(O)-N(-C₁₋₄-alkyl)₂, -S(O)₂-OH, -S(O)₂-O-C₁₋₄-alkyl, -O-C₁₋₄-alkyl.
G¹ is selected from -O-, -S-, -Se-, -C(-C₁₋₄-alkyl)₂-, -Si(-C₁₋₄-alkyl)₂-.
G² is selected from -CH₂-, -C(O)-, -S(O)₂- -P(O)₂-.
G³ is selected from -O-, -N(-C₁₋₄-alkyl)-.
G⁴ is selected from -OH, -O-C₁₋₄-alkyl, -NH₂, -NH-C₁₋₄-alkyl, -N(-C₁₋₄-alkyl)₂, -N-C(O)-C₁₋₄-alkyl, wherein any of the C₁₋₄-alkyl in G⁴ can be optionally substituted by W. R^{u} is selected from -H, -C₁₋₄-alkyl.

When A¹ is selected such that A¹-OH is a diagnostic agent of the phenylquinazolinone class: 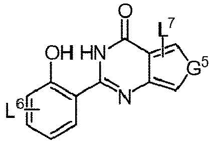 G⁵ is selected from -CH₂-, -CH=CH-, -O-, -S-, -Se-.
One or more substituent L⁶ and one or more substituent L⁷ are independently selected from
-H, -C₁₋₄-alkyl, -halogen, -O-C₁₋₄-alkyl, -CF₃, -NO₂.

When A¹ is selected such that A¹-OH is a diagnostic agent of the umbelliferone class: 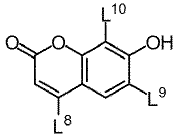 L⁸ is selected from -H, -C₁₋₄-alkyl, -halogen, -O-C₁₋₄-alkyl, -CF₃, -NO₂.
L⁹ is selected from -H, -C₁₋₄-alkyl, -halogen, -O-C₁₋₄-alkyl, -CF₃, -NO₂.
L¹⁰ is selected from -H, -C₁₋₄-alkyl, -halogen, -O-C₁₋₄-alkyl, -CF₃, -NO₂.

When A¹, A² or A³ are selected such that A¹-OH or A²-NH-A³ is a therapeutic agent of the seco-cyclopropabenzaindole (CBI) class of DNA-alkylating anti-cancer drugs: 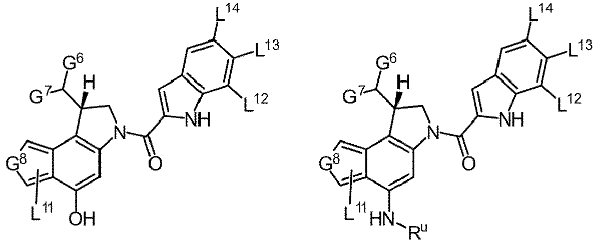 G⁶ is selected from -CI, -Br, -I, -OMs, -OTs, -OTf, -ONs.
G⁷ is selected from -H, -C₁₋₄-alkyl, -CF₃.
G⁸ is selected from -CH=CH-, -O-, -S-.
One or more substituent L¹¹ is independently selected from -H, -F, -C₁₋₄-alkyl, -CF₃, -OMe, -O-C₁₋₄-alkyl.
L¹² and L¹³ are independently selected from -H, -F, -C₁₋₄-alkyl, -CF₃, -OMe, -O-C₁₋₄-alkyl, wherein any of the C₁₋₄-alkyl in L¹² and L¹³ can be optionally substituted by W.
L¹⁴ is selected from -O-C₁₋₄-alkyl optionally substituted by W.

When A² and A³ are selected such that A²-NH-A³ is a diagnostic or theranostic agent of the leuco-methylene blue class: 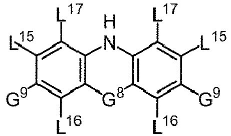 G⁸ is selected from -S-, -Se-, -Si(-C₁₋₄-alkyl)₂-, -Ge(-C₁₋₄-alkyl)₂-.
G⁹ is selected from -NH₂, -NH(-C₁₋₄-alkyl), -N(-C₁₋₄-alkyl)₂.
L¹⁵, L¹⁶ and L¹⁷ are independently selected from -H, -C₁₋₄-alkyl, -halogen, -O-C₁₋₄-alkyl, -CF₃, -NO₂.

When A¹ is selected such that A¹-OH is a therapeutic agent of the camptothecin class of DNA-intercalating anti-cancer drugs: 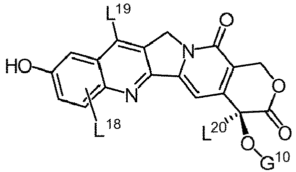 G¹⁰ is selected from -H, -C(O)-C_{alkyl}.
One or more substituent L¹⁸ are independently selected from -H, -F, -CF₃, -CH₂-CF₃, -C₁₋₄-alkyl, -CH₂-N(-C₁₋₄-alkyl)₂.
L¹⁹ is selected from -H, -F, -CF₃, -CH₂-CF₃, -C₁₋₄-alkyl, -CH₂-N(-C₁₋₄-alkyl)₂.
L²⁰ is selected from -CF₃, -CH₂-CF₃, -C₁₋₄-alkyl.

When A¹ or A² and A³ are selected such that A¹-OH or A²-NH-A³ is a therapeutic agent of the nitrogen mustard class of DNA-alkylating agents: 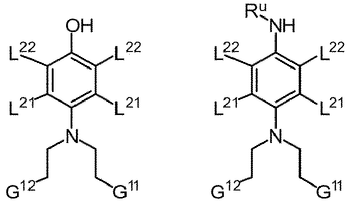 G¹¹ and G¹² are independently selected from -CI, -Br, -I, -OMs, -OTs, -OTf, -ONs.
L²¹ and L²² are independently selected from -H, -C₁₋₄-alkyl, -halogen, -O-C₁₋₄-alkyl, -CF₃, -NO₂.

When A¹ is selected such that A¹-OH is a diagnostic agent of the firefly luciferin class: 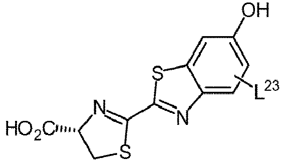 One or more substituent L²³ is selected from -H, -C₁₋₄-alkyl, -halogen, -O-C₁₋₄-alkyl, -CF₃, -NO₂.

When A¹ is selected such that A¹-OH is a therapeutic agent of the Amaryllidaceae class of anti-proliferative alkaloids: 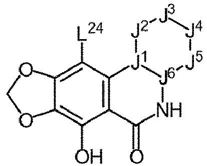 L²⁴ is selected from -H, -C₁₋₄-alkyl, -halogen, -O-C₁₋₄-alkyl, -CF₃, -NO₂.
J¹ and J⁶ are independently selected from -CH- or =C-.
J², J³, J⁴ and J⁵ are independently selected from -CH₂-, -CH(-OH)-,
-CH(-O-(C(O)-C₁₋₄-alkyl))-, -CH(-O-(galactoside)-, -CH(-O-(glucoside)- or =CH-.

When A¹ is selected such that A¹-OH is a diagnostic agent of the Black hole quencher class: 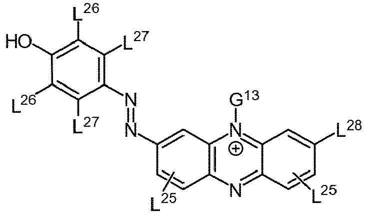 One or more substituent L²⁵ is independently selected from -H, -C₁₋₄-alkyl, -halogen, -O-C₁₋₄-alkyl, -CF₃, -NO₂.
L²⁶ and L²⁷ are independently selected from -H, -C₁₋₄-alkyl, -halogen, -O-C₁₋₄-alkyl, -CF₃, -NO₂.
L²⁸ is selected from -H, -C₁₋₄-alkyl, -halogen, -O-C₁₋₄-alkyl, -N(-C₁₋₄-alkyl)₂, -CF₃, -NO₂. G¹³ is selected from phenyl, pyridine-2-yl, pyridine-3-yl, pyridine-4-yl, 2-methoxyphenyl, 3-methoxyphenyl, 4-methoxyphenyl, 3,5-dimethoxyphenyl, 2,4-dimethoxyphenyl, 2,3,4-trimethoxyphenyl, pyrazin-2-yl.

When A² and A³ are selected such that A²-NH-A³ is a therapeutic agent of the anthracycline class: 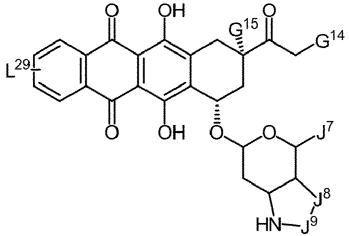 G¹⁴ is selected from H, -C₁₋₄-alkyl, -OH, -NH₂, -O-C₁₋₄-alkyl, -NH(-C₁₋₄-alkyl), -N(-C₁₋₄-alkyl)₂, -CF₃.
G¹⁵ is selected from H, -C₁₋₄-alkyl, -OH, -NH₂.
J⁷ is selected from H, -C₁₋₄-alkyl, -OH, -NH₂, -O-C₁₋₄-alkyl, -CF₃.
J⁸ is selected from H, -C₁₋₄-alkyl, -OH, -O-(tetrahydro-2H-pyran-2-yl), -NH₂, -O-C₁₋₄-alkyl, -CF₃, -CH₂-, -C(-C₁₋₄-alkyl)₂-, -O- -S-.
If J⁸ is selected from H, -C₁₋₄-alkyl, -OH, -O-(tetrahydro-2H-pyran-2-yl), -NH₂, -O-C₁₋₄-alkyl,
-CF₃, then J⁹ is selected from -CH₂-, -C(-C₁₋₄-alkyl)₂-.
If J⁸ is selected from -CH₂-, -C(-C₁₋₄-alkyl)₂-, -O-, -S-, then J⁹ is selected from -CH₂-, -C(-C₁₋₄-alkyl)₂-.
One or more substituent L²⁹ is independently selected from -O-C₁₋₄-alkyl, -OH, -halogen, -NH₂,-O-C₁₋₄-alkyl, -CF₃.

When A² and A³ are selected such that A²-NH-A³ is a therapeutic agent of the auristatin class: 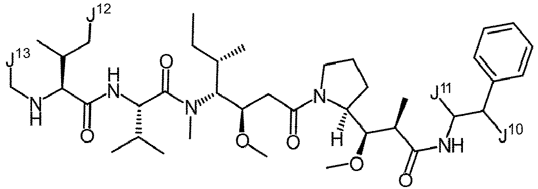 J¹⁰ is selected from H, -C₁₋₄-alkyl, -OH, -NH₂, -O-C₁₋₄-alkyl.
J¹¹ is selected from H, -C₁₋₄-alkyl, -OH, -NH₂, -O-C₁₋₄-alkyl, -C(O)-H, C(O)-O-C₁₋₄-alkyl, thiazo-2-yl, thiazo-4-yl, oxazo-2-yl, oxazo-4-yl.
J¹² is selected from H, -C₁₋₄-alkyl.

J¹³ is selected from H, -C₁₋₄-alkyl.

Illustrative embodiments of the compound having the formula (I) include

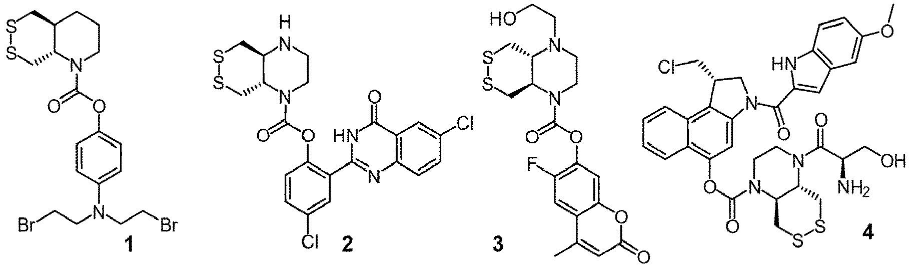

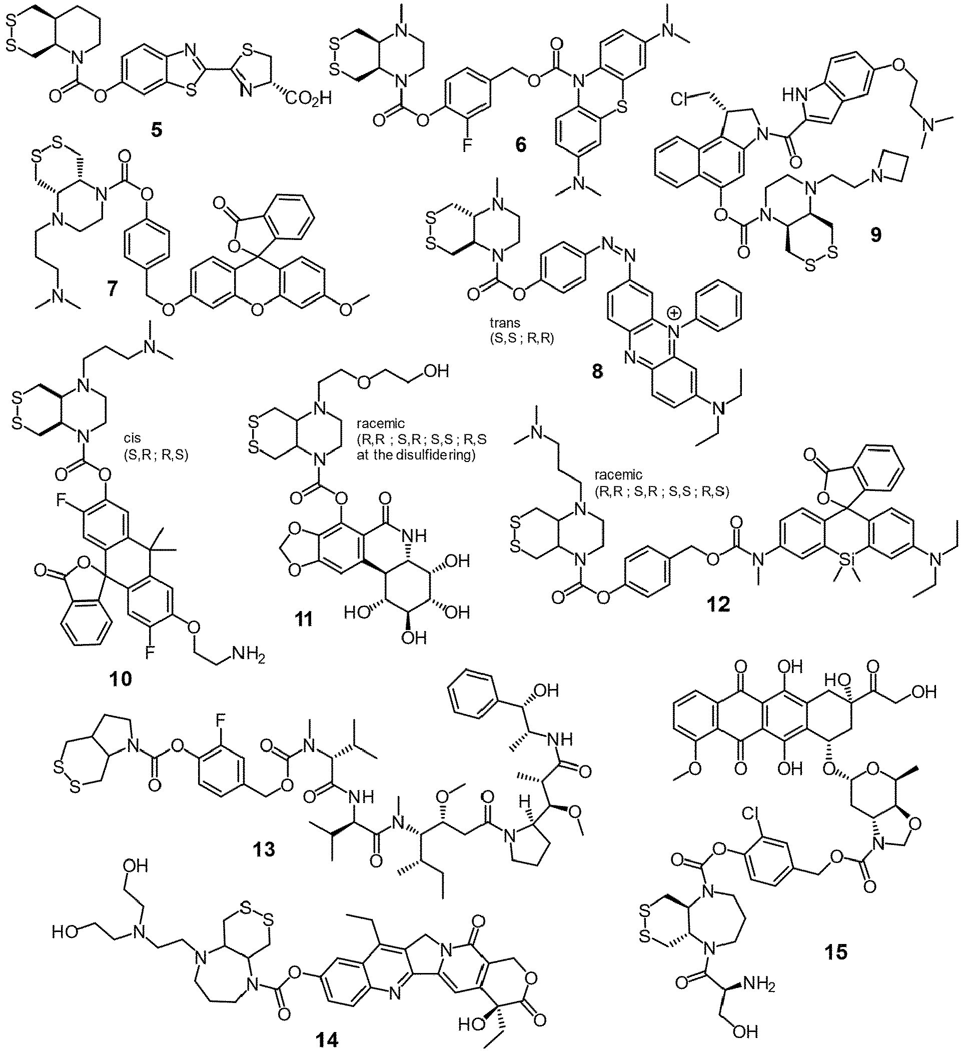

It is understood that all combinations of the above definitions and preferred definitions are envisaged by the present inventors.

### Synthesis of the compounds having the formula (I)

### A) General manufacturing of the disulfide-containing moiety A

In some embodiments, general manufacturing of the compound of the invention can begin from a diol precursor similar to diol **A1,** which itself can be prepared from commercially available starting materials using literature known procedures.

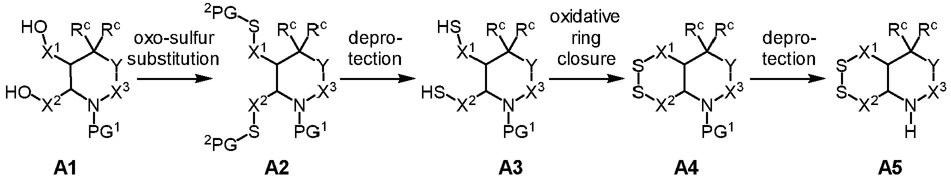

PG¹ represents any protecting group for the heterocyclic nitrogen atom, which intermediately masks the nitrogen's nucleophilic reactivity and can be cleaved without affecting other functional groups in the molecule. Preferably PG¹ can be selected from any of the following *N-*protecting groups: acetamide (Ac), trifluoroacetamide, *tert*-butyl carbamate (Boc), benzyl carbamate (Cbz), trityl (Trt), dimethoxy-trityl (DMT), toluenesulfonyl (Ts), o-nitro benzenesulfonyl (Ns), 9-fluorenmethyl carbamate (Fmoc), *p*-methoxy benzyl carbamate (Moc), benzoyl (Bz), 3,4-dimethoxybenzyl (DMPM), *p*-methoxybenzyl (PMB), p-methoxyphenyl (PMP), and trichloroethyl carbamate (Troc). More preferably PG¹ can be selected from one of the following *N*-protecting groups with preferred compatibility: acetamide (Ac), trifluoroacetamide, *tert-butyl* carbamate (Boc), trityl (Trt), dimethoxy-trityl (DMT), 9-fluorenmethyl carbamate (Fmoc), and trichloroethyl carbamate (Troc). Most preferably PG¹ can be selected from of following *N*-protecting groups with preferred compatibility and accessibility: acetamide (Ac), and *tert*-butyl carbamate (Boc).

Oxo-sulfur substitution can be generally achieved by any substitution reaction using any sulfur nucleophile and any activation method for alcohols. Preferably, oxo-sulfur substitution can be achieved by using (1) one of the following nucleophiles: thioacetic acid (HSAc) or its sodium/potassium salt (Na/KSAc), thiobenzoic acid (HSBz) or its sodium/potassium salt (Na/KSBz), p-nitro-thiobenzoic acid or its sodium/potassium salt, *tert*-butyl mercaptan (HS-tBu), benzyl mercaptan (HS-Bn), p-methoxybenzyl mercaptan (HS-PMB), methanethiol (HS-Me) or any alkyl thiol (HS-alkyl); and (2) one of the following activation reagents: *p-*toluenesulfonyl chloride (TsCI), methanesulfonyl chloride (MsCI), trifluoromethanesulfonic anhydride (Tf₂O), dialkyl azodicarboxylate activated with a trialkyl- or triarylphosphine (Mitsunobu conditions: e.g. PPh₃/DIAD), tetrahalogenmethane activated with a trialkyl- or triarylphosphine (PPh₃/CBr₄). More preferably oxo-sulfur substitution can be achieved by using mesylation/tosylation followed by potassium thioacetate in two separate steps or using Thio-Mitsunobu conditions (PPh₃/DIAD followed by thioacetic acid, thiobenzoic acid, or p-nitro-thiobenzoic acid in one step). Most preferably oxo-sulfur substitution can be achieved by using Thio-Mitsunobu conditions of PPh₃/DIAD followed by thioacetic acid in one step.

Depending on the selected conditions for oxo-sulfur substitution PG² can be selected from the following sulfur protecting groups: acetate (Ac), benzoate (Bz), *p*-nitro benzoate, *tert*-butyl, methyl, alkyl or p-methoxy benzyl. More preferably, PG² can be selected from the following sulfur protecting groups: acetate (Ac), benzoate (Bz), p-nitro benzoate. Most preferably, PG² is acetate (Ac).

Deprotection of **A2** can generally be achieved applying any appropriate deprotection conditions, which depending on PG¹ and/or PG² may be selected from the following list of general deprotection conditions: (1) acidic deprotection: solutions of HCI in organic solvents (HCI/MeOH, HCI/EtOH, HCI/dioxane, HCl/Et₂O), or carboxylic acids (trifluoroacetic acid (TFA), trichloroacetic acid); (2) basic deprotection: piperidine, NaOH, KOH, Na₂CO₃, K₂CO₃, or Cs₂CO₃; (3) oxidative deprotection: 1,2-dichloro-4,5-dinitro-quinone (DDQ), cerium(IV) ammonium nitrate (CAN), tetrapropylammonium perruthenate, potassium perruthenate; (4) reductive deprotection: hydrogenolysis (H₂ in presence of Pd/C), diisobutylaluminium hyride (DIBAL-H), Na/NH₃, Li/naphthalene, Zn/HCI, LiAlH₄, NaBH₄, NaCNBH₃. Preferred conditions are: (1) acidic deprotection: solutions of HCI in organic solvents (HCI/MeOH, HCI/EtOH, HCI/dioxane, HCl/Et₂O) or carboxylic acids (trifluoroacetic acid (TFA), trichloroacetic acid); (2) basic deprotection: piperidine, KOH, or K₂CO₃. More preferably, acidic deprotection conditions are HCI/dioxane and basic deprotection conditions are KOH.

Deprotection conditions depending on PG¹ and/or PG² can be selected, so that PG¹ and PG² are cleaved consecutively giving the free dithiol **A3** with a free amine, or so that only PG² is cleaved giving the free dithiol **A3** while leaving the *N*-protecting group PG¹ intact.

Oxidative ring closure (formation of the disulfide) can be accomplished by any 2-electron oxidation conditions suitable for dithiol/disulfide oxidation to give disulfide **A4.** Preferably these are selected from the following reaction conditions: I₂/MeOH, I₂/EtOH, I₂/DCM, O₂/MeOH, O₂/EtOH, O₂/DCM, DMSO. More preferably these are selected from the following conditions: I₂/MeOH or I₂/DCM.

Deprotection of **A4** can generally be achieved applying any appropriate deprotection conditions to give **A5,** which depending on PG¹ may be selected from the List of General Deprotection Conditions outlined above.

**A5** is obtained as a free amine or any acceptable ammonium salt of the same which may be selected from, but not limited to the following: acetate, benzoate, bromide, chloride, formate, iodide, nitrate, sulfate, or trifluoroacetate salt.

In other embodiments, general manufacturing of the invention can begin from a diol precursor similar to diol **A32,** which itself can be prepared from commercially available starting materials using literature known procedures. The four steps of oxo-sulfur substitution to yield **A33,** deprotection to yield A34, oxidative ring closure to yield **A35,** and deprotection to yield **A36,** can proceed by the reagents and conditions outlined above.

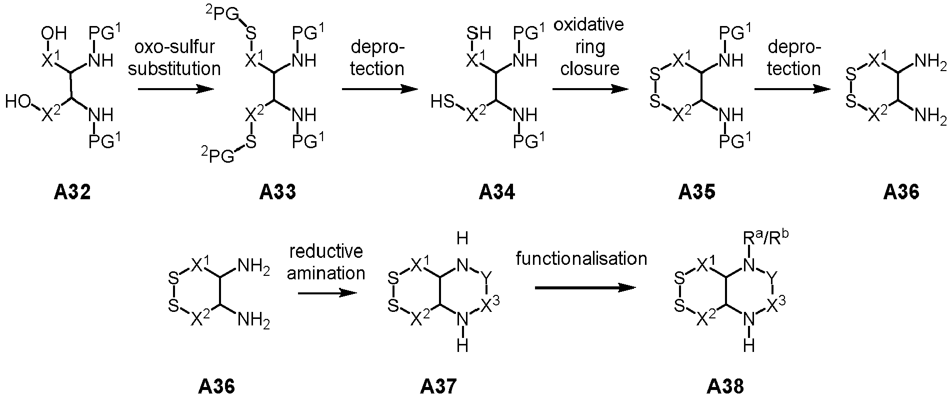

Reductive amination of **A36** can generally be achieved by any appropriate conditions for reductive amination. Depending on the nature of X³ and Y, 6-membered piperazine or 7-membered 1,4-diazepane ring structures can be prepared by applying the respective optionally substituted dialdehyde, diketone, or ketoaldehyde precursors, which may be selected, but are not limited to the following: glyoxal, malondialdehyde, diacetyl and pentane-2,4-dione, or technically acceptable formulations which release them *in situ.* For reduction of the intermediate cyclic diimine structure any reducing agent may be used. Preferably, a reducing agent may be selected from but is not limited to: LiAlH₄, LiBH₄, NaBH₄, NaCNBH₃, NaBH(OAc)₃, KBH₄, LiBHEt₃ or Ti(BH₄)₄(OEt₂). More preferably the cyclic diimine structure may be reduced using NaBH₄ or NaCNBH₃, most preferably using NaCNBH₃.

Monofunctionalisation of **A37** to achieve **A38** may be achieved by any *N*-alkylation, *N*-acylation or *N*-sulfonylation reaction with appropriate reagents, optionally with additional use of protecting groups, as necessary, and as known to those skilled in the art. Depending on the nature of R^{a}, **A37** may be treated with commercially available reagents, including but not limited to the following: For acylation: acetyl chloride, pivaloyl chloride, iPrC(O)-Cl, *n*BuC(O)Cl, methyl chloroformate, dimethyl carbamoylchloride; for sulfonylation: methanesulfonyl chloride; for alkylation: 3-(dimethylamino)propyl chloride, 3-(piperidin-1-yl)propyl chloride, 4-(azetidin-1-yl) butyl chloride, 1-(4-chlorobutyl)azetidine, 1-(3-chloropropyl)pyrrolidine, 2-(2-chloroethoxy)ethan-1-ol, methyl iodide, ethyl iodide. Depending on the nature of R^{b}, **A37** may be coupled with a proteinogenic amino acid to prepare a monopeptide thereof. Peptide coupling may be achieved by any appropriate coupling conditions for coupling of secondary amines with carboxylic acids. Preferably, peptide coupling may be achieved by using one of the following coupling reagents: DCC, DIC, EDCI, HATU, HBTU, PyBOP or 1-hydroxybenzotriazole. Preferably, peptide coupling may be achieved by using DCC or EDCI, most preferably by using EDCI.

### B) General manufacturing: compounds with no self-immolative linker

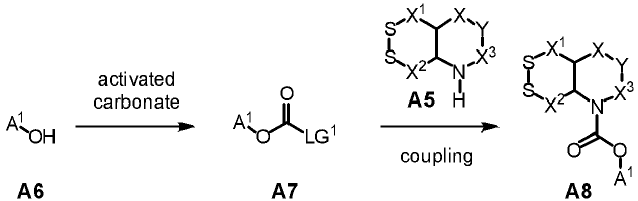

The phenolic structure **A6** represents a phenolic substructure of a motif which may be a therapeutic, diagnostic or theranostic cargo. Transformation of the phenolic alcohol function of **A6** into an activated carbonate derivative may be achieved by any appropriate method for the transformation of alcohols into activated carbonate derivatives.

Methods for the transformation of alcohols into activated carbonate derivatives include using one of the following reagents: phosgene, diphosgene (DP), triphosgene (TP), 1,1'-carbodiimidazole (CDI), *N*,*N'*-disuccinimidyl carbonate (DSC), di-2-pyridyl carbonate (DPC), dimethyl carbonate (DMC), bis(*p*-nitrophenyl) carbonate (DNPC), bis (*p*-chlorophenyl) carbonate (DCPC), phenyl chloroformate, *p*-nitrophenyl chloroformate, *p*-chlorophenyl chloroformate, or ethylene carbonate with chlorine. Preferably, the reagent is DP, TP, CDI, or p-chlorophenyl chloroformate. More preferably, the reagent is TP.

LG¹ depends on the selected method for the transformation of the phenolic alcohol function of **A6** into an activated carbonate derivative and preferably may be one of the following: chloro, *N*-imidazolyl, *N'*-methyl-*N*-imidazoliumyl, succinimidyl, 2-pyridyl, methoxy, p-nitrophenyloxy, p-chlorophenyloxy, or phenyloxy. More preferably, LG¹ may be one of the following: chloro, N-imidazolyl, or p-chlorophenyloxy. Most preferably, LG¹ is chloro.

The activated carbonate derivative **A7** is coupled with the free amine **A5** or any acceptable ammonium salt of the same by applying any appropriate Reagent Suitable for Carbamate Coupling, which may be selected from the following: a trialkylamine (such as NMe₃, NEt₃, diisopropylethylamine (DIPEA), isopropyldiethylamine, triisopropylamine), pyridine, or4-(N,N-dimethylamino)pyridine (DMAP); preferably, NEt₃ or DIPEA.

### C) General manufacturing: compounds including a benzylic self-immolative linker

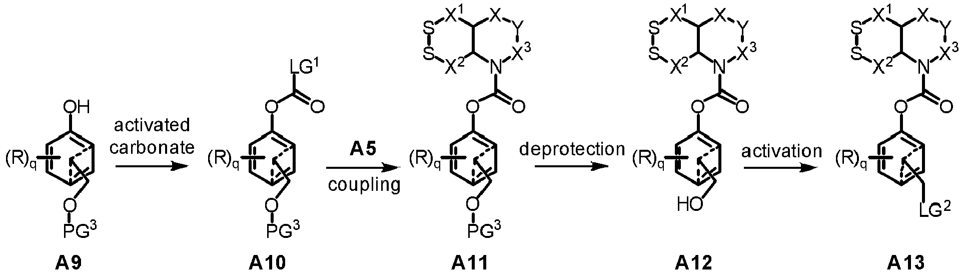

**A9** is used as a precursor to introduce an appropriate self-immolative spacer for the release of phenolic cargos. The attachment point at the aromatic system can either be in *para* (1,4) or *ortho* (1,2) position relative to the phenolic alcohol. **A9** can be prepared from e.g. commercially available 2-hydroxybenzyl alcohol or 4-hydroxybenzyl alcohol using literature known methods. PG³ represents any protecting group for the benzylic alcohol, which intermediately masks the oxygen's nucleophilic reactivity and can be cleaved without affecting other functional groups in the molecule. Preferably PG³ may be one of the following O-protecting groups: methoxymethyl ether (MOM), tetrahydropyranyl ether (THP), *tert*-butyl ether (tBu), allyl ether, benzyl ether (Bn), *tert*-butyldimethylsilyl ether (TBS), *tert*-butyldiphenylsilyl ether (TBDPS), benzoate (Bz), acetate (Ac), pivalate (Piv). More preferably, PG³ can be selected from any of the following O-protecting groups: MOM, THP, tBu, TBS.

Transformation of the phenolic alcohol function of **A9** into an activated carbonate derivative **A10** may be achieved by any appropriate method for the transformation of alcohols into activated carbonate derivatives, as outlined above. LG¹ depends on the selected method, as outlined above.

The activated carbonate **A10** is coupled with the free amine **A5** or any acceptable ammonium salt of the same by applying any reagent suitable for carbamate coupling, as outlined above, yielding **A11.**

Deprotection of **A11** can generally be achieved by applying any appropriate deprotection conditions to give **A12,** which depending on PG³ may be selected from the General Deprotection Conditions as outlined above or from organic hydrogen fluoride solutions or any other hydrogen fluoride or fluoride source (HF/pyridine, alkali fluorides, alkaline earth fluorides, tetraalkylammonium fluorides). Preferred conditions are: organic hydrogen fluoride solutions or any other hydrogen fluoride or fluoride source (HF/pyridine, alkali fluoride, alkaline earth fluoride, tetraalkylammonium fluoride such as TBAF). More preferred are HF/pyridine or TBAF.

Activation of the benzylic alcohol **A12** for nucleophilic substitution may be achieved by any activation method for alcohols. Preferably, this may be achieved using an activation reagent selected from the following list of Reagents for the Activation of Benzylic Alcohols: *para-*toluenesulfonyl chloride (TsCI), methanesulfonyl chloride (MsCI), trifluoromethanesulfonic anhydride (Tf₂O), tetrachloromethane or tetrabromomethane or iodine activated with a trialkylphosphine or a triarylphosphine (e.g. PPh₃/CBr₄), thionyl chloride (SOCl₂), phosphoryl chloride (POCl₃), hydrochloric acid (HCI), or hydrobromic acid (HBr). Preferred are *p-*toluenesulfonyl chloride (TsCI), methanesulfonyl chloride (MsCI), thionyl chloride (SOCl₂), phosphoryl chloride (POCl₃), hydrochloric acid (HCI) or hydrobromic acid (HBr). Most preferred are methanesulfonyl chloride (MsCI) or hydrobromic acid (HBr).

Depending on the selected conditions for the activation of the benzylic alcohol, LG² in **A13** is any acceptable leaving group suitable for nucleophilic substitution, such as -CI, -Br, -I, *para-*toluenesulfonate (OTs), methylsulfonate (OMs), or trifluoromethylsulfonate (OTf). Preferably, LG² is -Br, -CI, or OMs. More preferably, LG² is -Br.

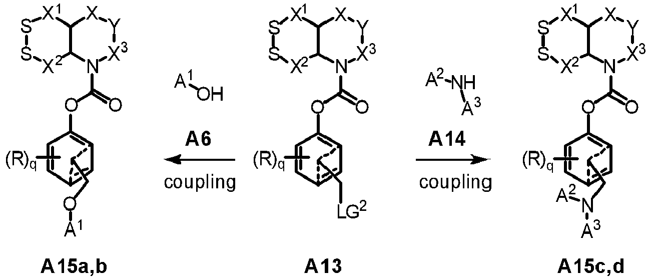

A¹ is selected, so that **A6** is a therapeutic, diagnostic or theranostic cargo of which the -OH group in A¹-OH is a phenolic or aromatic hydroxyl group. A² and A³ are selected, so that **A14** is a therapeutic, diagnostic or theranostic cargo of which the -NH- group in A²-NH-A³ is an amine or aniline group.

The reaction of **A13** with alcohol **A6** yielding benzylic ethers **A15a,b,** or the reaction of **A13** with amine **A14** yielding benzylic amines **A15c,d,** can be achieved by any appropriate method for nucleophilic substitution, which includes the use of appropriate reagents for nucleophilic substitution, which may be selected from the following: (1) alkali or alkaline earth hydrides (e.g. NaH, CaH₂), amides (e.g. NaNH₂, LDA, LHMDS, KHMDS), alkoxides (e.g. KO*t*Bu, NaOMe), hydroxides (e.g. NaOH, KOH), or carbonates (e.g. K₂CO₃, Cs₂CO₃); (2) trialkylamines (e.g. NMe₃, NEt₃, DIPEA, isopropyldiethylamine, triisopropylamine) or 4-dimethylaminopyridine (DMAP). Preferred are hydrides (NaH, CaH₂), hydroxides (NaOH, KOH), or carbonates (K₂CO₃, Cs₂CO₃). More preferred are NaH, KOH or K₂CO₃.

Alternatively, preparation of benzylic ethers **A15a,b** can be achieved from reaction of **A6** with **A12** using any appropriate conditions effecting *in situ* activation of the benzylic alcohol, including using dialkyl azodicarboxylate activated with a trialkylphosphine or triarylphosphine (Mitsunobu conditions: e.g. PPh₃/DIAD).

### D) General manufacturing: compounds including a benzyloxycarbonyl self-immolative linker

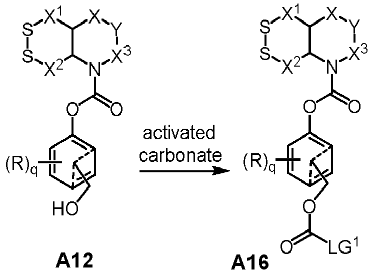

**A12** is also used to introduce a benzyloxycarbonyl self-immolative spacer for the release of amine/aniline cargos. Transformation of the benzylic alcohol function of **A12** into the activated carbonate **A16** may be achieved by any of the methods for the transformation of alcohols into activated carbonate derivatives outlined above. LG¹ depends on the selected method, as outlined above.

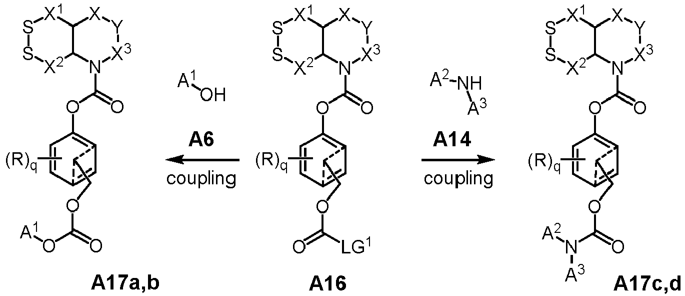

The activated carbonate **A16** is coupled with aromatic alcohol **A6** to yield **A17a,b,** or is coupled with amine **A14** or any acceptable ammonium salt of the same to yield **A17c,d,** by applying any Reagent Suitable for Carbamate Coupling as outlined above.

### Pharmaceutical or diagnostic compositions

The compounds of formula (I) can be administered to a patient in the form of a pharmaceutical or diagnostic composition which can optionally comprise one or more pharmaceutical carrier(s) or excipients.

The compounds of formula (I) can be administered by various well known routes, including oral, rectal and parenteral administration, e.g. intravenous, intramuscular, intradermal, subcutaneous, topical, and similar administration routes. Parenteral, oral and topical administration are preferred, particularly preferred is intravenous administration.

Particular preferred pharmaceutical or diagnostic forms for the administration of a compound of formula (I) are forms suitable for injectable use and include sterile aqueous solutions or dispersions and sterile powders for the extemporaneous preparation of sterile injectable solutions or dispersion. In all cases the final solution or dispersion form must be sterile and fluid. Typically, such a solution or dispersion will include a solvent or dispersion medium, containing, for example, water buffered aqueous solutions, e.g. biocompatible buffers, ethanol, polyol, such as glycerol, propylene glycol, polyethylene glycol, suitable mixtures thereof, surfactants or vegetable oils. A compound of the invention can also be formulated into liposomes, in particular for parenteral administration. Liposomes provide the advantage of increased half life in the circulation, if compared to the free drug and a prolonged more even release of the enclosed drug.

Sterilization of infusion or injection solutions can be accomplished by any number of art recognized techniques including but not limited to addition of preservatives like anti bacterial or anti-fungal agents, e.g. parabene, chlorobutanol, phenol, sorbic acid or thimersal. Further, isotonic agents, such as sugars or salts, in particular sodium chloride may be incorporated in infusion or injection solutions.

Production of sterile injectable solutions containing one or several of the compounds of the invention is accomplished by incorporating the respective compound in the required amount in the appropriate solvent with various ingredients enumerated above as required followed by sterilization. To obtain a sterile powder the above solutions are vacuum dried or freeze dried as necessary. Preferred diluents of the present invention are water, physiologically acceptable buffers, physiologically acceptable buffer salt solutions or salt solutions. Preferred carriers are cocoa butter and vitebesole. Excipients which can be used with the various pharmaceutical or diagnostic forms of a compound of the invention can be chosen from the following non limiting list:
a) binders such as lactose, mannitol, crystalline sorbitol, dibasic phosphates, calcium phosphates, sugars, microcrystalline cellulose, carboxymethyl cellulose, hydroxyethyl cellulose, polyvinyl pyrrolidone and the like;
b) lubricants such as magnesium stearate, talc, calcium stearate, zinc stearate, stearic acid, hydrogenated vegetable oil, leucine, glycerids and sodium stearyl fumarates,
c) disintegrants such as starches, croscaramellose, sodium methyl cellulose, agar, bentonite, alginic acid, carboxymethyl cellulose, polyvinyl pyrrolidone and the like.

In one embodiment the formulation is for oral administration and the formulation comprises one or more or all of the following ingredients: pregelatinized starch, talc, povidone K 30, croscarmellose sodium, sodium stearyl fumarate, gelatin, titanium dioxide, sorbitol, monosodium citrate, xanthan gum, titanium dioxide, flavoring, sodium benzoate and saccharin sodium.

Other suitable excipients can be found in the Handbook of Pharmaceutical Excipients, published by the American Pharmaceutical Association, which is herein incorporated by reference.

The pharmaceutical or diagnostic compositions comprising a compound of the invention can be produced in a manner known per se to the skilled person as described, for example, in Remington's Pharmaceutical Sciences, 15th Ed., Mack Publishing Co., New Jersey (1991).

It is to be understood that depending on the severity of the disorder and the particular type which is treatable with one of the compounds of the invention, as well as on the respective patient to be treated, e.g. the general health status of the patient, etc., different doses of the respective compound are required to elicit a therapeutic or prophylactic effect.

The cargo is, as defined above, any therapeutic, diagnostic or theranostic agents. Depending on the therapeutic, diagnostic or theranostic agent, the mode of administration, amongst other factors, the appropriate dose spans a wide range. The determination of the appropriate dose lies within the discretion of the attending physician.

If desired, the pharmaceutical or diagnostic composition can comprise a second pharmaceutically active agent. The second pharmaceutically active agent is not limited and can be selected from a vascular disrupting agent, a cytotoxic chemotherapeutic agent and an immunomodulator. Examples thereof include combretastatin A-4 (CA4), 3'-aminocombretastatin A-4, BNC105, ABT-751, ZD6126, combretastatin A-1, or prodrugs of the same (which includes but is not limited to combretastatin A-4 phosphate (CA4P), 3'-aminocombretastatin A-4 3'-serinamide (ombrabulin), combretastatin A-1 bisphosphate (CA1P), and BNC105 phosphate (BNC105P)), and pharmaceutically acceptable salts, solvates or esters of the same.

### Mechanism of Reductive Release

The mechanism by which reductive cleavage of the disulfide leads to release of the cargo agent, is shown in the following schemes depicting the release of H-O-A¹ (phenol-type) or H-N(A²)(A³) (amine-type) cargos from compounds of the invention. Without wishing to be bound by theory, it is assumed that the reduction of the disulfide bond of the compounds of the invention could, in cells, occur by a 2-step transthiolation/thiol-resolution process performed by a reducing enzyme or protein with a dithiol or selenothiol active site, for example, a disulfide reductase or its effector disulfide protein such as thioredoxin, thioredoxin reductase, or glutathione reductase.
1. When L is a bond: 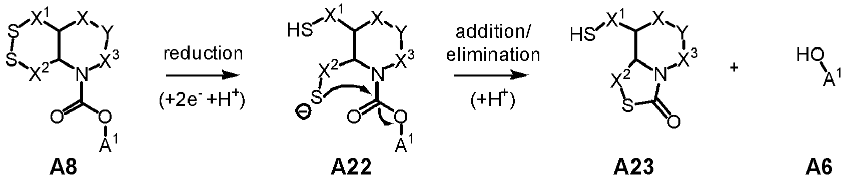 When L is a bond, the phenolic cargo (A¹)-OH is directly linked to A as a carbamate, giving compounds of type **A8.** Upon reduction of the disulfide bond in **A8,** the reduced thiol/thiolate species **A22** is formed. Depending on the redox microenvironment and local pH **A22** can intramolecularly cyclise *via* an addition/elimination mechanism, which irreversibly releases the free phenolic cargo (i.e. therapeutic, diagnostic or theranostic agent) H-O-A¹ **(A6)** as well as the byproduct carbamothioate species **A23.**
2. When L is a benzylic (1,4)- or (1-6)-self-immolative linker which is linked to A as a phenolic carbamate, and is connected to B as a benzylic ether or benzylamine 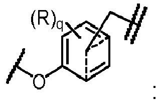 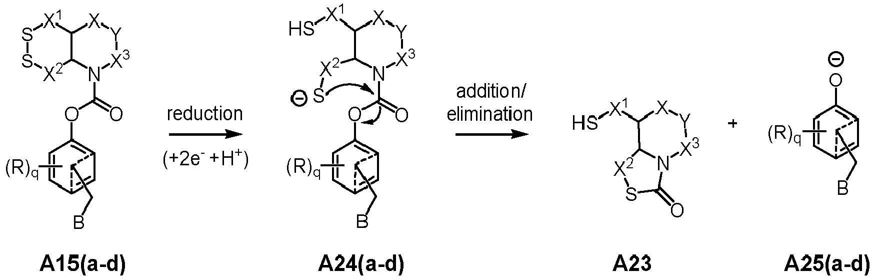 General Mechanism: When L is a benzylic (1,4)- or (1,6)-self-immolative linker which is linked to A as a carbamate, and is connected to B as a benzylic ether or benzylamine, then B can be selected from phenol-type (A¹)-OH or amine-type HN(A²)(A³) cargos, giving compounds **A15(a-d)** as defined below. Upon reduction of the disulfide bond in **A15(a-d),** the corresponding reduced thiol/thiolate species **A24(a-d)** is formed. Depending on the redox microenvironment and local pH **A24(a-d)** can intramolecularly cyclise *via* an addition/elimination mechanism, which irreversibly releases the corresponding intermediate phenolic species **A25(a-d)** as well as carbamothioate byproduct **A23.** The nature of compounds **A15(a-d)** and how the corresponding intermediate **A25(a-d)** releases the cargo B are shown below:
   When L is the (1,6)-self-immolative linker 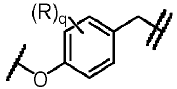 and B is the phenolic cargo 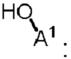 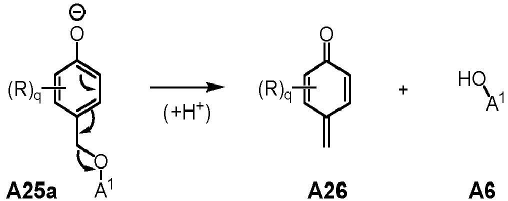 In compounds of type **A15a,** L is a benzylic (1,6)-self-immolative linker and B is a phenolic (A¹)-OH cargo which is connected to the linker as a benzylic ether. Reduction of **15a** gives **24a** which after cyclisation gives intermediate **A25a. A25a** can perform an intramolecular 1,6-elimination releasing the quinone-methide species **A26** and the free phenolic cargo **A6.**
   When L is the (1,4)-self-immolative linker 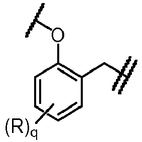 and B is the phenolic cargo 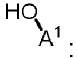 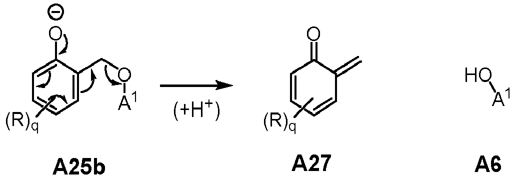 In compounds of type **A15b,** L is a benzylic (1,4)-self-immolative linker and B is a phenolic (A¹)-OH cargo, which is connected to the linker as a benzylic ether. Reduction of **15b** gives **24b** which after cyclisation gives intermediate **A25b. A25b** can perform an intramolecular 1,4-elimination releasing the quinone-methide species **A27** and the free phenolic cargo **A6.**
   When L is the (1,6)-self-immolative linker' 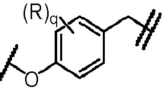 and B is the amine-type cargo 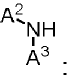 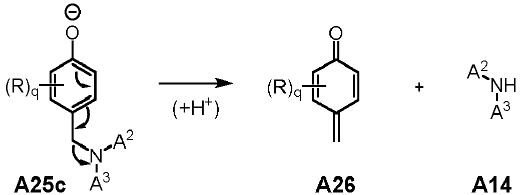 In compounds of type **A15c,** L is a benzylic (1,6)-self-immolative linker and B is an amine-type HN(A²)(A³) cargo, which is connected to the linker as a benzylamine. Reduction of **15c** gives **24c** which after cyclisation gives intermediate **A25c. A25c** can follow an intramolecular 1,6-elimination releasing the quinone-methide species **A26** and the free amine-type cargo (i.e., therapeutic, diagnostic or theranostic agent) **A14.**
   When L is the (1,4)-self-immolative linker 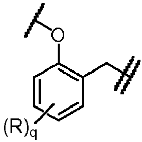 and B is the amine-type cargo 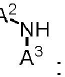 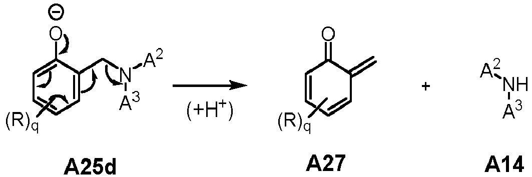 In compounds of type **A15d,** L is a benzylic (1,4)-self-immolative linker and B is an amine-type HN(A²)(A³) cargo, which is connected to the linker as a benzylamine. Reduction of **15d** gives **24d** which after cyclisation gives intermediate **A25d. A25d** can perform an intramolecular 1,6-elimination releasing the quinone-methide species **A27** and the free amine-type cargo **A14.**
3. When L is a benzylic (1,4)- or (1-6)-self-immolative linker which is linked to A as a phenolic carbamate, and is connected to B as a benzylic carbonate or carbamate

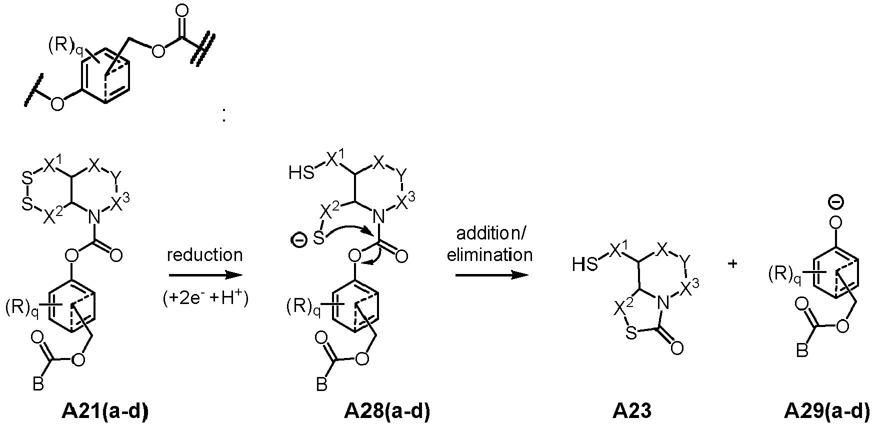

General Mechanism: When L is a benzylic (1,4)- or (1,6)-self-immolative linker which is linked to A as a carbamate, and is connected to B as a benzylic carbonate or carbamate, then B can be selected from phenol-type (A¹)-OH or amine-type HN(A²)(A³) cargos, giving compounds **A21(a-d)** as defined below. Upon reduction of the disulfide bond in **A21(a-d),** the corresponding reduced thiol/thiolate species **A28(a-d)** is formed. Depending on the redox microenvironment and local pH **A28(a-d)** can intramolecularly cyclise *via* an addition/elimination mechanism, which irreversibly releases the corresponding intermediate phenolic species **A29(a-d)** as well as carbamothioate byproduct **A23.** The nature of compounds **A21(a-d)** and how the corresponding intermediate **A29(a-d)** releases the cargo B are shown below:

When L is the (1,6)-self-immolative linker 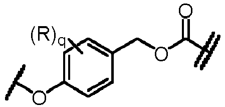 and B is the phenolic cargo

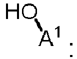

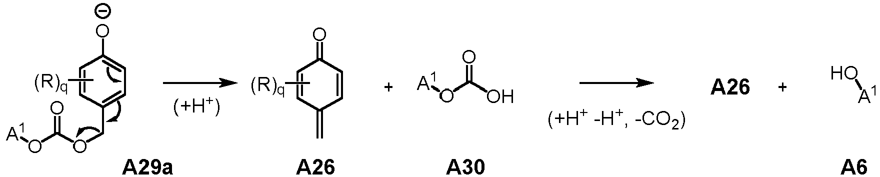

In compounds of type **A21a,** L is a benzylic (1,6)-self-immolative linker and B is a phenolic (A¹)-OH cargo which is connected to the linker as a benzylic carbonate. Reduction of **21a** gives **28a** which after cyclisation gives intermediate **A29a. A29a** can perform an intramolecular 1,6-elimination releasing the quinone-methide species **A26** as well as the carbonic acid species **A30** which further eliminates carbon dioxide to give the free phenolic cargo **A6.**

When L is the (1,4)-self-immolative linker 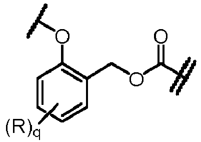 and B is the phenolic cargo

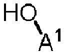

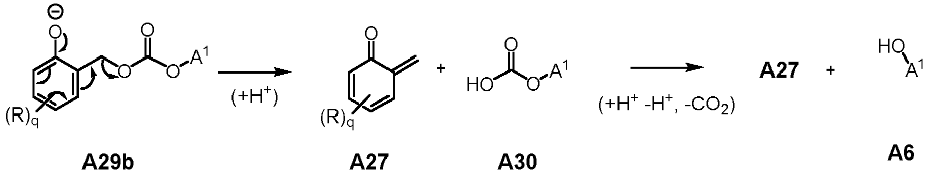

In compounds of type **A21b,** L is a benzylic (1,4)-self-immolative linker and B is a phenolic (A¹)-OH cargo which is connected to the linker as a benzylic carbonate. Reduction of 21b gives **28b** which after cyclisation gives intermediate **A29b. A29b** can perform an intramolecular 1,4-elimination releasing the quinone-methide species **A27** as well as the carbonic acid species **A30** which further eliminates carbon dioxide to give the free phenolic cargo **A6.**

When L is the (1,6)-self-immolative linker 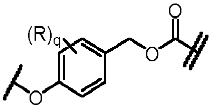 and B is the amine-type cargo

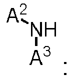

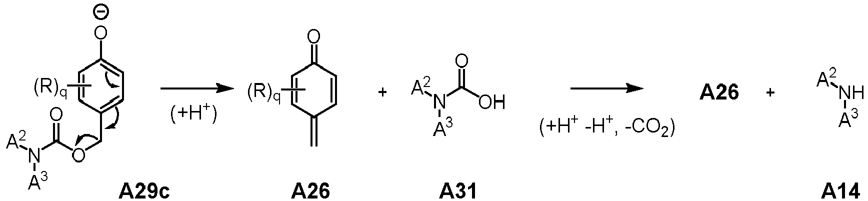

In compounds of type **A21c,** L is a benzylic (1,6)-self-immolative linker and B is an amine-type cargo HN(A²)(A³) which is connected to the linker as a benzylic carbamate. Reduction of **21c** gives **28c** which after cyclisation gives intermediate **A29c. A29c** can perform an intramolecular 1,6-elimination releasing the quinone-methide species **A26** as well as the carbamic acid species **A31** which further eliminates carbon dioxide to give the free phenolic cargo **A14.**

When L is the (1,4)-self-immolative linker 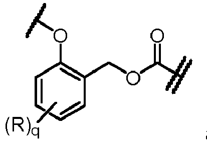 and B is the amine-type cargo

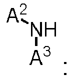

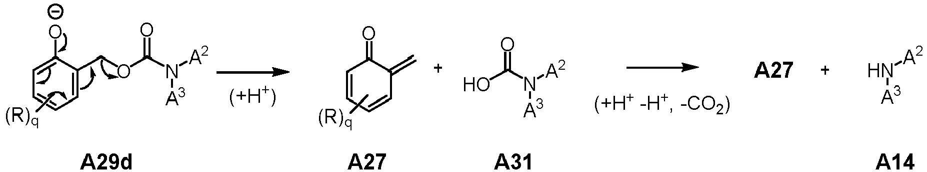

In compounds of type **A21d,** L is a benzylic (1,4)-self-immolative linker and B is an amine-type cargo HN(A²)(A³) which is connected to the linker as a benzylic carbamate. Reduction of **21d** gives **28d** which after cyclisation gives intermediate **A29d. A29d** can perform an intramolecular 1,4-elimination releasing the quinone-methide species **A27** as well as the carbamic acid species **A31** which further eliminates carbon dioxide to give the free phenolic cargo **A14.** Without wishing to be bound by theory, it is assumed that the specific bicyclic structure common to the presently claimed compounds can ensure that the disulfide bond is stabilized against nonspecific reduction.

It is furthermore assumed that in some of the claimed compounds this stabilised disulfide is reductively cleaved selectively by certain reductants, such as one or more oxidoreductases or redox effector proteins, and therefore that cargo release can be used as a quantification method for the activity of those reductants, which is a goal of probe development in diagnostics and in research.

It is furthermore assumed that in some of the claimed compounds this stabilised disulfide is reductively cleaved selectively in cells with dysregulated redox properties (including cells located in hypoxic environments, and cells with upregulated oxidoreductases) such as tumor cells and cells located in inflammatory microenvironments. Therefore, the effect of the agent that is released after reductive cleavage can be localized to pathological cells and environments which ensures that healthy cells and tissues are not exposed to similar levels of the agent. This pathology-specificity allows the presently claimed compounds which include a therapeutic or theranostic agent to be used as therapeutics for pathological cells and environments; and allows the presently claimed compounds which include a diagnostic or theranostic agent to be used as diagnostics for pathological cells and environments. Such selectivity is a longstanding goal of prodrug and diagnostic development, and offers a significant advantage compared to prior art disulfide-reduction-triggered compound designs which are activated by typical cellular conditions without specificity for pathological cells (see e.g. Hyman, L. M. et al. Coordination Chemistry Reviews 2012, 256, 2333-2356. https://doi.org/10.1016/j.ccr.2012.03.009).

### Utility, Diseases, and Examples

The compounds having the formula (I) are particularly useful as therapeutics or diagnostics.

The compounds having the formula (I) can be used in the diagnosis, treatment, amelioration or prevention of a disorder selected from a neoplastic disorder; atherosclerosis; an autoimmune disorder; an inflammatory disease; a chronic inflammatory autoimmune disease; ischaemia; and reperfusion injury.

Preferably, the disorder is a neoplastic disorder, in particular cancer. The cancer is not particularly limited, preferably the cancer is selected from acoustic neuroma, adenocarcinoma, angiosarcoma, basal cell carcinoma, bile duct carcinoma, bladder carcinoma, breast cancer, bronchogenic carcinoma, cervical cancer, chondrosarcoma, chordoma, choriocarcinoma, craniopharyngioma, cystadenocarcinoma, embryonal carcinoma, endotheliosarcoma, ependymoma, epithelial carcinoma, Ewing's tumor, fibrosarcoma, hemangioblastoma, leiomyosarcoma, liposarcoma, Merkel cell carcinoma, melanoma, mesothelioma, myelodysplastic syndrome, myxosarcoma, oligodendroglioma, osteogenic sarcoma, ovarian cancer, pancreatic cancer, papillary adenocarcinomas, papillary carcinoma, pinealoma, prostate cancer, renal cell carcinoma, retinoblastoma, rhabdomyosarcoma, sebaceous gland carcinoma, seminoma, squamous cell carcinoma, sweat gland carcinoma, synovioma, testicular tumor, Wilms' tumor, adrenocortical carcinoma, urothelial carcinoma, gallbladder cancer, parathyroid cancer, Kaposi sarcoma, colon carcinoma, gastrointestinal stromal tumor, anal cancer, rectal cancer, small intestine cancer, brain tumor, glioma, glioblastoma, astrocytoma, neuroblastoma, medullary carcinoma, medulloblastoma, meningioma, leukemias including acute myeloid leukemia, multiple myeloma, acute lymphoblastic leukemia, liver cancer including hepatoma and hepatocellular carcinoma, lung carcinoma, non-small-cell lung cancer, small cell lung carcinoma, lymphangioendotheliosarcoma, lymphangiosarcoma, primary CNS lymphoma, non-Hodgkin lymphoma, and classical Hodgkin's lymphoma.

The therapeutic, diagnostic or theranostic compounds having the formula (I) can be used in combination with one or more other therapeutics, diagnostics or theranostics. The type of the other therapeutics, diagnostics or theranostics is not particularly limited and will depend on the disorder to be treated and/or diagnosed. Preferably, other therapeutics can enhance the selectivity and/or the turnover of the compound of formula (I) in the targeted tissue type; for example, the other therapeutic may be an agent that can stimulate transient hypoxia in tumors and thereby enhance the tumor-specific release of the cargo of the compound of formula (I) (which may be a therapeutic, diagnostic, or theranostic). Preferably in the case of compounds of formula (I) which are therapeutics and theranostics releasing a cargo used to treat a disorder, the other medicaments will be further therapeutics which are used to treat the same disorder; for example, further therapeutics that can treat a neoplastic disorder such as cancer may be applied in combination with compounds of formula (I) that are therapeutic proagents releasing a cargo that can treat a neoplastic disorder such as cancer.

The one or more other medicament, diagnostic or theranostic can be administered prior to, after, or simultaneously with the compound having formula (I). In another preferred embodiment, the other medicament can be an agent that can stimulate transient hypoxia in tumors which can be administered prior to, after or simultaneously to administration of compound of formula (I).

Agents which are capable of stimulating hypoxia in diseased tissues include but are not limited to vascular disrupting agents (including colchicine domain tubulin inhibitors such as combretastatin A-4 (CA4), 3'-aminocombretastatin A-4, BNC105, ABT-751, ZD6126, combretastatin A-1, or prodrugs of the same (which includes but is not limited to combretastatin A-4 phosphate (CA4P), 3'-aminocombretastatin A-4 3'-serinamide (ombrabulin), combretastatin A-1 bisphosphate (CA1P), and BNC105 phosphate (BNC105P), and pharmaceutically acceptable salts of the same), vascular-acting drugs such as vasodilator or antihypertensive drugs (including hydralazine), and inhibitors of prolyl hydroxylase or stabilizers of hypoxia-inducible factor 1 such as daprodustat, desidustat and molidustat.

For *in vivo* usage, a diagnostic compound of the invention is administered to a subject or patient by any acceptable route, preferably *i.v., i.p.* or *per* os, and the subject or patient is imaged thereafter according to the imaging modality of the cargo, preferably by fluorescence imaging, photoacoustic imaging, luminescence imaging, or positron emission tomography. For usage *in vitro,* a diagnostic compound of the invention is administered to a test sample which, for instance, has been obtained from a subject, and the test sample is imaged thereafter according to the imaging modality of the cargo, preferably by fluorescence imaging, absorbance, or luminescence imaging; the sample may preferably be cells, a tissue section, a small animal model or a biopsy sample.

The compounds of the present invention can be used to predict the suitability of a compound of the invention for treating a patient who is suffering from a disorder selected from a neoplastic disorder; atherosclerosis; an autoimmune disorder; an inflammatory disease; a chronic inflammatory autoimmune disease; ischaemia; and reperfusion injury.

In a first step, a sample of the effected tissue or body fluid is obtained from the patient. The sample is then contacted with a compound (I*) of the present invention, wherein A is A*, L is L* and A¹ is selected such that A¹-OH is a diagnostic or theranostic agent or A² and A³ are independently selected such that A²-NH-A³ is a diagnostic or theranostic agent. Subsequently, the sample is imaged according to the imaging modality of the diagnostic or theranostic agent, for instance, by fluorescence imaging, photoacoustic imaging, luminescence imaging, or positron emission tomography, to determine whether the compound (I*) of the present invention has been reductively cleaved to release A¹-OH or A²-NH-A³. If compound of the present invention has been reductively cleaved then it can be assumed that a compound (I**) of the present invention which has the same moieties A* and L* as the compound (I*) but in which A¹-OH is a therapeutic or theranostic agent or A² and A³ are independently selected such that A²-NH-A³ is a therapeutic or theranostic agent will be effective for treating the patient. The theranostic agent which is used in the compounds (I*) and (II*) can be the same or different.

The extent to which the compound (I*) of the present invention is reductively cleaved to release A¹-OH or A²-NH-A³ is not particularly limited. It should be sufficient to provide a therapeutically effective amount of A¹-OH or A²-NH-A³ to the tissue or body fluid. For instance, 0.1-20 %, preferably 0.5-5 % of the compound (I*) of the present invention can be reductively cleaved.

In the following, examples of preferred compounds having the formula (I) are described, to illustrate some of the ways in which conjugation of a cargo H-B as the compound A-L-B masks its diagnostic and/or therapeutic activity, until the cargo is released following reduction.

These examples include the following therapeutic designs:
1 releases a DNA-alkylating nitrogen mustard cargo subunit of a type which is of interest especially for cancer therapy. The released bis(2-haloethyl)aniline species will feature similar activity to PR-104's bioactive alkylating metabolite PR-104M whereas the intact species 1 will not be as potently alkylating as the released cargo, since unmasking the electron-donating phenol in *para* to the aniline nitrogen increases electronic density and alkylating reactivity (similar principles are known for PR-104 and a range of related prodrugs; Sharma, A. et al. Chem. Soc. Rev. 2019, 48, 771-813. https://doi.org/10.1039/C8CS00304A). Note that a range of leaving groups (halogens -CI, -Br, -I or other leaving groups such as -OS(O)₂CH₃) can be used as the halogen substituents of the 2-haloethyl groups without compromising functionality, and/or an aniline nitrogen (requiring the use of a spacer subunit) could be used instead of the phenolic hydroxyl as the unmasked group of the cargo, while remaining within the scope of a compound having the formula (I).
**4** and **9** are similar to compound **P4** in that they release cargo subunits that are analogues of 5-hydroxy-seco-cyclopropabenzaindole, which (when their phenolic hydroxyl is unmasked) can then undergo Winstein cyclisation to the active DNA-alkylating cyclopropabenzaindoles (CBls), which are of interest especially for cancer therapy, whereas no Winstein cyclisation can take place until the phenol is unmasked. In this way only the released cargo (and not the proagents) will be bioactive (similar principles are known for a range of related prodrugs; Twum, E. A. et al. Bioorganic & Medicinal Chemistry 2015, 23, 3481-3489. https://doi.org/10.1016/j.bmc.2015.04.034). Note that a range of analogues of 5-hydroxy- or 5-amino-seco-cyclopropabenzaindoles or related cargos with various substitution motifs (such as the inclusion of the dimethylamino substituent on **9** to enhance solubility and DNA-binding potency) could be used, while remaining within the scope of a compound having the formula (I).
**11** releases a cargo subunit that is the Amaryllidaceae-type alkaloid pancratistatin. Amaryllidaceae-type alkaloids (such as pancratistatin or narciclasine) are metabolism-affecting drugs that have shown utility in disease indications featuring metabolic deregulation, from cancer to chronic inflammation (Lubahn, C. et al. Rheumatology International 2012, 32, 3751-3760. https://doi.org/10.1007/s00296-011-2217-z; McNulty, J. et al. J. Nat. Prod. 2008, 71, 357-363. https://doi.org/10.1021/np0705460). The cargo release process (following triggering of the disulfide trigger subunit of 11) removes steric hindrance and so allows binding of the lycorane to its molecular target, such that only the released cargo is bioactive. Note that a range of lycorane-type alkaloids with various substitution motifs could be used, while remaining within the scope of a compound having the formula (I).
**14** releases a cargo subunit that is SN-38, the active metabolite of the camptothecin class topoisomerase inhibitor irinotecan, a class which also contains the agent topotecan. As known for irinotecan, blocking the phenol removes bioactivity of the proagent so **14** will be biologically inactive except if triggered to release cargo; note that a range of similar cargo structures (e.g. modifying the ethyl group at the 7-position, or introduction of solubility and potency enhancing groups such as (dimethylamino)methyl as seen in topotecan) could be used, as is known for a range of related prodrugs (Sharma, A. et al. Chem. Soc. Rev. 2019, 48, 771-813. https://doi.org/10.1039/C8CS00304A), while remaining within the scope of a compound having the formula (I).

It is illustrated by **1, 4, 9, 11** and **14** that a broad range of bioactive phenolic cargos with a range of different bioactivity mechanisms and disease indication scopes can therefore be used, including with extensive structural substitutions, within the scope of the invention to deliver cargo-release-based activation of proagent bioactivity, i.e. functional prodrugs dependent upon triggering of the key disulfide of a compound having the formula (I).
**13** releases an amine cargo subunit that is a tubulin-inhibiting auristatin derivative, of particular interest for cancer therapy and for neoangiogenic vasculopathies. A range of auristatins (and related peptide tubulin binders such as dolastatins and tubulysins) have been used in prodrugs where bioactivity is suppressed by conjugation of the agent to a large but cleavable trigger and/or trigger+spacer subunit (Singh, Y. et al. Curr Med Chem 2008, 15, 1802-1826), then restored upon its removal. Note that a range of such agents with various substitution patterns (including monomethyl auristatin E, monomethyl auristatin F, dolastatin or symplostatin) could be used; and/or that beyond the 3-fluoro-4-hydroxybenzyl alcohol spacer depicted in **13,** a range of spacers could instead be used, while remaining within the scope of a compound having the formula (I), to activate cytotoxic activity upon triggering of the proagent.
**15** releases an amine cargo subunit that is of the anthracycline class of topoisomerase-inhibitors. This class includes doxorubicin, daunorubicin and idarubicin, and is of particular interest for cancer therapy. Basicity of the aliphatic amine of doxorubicin and its analogues is important for bioactivity; its carbamylation in **15** blocks bioactivity, but bioactivity can be restored by disulfide triggering followed by spacer-mediated release of the doxorubicin analogue (Singh, Y. et al. Curr Med Chem 2008, 15, 1802-1826). Note that a range of related cargos with various substitution motifs and/or related spacers could instead be used, while remaining within the scope of a compound having the formula (I), to activate cytotoxic activity upon triggering of the proagent.

It is illustrated by **13** and **15** that a broad range of bioactive amine cargos with different bioactivity mechanisms and disease indication scopes can therefore be used in conjunction with a range of spacers, each with optional extensive structural substitutions, while remaining within the scope of the invention to deliver cargo-release-based activation of proagent bioactivity, i.e. functional prodrugs dependent upon triggering of the key disulfide of a compound having the formula (I).

These examples include the following diagnostic designs:
**2** releases a fluorescent precipitating phenolic cargo subunit with large Stokes shift and green emission; **3, 7** and **10** release fluorescent phenolic cargos with small Stokes shifts and UV, cyan, or orange emission; all the proagents **2, 3, 7** and **10** are nonfluorescent due to masking of the phenol groups (either due to blocking excited-state intramolecular proton transfer in **2,** or reducing electron-donating capacity in **3,** or blocking the opening of the spirolactone in **7** and **10);** and all these designs provide a signal turn-on upon cargo release stimulated by triggering of the trigger subunits (and similar principles are known for a range of related probe cargos; Thorn-Seshold, O. et al. Chem. Commun. 2012, 48, 6253-6255. https://doi.org/10.1039/C2CC32227G); they are thus diagnostic fluorescence probes that may be useful in a variety of diagnostic purposes. It is also illustrated that spacer subunits can be optionally included **(7),** and that a range of cargos [e.g. **2** (2-hydroxyphenyl)quinazolin-4(3*H*)-one), **3** (coumarin), **7** and **10** (xanthene fluorophores of the fluorescein type)] with a range of structures and diagnostic properties are possible while remaining within the scope of a compound having the formula (I).
**5** releases a phenolic cargo subunit that is D-luciferin, the substrate of firefly luciferase enzyme, whereas the intact probe **5** is too bulky to be a substrate of luciferase. Therefore **5** is a disulfide-reduction-triggered diagnostic luminescence probe allowing detection by bioluminescence in the presence of luciferase, which may be useful as a diagnostic for reductive probe turnover with ultra-low background signal (high sensitivity). Note that a range of luciferins for different luciferases and with different structural modifications are known and could also be used as cargo subunits (Kaskova, Z. M. et al. Chem. Soc. Rev. 2016, 45, 6048-6077. https://doi.org/10.1039/C6CS00296J) while remaining within the scope of a compound having the formula (I).
**8** releases a phenolic cargo subunit that is a functional analogue of Black Hole Quencher 3 (BHQ3) which has fluorescence quenching and photoacoustic signal generation properties (Chevalier, A. et al. Chem.-Eur. J 2013, 19, 1686-1699. https://doi.org/10.1002/chem.201203427). The proagent **8** is non-signal-generating and non-quenching due to masking of the key electron-donating phenol group, while the released cargo has no masking of the phenol and therefore has fluorescence quenching and photoacoustic signal generation activity, thus **8** functions as a multimodal diagnostic proagent. Note that similar unmasking-based activity turn-on principles are known for a range of related compounds including other quenchers, which could also be used as cargo subunits while remaining within the scope of a compound having the formula (I).
**12** releases a spacer-cargo system that fragments to release an aniline cargo subunit that is a silarhodamine derivative which has fluorescence and photoacoustic signal generation properties (Myochin, T. et al. J. Am. Chem. Soc. 2015, 137, 4759-4765. https://doi.org/10.1021/jacs.5b00246). The proagent **12** is non-signal-generating and nonfluorescent due to the masking of the amine group by carbamylation, while the released cargo silarhodamine is active in both diagnostic modalities. Note that a range of related or analogous cargos with various substitution motifs (such as a range of aniline-based fluorophores or photoacoustics dyes, including rhodols and rhodamines) could also be used as cargo subunits along the same design principles while remaining within the scope of a compound having the formula (I).

It is illustrated by **2, 3, 5, 7, 8, 10** and **12** that a broad range of diagnostically active phenol or amine cargos, eabling a range of structures and diagnostic detection methods, can therefore be used, including with extensive structural substitutions, within the scope of the invention, to deliver cargo-release-based activation of proagent diagnostic activity, i.e. functional diagnostic probes dependent upon triggering of the key disulfide of a compound having the formula (I).

These examples also include the theranostic design **6** that releases a cargo subunit that is *leuco*-methylene blue, a reduced form of the theranostic compound methylene blue which after release in biological media is spontaneously converted to methylene blue (similar principles are known for a range of related prodrugs). Methylene blue can be useful therapeutically including as a photosensitiser in photodynamic therapy (e.g. of the autoimmune disorder psoriasis, or of cancers) and/or as a redox-active selective toxin (e.g. against malaria parasite); as well as diagnostically (e.g. as a fluorescent agent or a photoacoustic dye); therefore being capable of theranostic activity (Schirmer, R. H. et al. Neurobiology of Aging 2011, 32, 2325.e7-2325.e16. https://doi.org/10.1016/j.neurobiolaging.2010.12.012). Note that a range of functionally and structurally related compounds (e.g. phenoxazines) can also be used as cargo subunits in theranostics following the same design principles while remaining within the scope of a compound having the formula (I).

Various modifications and variations of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be covered by the present invention.

### Examples

The invention will be more fully understood by reference to the following examples. They should not, however, be construed as limiting the scope of the invention.

### 1. Abbreviations

- A549:: human lung cancer cell line A549
- aq.:: aqueous (solution)
- 4T1:: Balb/c mouse breast cancer cell line
- Boc:: *tert*-butoxycarbonyl
- calc:: calculated
- DCM:: dichloromethane
- DIAD:: DIAD
- DMF:: dimethylformamide
- DMSO:: dimethylsulfoxide
- EDCI:: 1-(3-dimethylaminopropyl)-3-ethylcarbodiimide
- EI:: electron ionization
- ESI:: electron spray ionization
- FCC:: flash column chromatography
- HeLa:: *Henrietta Lacks,* human cervical cancer cell line
- (HP)LC:: (high performance) liquid chromatography
- HRMS:: high-resolution mass spectrometry
- DIPEA: diisopropylethylamine
- J:: coupling constant
- LC-MS:: liquid chromatography mass spectrometry
- LRMS:: low-resolution mass spectrometry
- MEF:: mouse embryonic fibroblast cell line
- MF-OH:: 3'-hydroxy-6'-methoxy-3*H*-spiro[isobenzofuran-1,9'-xanthen]-3-one
- NMR:: nuclear magnetic resonance
- Pd/C:: palladium on charcoal, 10%wt
- (δ) ppm:: chemical shift (parts per million)
- PMB:: p-methoxy benzyl
- PQ-OH:: 6-chloro-2-(5-chloro-2-hydroxyphenyl)quinazolin-4(3*H*)-one
- p-TSA:: p-toluenesulfonic acid
- R*_{f}*:: retentation factor

- RP:: reversed phase
- r.t.:: room temperature
- sat.: saturated (solution)
- THF:: tetrahydrofuran
- TLC:: thin layer chromatography

### 2. General Synthesis Conditions

Unless stated otherwise, (1) all reactions and characterisations were performed with solvents and reagents, used as obtained, under closed air atmosphere without special precautions; (2) DCM, Et₂O, THF and DMF used for synthesis were distilled, then dried on activated molecular sieves; (3) isohexane and EtOAc used for chromatography was distilled from commercial crude iso-hexane fraction; (4) "column" and "chromatography" refer to FCC, performed on Merck silica gel Si-60 (40-63 µm); (5) procedures and yields are unoptimised; (6) yields refer to isolated chromatographically and spectroscopically pure materials, corrected for residual solvent content.

NMR: Standard NMR characterisation was carried out by ¹H- and ¹³C-NMR spectra. Avance III Bruker BioSpin 400 MHz, 500 MHz and 600 MHz spectrometers were used (¹H: 400 MHz, 500 MHz or 600 MHz, ¹³C: 101 MHz, 126 MHz and 151 MHz respectively) typically at 298 K, although with some carbamates spectroscopy was simplified by measuring at 373 K to induce a dynamic equilibrium between their rotameric species. Chemical shifts (δ) are reported in ppm calibrated to residual non-perdeuterated solvent as an internal reference. Peak descriptions singlet (s), doublet (d), triplet (t), quartet (q), pentuplet (p), and multiplet (m) are used. Coupling constants J are given in Hz.

Mass spectra: Unit mass measurements were performed on an AGILENT 1200 SL coupled LC-MS system with ESI mode ionisation, with binary eluent mixtures of H₂O:MeCN, with the water containing formic acid. HRMS was carried out by the Zentrale Analytik of the Ludwig-Maximilian-Univeristy (LMU), Munich (electron impact (EI) at 70 eV with a Thermo Finnigan MAT 95 or a Jeol GCmate II spectrometer; electrospray ionization (ESI) with a Thermo Finnigan LTQ FT Ultra Fourier Transform Ion Cyclotron resonance mass spectrometer) in positive or negative mode as stated.

### 3. General Synthesis Protocols

### General protocol A: Amine protection using di-tert-butyl dicarboxylate

To a solution of the amine (1.0 eq) in dioxane:H₂O (1:1; 0.3 M) was added NEt₃ (1.3 eq per amine function). A solution of Boc₂O (1.2 eq per amine function) in dioxane was added to the reaction mixture and the resulting biphasic solution was stirred at 25°C for 15 h. The reaction mixture was then acidified to pH < 4 using aq. HCI (2 M), EtOAc was added, and the organic phase was separated and washed with a sat. aq. NaCl. The combined aq. layers were extracted with EtOAc (3x), then the combined organic layers were dried over Na₂SO₄, filtered and concentrated to afford the desired *N*-Boc-protected amine as a colourless solid. Further purification was achieved by FCC or by recrystallization from EtOH or MeOH.

**General protocol B: Thioacetylation using a 2-step sulfonylation/thioacetylation of primary/secondary alcohols.**

Step 1: To a solution of the primary or secondary alcohol in anhydrous pyridine (0.3 M) at 0 °C was added methane sulfonyl chloride (1.1 eq per alcohol function) and the mixture was stirred at 0°C for 30 min, was then allowed to warm to r.t. and was further stirred for 2-15 h. The resulting mixture was poured into ice water (water:pyridine, 5:1) and precipitation of a colourless solid was observed. The solid was filtered off, washed with cold water and cold diethylether and then dried to obtain the crude material as a colourless solid.

Step 2: The material obtained in step 1 was dissolved in acetone (0.1 M) or anhydrous DMF (0.05 M), solid KSAc (1.1 eq per alcohol function) was added and the resulting mixture was stirred at reflux for 4-15 h, before being cooled to r.t. and concentrated under reduced pressure. The residue was taken into EtOAc and washed with aq. NaCl (3x). The combined aq. layers were extracted with EtOAc (3x), then the combined organic layers were dried over Na₂SO₄, filtered and concentrated. Further purification was achieved by FCC (isohexane/EtOAc) to afford the desired bis(ethanethioate) derivatives.

### General protocol C: Basic ester hydrolysis and dithiol oxidation under air.

Similarly to Raines, Lukesh *et al.* (Lukesh, J. C. et al. J. Am. Chem. Soc. 2012, 134, 4057-4059. https://doi.org/10.1021/ja211931f) an oven-dried round-bottom flask was charged with a bis(ethanethioate) derivative (1 eq) and KOH (0.17 M in MeOH). The solution was stirred under air for 16 h. Reaction progress was monitored by LC-MS. In case of incomplete oxidation to the disulfide, a methanolic solution of I₂ was added dropwise until a permanently coloured solution was observed. The solvent was removed under reduced pressure, the resulting oil was taken into aq. Na₂S₂O₃ (which immediately removed any remaining colouration) and the mixture was extracted with EtOAc (4x). The combined organic layers were dried over Na₂SO₄, filtered and concentrated under reduced pressure to afford the desired product as a colourless solid. No further purification was required.

### General protocol D: Deprotection of N-Boc protected amines by organic HCI.

A N-Boc protected primary or secondary amine was dissolved in anhydrous DCM (0.1 M) and a solution of HCI (10 eq per amine function) in dioxane (4 M) was added dropwise. The mixture was stirred at r.t. for 2-15 h and was then added dropwise to cold Et₂O (Et₂O:DCM/dioxane, 10:1) and precipitation of a colourless solid was observed. The solid was filtered off, washed with Et₂O and dried under reduced pressure to obtain the desired primary or secondary amine hydrochloride salt as a colourless solid. No further purification was required.

### General protocol E: Carbamate coupling using triphosgene

Step 1: Similarly to Felber *et al.* (Felber, J. et al. ChemRxiv 2020. https://doi.org/10.26434/chemrxiv.13483155.v1) under inert gas atmosphere the phenolic compound (1 eq) was dissolved in anhydrous DCM (0.02 M). The reaction mixture was cooled to 0°C and a solution of triphosgene (BTC) (1.05 eq, 0.5 M in dry DCM) was added dropwise, followed by the addition of DIPEA (1.0 eq) dissolved in anhydrous DCM (1 M). The mixture was stirred at 0°C for 0.5 h, was warmed to r.t. and further stirred for 0.5 h. All volatiles were removed under reduced pressure employing an external liquid nitrogen trap to afford the corresponding phenolic chloroformate as a solid.

Step 2: The secondary amine hydrochloride (0.5-1.1 eq) was suspended in anhydrous DCM (0.05 M), and DIPEA (1.1 eq) was added and a clear solution was observed, which was added dropwise to a solution of the phenolic chloroformate in anhydrous DCM (0.02 M) was added dropwise at 0°C and the mixture was stirred for 0.5 h, was allowed to warm to r.t. and was further stirred for 0.5 h. A sat. aq. solution of NaHCO₃ was added and the mixture was diluted with DCM. The organic layer was separated and washed with sat. aq. NaCl. The combined aq. layers were extracted with DCM (2x) and the combined organic layers were dried over Na₂SO₄, filtered and concentrated under reduced pressure, to afford the crude product as a coloured solid. Purification using FCC gave the desired product as a colourless solid.

### 4. Synthesis

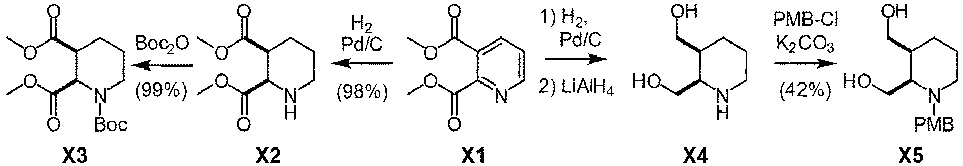

### (cis)-1-(tert-butyl)-2,3-dimethyl-piperidine-1,2,3-tricarboxylate (X3) (racemic)

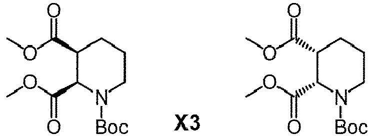

Step 1: To a solution of commercial dimethyl pyridine-2,3-dicarboxylate X1 (8.00 g, 41.0 mmol) in MeOH (35 mL) was added 96% H₂SO₄ (0.22 mL, 4.1 mmol) and 10% Pd/C (250 mg, 0.235 mmol). The mixture was stirred under H₂-atmosphere (40 bar) for 15 h, filtered over Kieselgur and concentrated to afford compound **X2** (8.09 g, 40.2 mmol 98%) as a colourless oil. The material was used without further purification.

Step 2: Prepared from the material obtained in step 1 (3.00 g, 14.9 mmol) according to **general protocol A.** Compound **X3** (4.43 g, 14.7 mmol, 99%, 97% over 2 steps) was obtained as a colourless solid by FCC (isohexane/EtOAc).

***R_{f}*** = 0.35 (isohexane:EtOAc, 2:1). **HRMS** (ESI⁺): C₁₄H₂₃NNaO₆: calc. 324.14176, found 324.14165. **¹H-NMR** (400 MHz, tetrachlorethane-*d₂*, 373 K): δ (ppm) = 5.32 (s, 1H), 4.05 - 3.87 (m, 1H), 3.68 (s, 3H), 3.66 (s, 3H), 2.85 - 2.71 (m, 1H), 2.59 (ddd, *J* = 12.7, 5.0, 3.6 Hz, 1H), 2.11-1.92 (m, 1H), 1.74-1.66 (m, 1H), 1.61 (td, *J=* 13.3, 3.9 Hz, 1H), 1.45 (s, 9H), 1.44 - 1.36 (m, 1H). **¹³C-NMR** (101 MHz, tetrachlorethane-*d₂*, 373 K): δ (ppm) = 171.7, 170.3, 155.0, 80.5, 55.9, 51.9, 51.7, 43.5, 41.3, 28.5, 24.3, 22.6.

### (cis)-N-(4-methoxybenzyl)-2,3-bis(hydroxymethyl)piperidine (X5) (racemic)

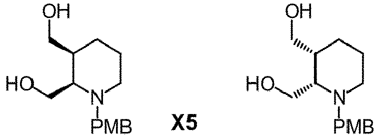

Step 1: **X2** was prepared from dimethyl pyridine-2,3-dicarboxylate (4.11 g, 21.1 mmol) as outlined above.

Step 2: The material obtained in step 1 was mixed with anhydrous THF (20 mL). The solution was added dropwise to a suspension of LiAlH₄ (2.40 g, 63.3 mmol, 3.0 eq.) in anhydrous THF (125 mL) at 0 °C under N₂-atmosphere. The mixture was allowed to warm to r.t. and was further stirred at r.t. for 15 h, was then cooled to 0 °C and EtOAc (5 mL), MeOH (5 mL), water (10 mL) and 2 M aq. NaOH (10 mL) were added carefully in the specified order and filtered. The solids were washed with THF (2x30 mL) and discarded. The filtrate was evaporated under reduced pressure. The residue as taken up in THF (30 mL) and MeOH (10 mL). After drying over Na₂SO₄ all volatiles were removed under reduced pressure and the residue containing **X4** was directly used further.

Step 3: To residue obtained in step 2 were added EtOH (45 mL), K₂CO₃ (7.29 g, 52.8 mmol, 2.5 eq.) and PMB-Cl (4.29 mL, 31.7 mmol, 1.5 eq.). The mixture was stirred at r.t. for 15 h, then the solids were filtered off, washed with EtOH and discarded. The filtrate was evaporated under reduced pressure, 2 M aq. HCI (60 mL) was added and the mixture was washed with DCM (3×30 mL). The organic washings were discarded, the aqueous solution was basified with 2 M aq. NaOH (70 mL) and extracted with DCM (2x60 mL). The extracts were dried over Na₂SO₄ and evaporated under reduced pressure. The residue was purified by FCC to afford compound **X5** (2.36 g, 8.89 mmol, 42% over 3 steps) as a faint orange oil.

**R*_{f}* =** 0.20 (EtOAc). **HRMS** (ESI⁺): C₁₅H₂₄NO₃: calc. 266.17507, found 266.17468. **¹H-NMR** (400 MHz, CDCl₃): δ (ppm) = 7.24 (d, *J*= 8.5 Hz, 2H), 6.86 (d, *J*= 8.6 Hz, 2H), 3.86 (d, *J=* 13.1 Hz, 1H), 3.81 (t, *J=* 9.5 Hz, 1H), 3.79 (s, 3H), 3.71 (d, *J=* 13.1 Hz, 1H), 3.67 - 3.52 (m, 3H), 2.98 - 2.90 (m, 1H), 2.74 (ddd, *J=* 13.6, 10.6, 3.3 Hz, 1H), 2.49 (dd, *J=* 13.5, 3.9 Hz, 1H), 2.17 (dd, *J=* 10.5, 4.4 Hz, 1H), 1.74 - 1.60 (m, 2H), 1.54 - 1.37 (m, 2H). **¹³C-NMR** (101 MHz, CDCl₃): δ (ppm) = 159.0, 130.8, 130.1, 114.0, 64.9, 60.8, 57.5, 57.3, 55.4, 46.4, 36.4, 25.0, 20.9.

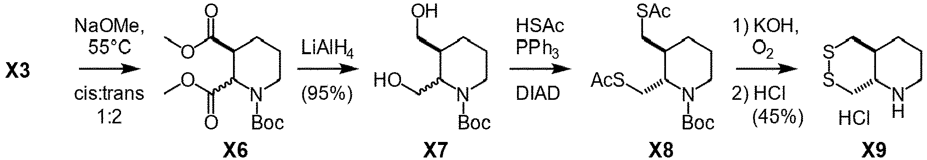

### (cis/trans)-1-(tert-butyl)-2,3-bis(hydroxymethyl)piperidine carboxylate (X7) (racemic)

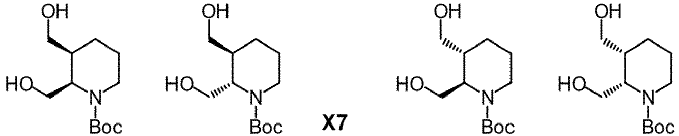

Step 1: A mixture of compound **X3** (5.33 g, 17.7 mmol, 1.0 eq.), anhydrous MeOH (50 mL) and NaOMe (1.15 g, 21.2 mmol, 1.2 eq.) was stirred under N₂-atmosphere at 55 °C for 15 h, before AcOH (1.52 mL, 26.5 mmol, 1.5 eq.) was added and all volatiles were removed under reduced pressure. The residue was suspended in DCM and filtered. The filtrate was diluted with isohexane and directly purified by FCC (isohexane/EtOAc) to afford a *cis*/*trans* mixture of 1-(*tert*-butyl)dimethyl piperidine-1,2,3-tricarboxylate **X6** (4.08 g, 13.5 mmol, 76%; 62% *trans)* as a yellow oil.

Step 2: To a suspension of LiAlH₄ (1.54 g, 40.6 mmol, 3.0 eq.) in anhydrous THF (80 mL) at 0 °C under N₂-atmosphere was dropwise added a solution of the material obtained in step 1 (4.08 g, 13.5 mmol, 1.0 eq.) in anhydrous THF (5.5 mL). The mixture was stirred for 30 min, was then allowed to warm to r.t. and further stirred for 2 h. The mixture was cooled to 0 °C, then EtOAc (5 mL), MeOH (5 mL), water (10 mL) and 2 M aq. NaOH (10 mL) were added carefully in the specified order and filtered. The solids were washed with a small amount of THF and discarded. The filtrate was evaporated under reduced pressure, Et₂O (50 mL) and Na₂SO₄ (30 g) were added and the mixture was filtered. The filtrate was evaporated under reduced pressure and remaining yellowish oil (3.16 g, 12.9 mmol, 95%) was further used in the next step. An analytically pure sample of compound **X7** was obtained by FCC (isohexane/EtOAc).

R*_{f}* = 0.30 (EtOAc). **HRMS** (EI) for C₁₁H₂₀NO₃ (M - CH₂O): calc. *m*/*z* 214.1438, found *m*/*z* 214.1435. **¹H-NMR** (400 MHz, CDCl₃) δ (ppm) = 4.41 (s, 1H), 3.86 (dd, *J*= 11.1, 7.3 Hz, 1H), 3.69-3.52 (m, 3H), 3.08 (s, 2H), 2.79 (m, 1H), 2.73 (s, 1H), 1.93 (dq, *J* = 7.9, 3.7 Hz, 1H), 1.90-1.80 (m, 1H), 1.69 (d, *J* = 5.2 Hz, 1H), 1.63-1.53 (m, 1H), 1.45 (s, 9H), 1.43-1.38 (m, 2H). **¹³C-NMR** (101 MHz, CDCl₃) δ (ppm) = 171.3, 80.1, 68.1, 64.6, 60.5, 59.6, 40.7, 28.5, 25.7, 25.3, 22.7, 21.2, 14.3.

### (trans)-N-Boc 2,3-bis((acetylthio)methyl)piperidine (X8) (racemic)

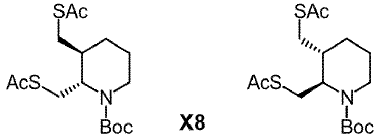

PPh₃ (6.28 g, 23.9 mmol) was dissolved in anhydrous THF (0.25 M) and DIAD (4.7 mL, 23.9 mmol) was added dropwise at 0 °C. Compound **X7** (2.35 g, 9.58 mmol) was dissolved in anhydrous THF (0.1 M) and pre-mixed with HSAc (1.7 mL, 23.9 mmol) before being added to the reaction mixture at 0 °C similar to Raines, Lukesh *et al.* (Lukesh, J. C. et al. J. Am. Chem. Soc. 2012, 134, 4057-4059. https://doi.org/10.1021/ja211931f). All volatiles were removed and the residue was suspended in a mixture of Et₂O/isohexane (1/2, 150 mL) and filtered. The solids were washed with additional Et₂O/isohexane (1/2, 60 mL) and discarded. The filtrate was evaporated under reduced pressure to give compound **X8** (4.93 g) as crude material. An analytically pure sample was obtained separately by FCC.

***R_{f}*** = 0.50 (isohexane:EtOAc, 3:1). **HRMS** (ESI⁺): C₁₆H₂₇NNaO₄S₂: calc. 384.12737, found 384.12765. **¹H-NMR** (400 MHz, CDCl₃): δ (ppm) = 4.26 (s, 1H), 4.15 - 3.80 (m, 1H), 3.33 - 3.13 (m, 1H), 3.06 (s, 1H), 2.99 (dd, *J=* 13.7, 6.4 Hz, 1H), 2.88 (dd, *J=* 13.7, 8.4 Hz, 1H), 2.73 (s, 1H), 2.33 (s, 3H), 2.32 (s, 3H), 1.89 - 1.74 (m, 2H), 1.70 - 1.52 (m, 2H), 1.45 (s, 9H), 1.44 - 1.39 (m, 1H). **¹³C-NMR** (101 MHz, CDCl₃): δ (ppm) = 195.7, 195.3, 155.4, 80.0, 52.6, 37.7, 36.3, 31.4, 30.8, 30.7, 29.8, 28.5, 23.1, 19.9.

### (trans)-hexahydro-[1,2]dithiino[4,5-b]pyridine hydrochloride (X9) (racemic)

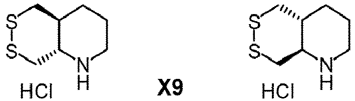

Prepared from the material containing compound **X8** obtained in the previous step (4.93 g) according to a 2-step sequence, first by **general protocol C** with purification by FCC, followed by **general protocol D,** giving compound **X9** (908 mg, 4.29 mmol, 45% over 3 steps) with stereochemical purity (pure *trans,* racemic).

**¹H-NMR** (400 MHz, DMSO-d₆): 9.34 (d, *J=* 20.9 Hz, 2H), 3.29 - 3.16 (m, 2H), 3.11 (dd, *J=* 13.0, 3.1 Hz, 1H), 3.00 (dd, *J=* 12.9, 11.3 Hz, 1H), 2.97 - 2.84 (m, 2H), 2.82 (dd, *J* = 13.8, 10.9 Hz, 1H), 2.01 (tdd, *J=* 10.9, 7.4, 3.3 Hz, 1H), 1.82 - 1.69 (m, 2H), 1.64 (d, *J=* 13.1 Hz, 1H), 1.34 (dt, *J=* 21.0, 12.4 Hz, 1H). **¹³C-NMR** (101 MHz, DMSO-d₆): 59.4, 43.9, 40.2, 38.6, 34.2, 28.8, 21.9.

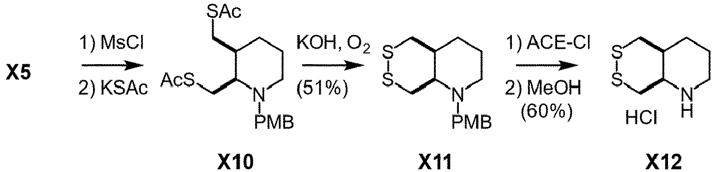

### (cis)-S,S'-((1-(4-methoxybenzyl)piperidine-2,3-diyl)bis(methylene))bis(ethanethioate) (X10) (racemic)

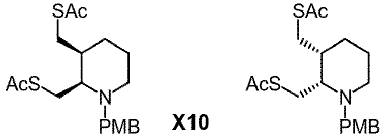

Prepared from compound X3 (1.11 g, 4.18 mmol) according to **general protocol B** using KSAc (3.0 eq) in DMF (0.05 M), giving **X10** (922 mg) after FCC (isohexane/EtOAc) as a colourless oil, which was used for for the next steps without further purification. An analytically pure sample was obtained separately by FCC (isohexane/EtOAc).

**R*_{f}*** = 0.36 (isohexane:EtOAc, 4:1). **HRMS** (ESI): C₁₉H₂₈NO₃S₂: calc. 382.15051, found 382.15002. **¹H-NMR** (400 MHz, CDCl₃): δ (ppm) =7.24 (d, *J=* 8.4 Hz, 2H), 6.84 (d, *J=* 8.6 Hz, 2H), 3.80 (s, 3H), 3.67 (s, 2H), 3.25 (dd, *J=* 13.6, 8.0 Hz, 1H), 2.99 (dd, *J=* 13.6, 5.4 Hz, 1H), 2.91 (dt, *J* = 8.5, 4.5 Hz, 1H), 2.88 - 2.77 (m, 2H), 2.60 (td, *J* = 12.3, 11.3, 3.2 Hz, 1H), 2.44 (d, *J=* 13.3 Hz, 1H), 2.33 (s, 3H), 2.33 (s, 3H), 2.13 - 2.00 (m, 1H), 1.75 - 1.65 (m, 1H), 1.63 - 1.53 (m, 1H), 1.48 - 1.40 (m, 1H), 1.40 - 1.30 (m, 1H). **¹³C-NMR** (101 MHz, CDCl₃): δ (ppm) = 196.4, 195.8, 158.7, 131.8, 129.8, 113.7, 61.0, 57.8, 55.4, 45.3, 37.6, 32.0, 30.8, 30.7, 26.0, 25.5, 22.3.

### (cis)-N-(4-methoxybenzyl)-octahydro-[1,2]dithiino[4,5-b]pyridine (X11) (racemic)

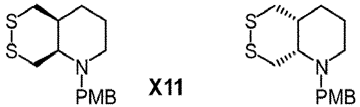

Prepared according to **general protocol C** from the 922 mg of material containing **X10** obtained in the previous step. Compound **X11** (629 mg, 2.13 mmol, 51% over 3 steps) was obtained by FCC (EtOAc/isohexane) as a yellowish oil.

**R*_{f}*** = 0.47 (isohexane:EtOAc, 4:1). **HRMS** (ESI): C₁₅H₂₂NOS₂: calc. 296.11373, found 296.11341. **¹H-NMR** (400 MHz, DMSO-d₆): δ (ppm) = 7.22 (d, *J*=8.2 Hz, 2H), 6.87 (d, *J* = 8.5 Hz, 2H), 3.73 (s, 3H), 3.66 (d, *J* = 12.5 Hz, 1H), 3.52 (d, *J* = 12.8 Hz, 1H), 3.40 - 3.31 (m, 1H), 3.25 (d, *J=* 13.0 Hz, 1H), 2.94 (d, *J=* 9.6 Hz, 1H), 2.84 (d, *J=* 12.9 Hz, 1H), 2.70 (dd, *J=* 12.3, 7.1 Hz, 1H), 2.49 - 2.32 (m, 2H), 2.25 - 2.14 (m, 1H), 2.09 (d, *J=* 10.9 Hz, 1H), 1.69 - 1.56 (m, 1H), 1.56 - 1.48 (m, 1H), 1.34(d, *J*= 11.8 Hz, 1H). **¹³C-NMR** (101 MHz, DMSO-d₆): δ (ppm) = 158.2, 131.1, 129.5, 113.6, 58.2, 56.5, 55.0, 45.0, 41.8, 36.8, 25.0, 22.6.

### (cis)-octahydro-[1,2]dithiino[4,5-b]pyridine (X12) (racemic)

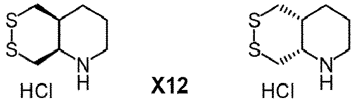

1-chloroethyl chloroformate (362 µL, 3.34 mmol, 2.0 eq.) was added dropwise to a solution of compound **X11** (494 mg, 1.67 mmol, 1.0 eq.) in anhydrous THF (5 mL) at 0°C. The mixture was warmed to r.t. and stirred for 3 days, before Et₂O (30 mL) was added and the mixture was washed with 2 M aq. HCI (25 mL). The aq. washings were extracted with Et₂O (2x30 mL), dried over MgSO₄, filtered and concentrated under reduced pressure. The residue was purified by FCC (DCM/isohexane), collecting a fraction with R_{f} = 0.5 (DCM). To the resulting colourless oil was added anhydrous MeOH (50 mL), the mixture was stirred at 80 °C for 1 h and evaporated under reduced pressure. The residue was washed with Et₂O to obtain compound **X12** (207 mg, 0.977 mmol, 58%) as an off-white solid.

**¹H-NMR** (400 MHz, DMSO-d₆, 373 K): δ (ppm) = 9.55 (s, 2H), 3.66 (dt, *J=* 6.7, 3.2 Hz, 1H), 3.51 (dd, *J* = 13.9, 7.6 Hz, 1H), 3.29 (s, 1H), 3.14 (d, *J* = 12.9 Hz, 2H), 3.04 - 2.85 (m, 1H), 2.35 (s, 1H), 1.91 (s, 1H), 1.82 (ddq, *J* = 12.6, 8.4, 4.3 Hz, 1H), 1.76 - 1.65 (m, 1H), 1.61 (td, *J=* 9.2, 4.5 Hz, 1H).

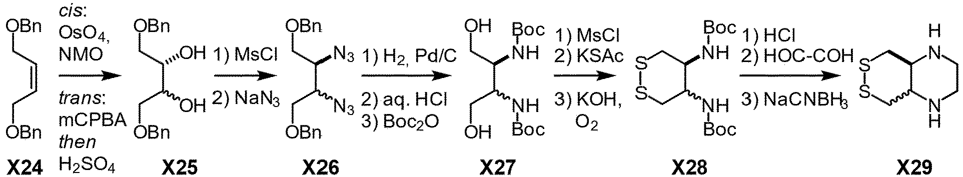

### (cis)-1,4-bis(benzyloxy)butane-2,3-diol (cis-X25) (racemic)

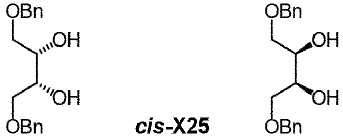

To a solution of commercial *cis*-1,4-dibenzyloxy-2-butene (15.8 g, 58.9 mmol) acetone:H₂O (3:1, 260 mL) were added 4-methylmorpholine *N*-oxide (19.9 g, 147 mmol) and K₂[OsO₂(OH)₄] (141 mg, 0.383 mmol). The reaction mixture was stirred at r.t. for 15 h, before being poured into sat. aq. sodium bisulfite solution. The aq. layer was extracted with EtOAc (3x200 mL) and the combined organic layers were washed with sat. aq. NaCl, dried and concentrated under reduced pressure to give ***cis*-X25** as a colourless solid (16.5 g, 54.6 mmol, 93%) without further purification.

R*_{f}* = 0.33 (hexanes:EtOAc, 1:1). **HRMS (EI+):** m/z calc. for C₁₈H₂₂O₄: [M]⁺ 302.1518, found: 302.1523. **¹H-NMR** (400 MHz, CDCl₃): δ (ppm) = 7.41 - 7.27 (m, 9H), 4.55 (s, 4H), 3.84 (dq, *J* = 4.2, 1.7 Hz, 2H), 3.76 - 3.56 (m, 4H), 2.68 (s, 2H). **¹³C-NMR** (101 MHz, CDCl₃): δ (ppm) = 137.89, 128.61, 127.99, 127.92, 73.66, 71.54, 71.19.

### (trans)-1,4-bis(benzyloxy)butane-2,3-diol (trans-X25)

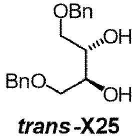

Step 1: m-CPBA (77%, 33.1 g, 148 mmol) was added in small portions to a solution of **X24** (33.0 g, 123 mmol) in DCM (120 mL) at 0°C. The reaction mixture was allowed to warm to r.t. and further stirred for 15 h, before being poured into sat. aq. NaHCO₃ (200 ml) and the aq. layer was extracted with DCM (3x150 mL). The combined organic layers were washed with sat. aq. NaCl and dried over MgSO₄, filtered and concentrated under reduced pressure and purified by FCC (hexanes/EtOAc) to give *cis*-1,4-dibenzyloxy-2,3-epoxybutane as a colourless oil (28.1 g, 99 mmol, 80%).

Step 2: To a solution of combined fractions of *cis*-1,4-dibenzyloxy-2,3-epoxybutane (37.2 g, 131 mmol) in THF:water (6:1, 280 mL) H₂SO₄ (34.9 mL, 654 mmol, 5.0 eq.) was added at 0°C. The reaction mixture was allowed to warm to r. t. and was further stirred for 15 h, before being diluted with water (300 mL) and EtOAc (200 mL) and the aq. layer was extracted with EtOAc (3×150 mL). The combined organic layers were washed with sat. aq. NaCl and dried over MgSO₄, filtered concentrated under reduced pressure and purified by FCC (isohexane/EtOAc 3:1) to give ***trans-X25*** as a colourless oil (25.7 g, 85.0 mmol, 65%).

**R*_{f}***= 0.38 (isohexane:EtOAc, 1:1). **HRMS** (EI): for C₁₈H₂₃O₄: calc. 303.1591, found: 303.1595. **¹H-NMR** (400 MHz, CDCl₃): δ (ppm) = 7.39 - 7.25 (m, 10H), 4.61 - 4.49 (m, 4H), 3.92 - 3.84 (m, 2H), 3.67-3.53 (m, 4H), 2.74 (s, 2H). **¹³C-NMR** (101 MHz, CDCl₃): δ (ppm) = 137.9, 128.6, 128.0, 127.9, 73.8, 72.1, 70.7.

### (cis)-(((2,3-diazidobutane-1,4-diyl)bis(oxy))bis(methylene))dibenzene (cis-X26) (racemic)

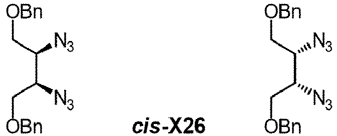

Step 1: ***cis*-X25** (16.5 g, 54.6 mmol) was dissolved in pyridine (600 mL, 0.1 M) and methane sulfonyl chloride (10.0 mL, 130.0 mmol) was added at 0 °C. The reaction was stirred for 1 h, was then allowed to warm to r.t. and was further stirred for 15 h, before being poured into cold water (5.0 L). The solid precipitate was filtered off, was washed with H₂O and dried under reducd pressure to give *(cis)*-1,4-bis(benzyloxy)butane-2,3-diyl dimethanesulfonate (22.1 g, 48.2 mmol, 88%) as a colourless solid.

Step 2: A mixture of the material obtained in step 1 and sodium azide (7.21 g, 111 mmol, 2.3 equiv.) in DMSO (140 mL, 0.3 M) was stirred at 80°C for 96 h, cooled to r.t., and the colourless suspension was diluted with water/brine (1:1, 300 mL). The aq. layer was extracted with EtOAc (3x150 mL) and the combined organic layers were dried over Na₂SO₄, filtered and concentrated under reduced pressure. The yellow crude oil was purified by FCC (hexane/EtOAc 9:1) giving **cis-X26** as a colourless liquid (15.8 g, 44.8 mmol, 93%, 81% over 2 steps).

**R*_{f}* =** 0.45 (isohexane:EtOAc, 9:1). **LRMS** (ESI⁺): for C₁₈H₂₀N₆O₂: calc. 325.2, found: 325.1. **¹H-NMR** (400 MHz, CDCl₃): δ (ppm) = 7.44 - 7.27 (m, 10H), 4.58 (s, 4H), 3.80 - 3.71 (m, 2H), 3.67 (m, 4H). **¹³C-NMR** (101 MHz, CDCl₃): δ (ppm) = 137.6, 128.6, 128.0, 127.8, 73.7, 69.8, 61.1.

### (trans)-(((2,3-diazidobutane-1,4-diyl)bis(oxy))bis(methylene))dibenzene (trans-X26)

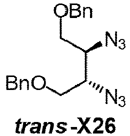

Step 1: To a solution of ***trans-X25*** (6.6 g, 22 mmol, 1.0 eq.) and pyridine (8.8 mL, 0.11 mol, 5.0 eq.) in dry DCM (45 mL), methane sulfonyl chloride (3.7 mL, 48 mmol, 2.2 eq.) was added dropwise at 0 °C. The reaction mixture was stirred for 1 h, was then allowed to warm to r.t. and further stirred for 15 h, before being diluted with DCM (50 mL), poured into sat. aq. NH₄Cl (200 mL) and extracted with EtOAc (3x100 mL). The combined organic layers were washed with sat. aq. NaCl, dried over MgSO₄ and concentrated under reduced pressure to yield *(trans)-*1,4-bis(benzyloxy)butane-2,3-diyl dimethanesulfonate as a colourless solid.

Step 2: A mixture of yield *(trans)*-1,4-bis(benzyloxy)butane-2,3-diyl dimethanesulfonate (2.3 g, 5.0 mmol), and sodium azide (0.82 g, 13 mmol) in DMSO (15 mL) was stirred at 80°C for 36 h, then cooled to r.t. and poured into water/NaHCO₃ (1:1, 300 mL) and extracted with EtOAc (3x100 mL). The combined organic layers were washed with sat. aq. NaCl, dried over MgSO₄, concentrated under reduced pressure and purified by FCC (isohexane/EtOAc) to give the ***trans*-X26** as a colourless oil (1.5 g, 4.2 mmol, 81% over 2 steps).

**R*_{f}*=** 0.46 (isohexane:EtOAc, 9:1). **¹H-NMR** (400 MHz, CDCl₃): δ (ppm) = 7.39-7.28 (m, 10H), 4.55 (s, 4H), 3.79 -3.71 (m, 2H), 3.69 - 3.64 (m, 4H). **¹³C-NMR** (101 MHz, CDCl₃): δ (ppm) = 137.5, 128.7, 128.1, 127.9, 73.7, 69.8, 61.1.

### (cis)-di-tert-butyl 1,4-dihydroxybutane-2,3-diyl)dicarbamate (cis-X27) (racemic)

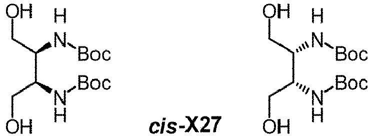

Step 1: To a solution of ***cis*-X26** (15.7 g, 44.6 mmol) in MeOH (400 mL, 0.1 M) was added Pd/C (10%, 634 mg). The mixture was stirred at r.t. under H₂-atmosphere at 1 bar for 15 h. The catalyst was removed by filtration over Kieselgur and solvent was removed under reduced pressure to give (*cis*)-1,4-bis(benzyloxy)butane-2,3-diamine as a colourless oil (12.6 g, 41.9 mmol, 94%).

Step 2: The material obtained in step 1 was charged with concentrated HCI (186 mL, 2.2 mol, 50 equiv.) and was heated to 80°C for 8 h, was then further stirred at r.t. for 15 h. The reaction mixture was concentrated under reduced pressure and the residue was dried by azeotropic evaporation using toluene (2x200 mL), was then washed with Et₂O (300 mL) and dried under reduced pressure to give the 1,4-dihydroxybutane-2,3-diamine dihydrochloride as a brown solid without further purification.

Step 3: Following **general protocol A,** the material obtained in step 2 was reacted with Boc₂O (23.4 g, 107 mmol) and NEt₃ (50 mL, 357 mmol) to give ***cis*-X27** as a colourless solid (11.1 mg, 34.7 mmol, 78% over 3 steps).

**R*_{f}***= 0.32 (hexanes/EtOAc 1:1). **HRMS (EI+):** m/z calc. for C₁₈H₂₂O₄: [M]⁺ 320.1947, found: 302.1946. **¹H-NMR** (400 MHz, DMSO-d₆): δ (ppm) = 6.42 (d, *J* = 7.3 Hz, 2H), 4.49 (t, *J* = 5.6 Hz, 2H), 3.51 (q, *J=* 5.3, 4.8 Hz, 2H), 3.41 (t, *J=* 4.6, 4.2 Hz, 4H), 1.38 (s, 18H). **¹³C-NMR** (101 MHz, DMSO-d₆): δ (ppm) = 155.54, 77.71, 60.80, 52.92, 28.23.

### (trans)-di-tert-butyl 1,4-dihydroxybutane-2,3-diyl)dicarbamate (trans-X27)

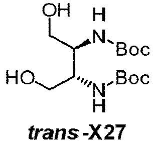

Step 1: To a solution of ***trans*-X26** (0.20 g, 0.57 mmol, 1.0 eq.) in MeOH (6.5 mL) was added Pd/C (10%, 39 mg). The mixture was stirred at r.t. under H₂-atmosphere at 5 bar for 20 h. The catalyst was removed by filtration over Kieselgur and the solvent was evaporated to give (*trans*)-1,4-bis(benzyloxy)butane-2,3-diamine as a colourless oil.

Step 2: The material obtained in step 1 was treated according **general protocol A.** The crude product was purified by FCC (isohexanes/EtOAc) to give di-tert-butyl *(trans)-1,4-*bis(benzyloxy)butane-2,3-diyl)dicarbamate as a colourless solid.

Step 3: A solution of the material obtained in step 2 in MeOH (2 mL) was added Pd/C (10%, 30 mg). The mixture was stirred at r.t. under H₂-atmosphere at 40 bar for 43 h. The catalyst was removed by filtration over Kieselgur, the solvent was evaporated under reduced pressure and the crude was purified by FCC (isohexane/EtOAc) to give ***trans-X27*** as a colourless solid (59 mg, 0.18 mmol, 59% over 3 steps).

**R*_{f}***= 0.38 (EtOAc). **HRMS** (EI): for C₁₄H₂₉N₂O₆: calc. 321.2020, found: 321.2029. **¹H-NMR** (400 MHz, CDCl₃): δ (ppm) = 4.90 (d, *J*= 8.3 Hz, 2H), 3.86 (s, 2H), 3.80 - 3.71(m, 4H), 3.50 (s, 2H), 1.44 (s, 18H). **¹³C-NMR** (101 MHz, CDCl₃): δ (ppm) = 157.3, 80.4, 62.9, 52.6, 28.4.

### (cis)-di-tert-butyl 1,2-dithiane-4,5-diyl)dicarbamate (cis-X28) (racemic)

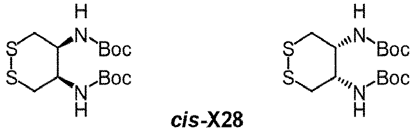

Step 1: Compound **cis-X27** (500 mg, 1.56 mmol) was treated with MsCI (0.278 mL, 0.359 mmol, 2.3 equiv.) in pyridine (15 mL, 0.1 M) at 0°C to give (*cis*)*- S, S'*-(2,3-bis((tert-butoxycarbonyl)amino)butane-1,4-diyl) dimesylate (680 mg, 1.43 mmol, 92%) as a colourless solid after precipitation of the reaction mixture from cold water and filtration of the solids.

Step 2: The material obtained in step 1 (600 mg, 1.26 mmol) was treated according to **general protocol B,** to yield (*cis*)-S,S'-(2,3-bis((*tert*-butoxycarbonyl)amino)butane-1,4-diyl) diethanethioate, which was isolated through FCC (isohexane/EtOAc) (27 mg, 0.06 mmol, 5%). Step 3: The thioacetylated intermediate obtained above (600 mg, 1.26 mmol) was treated according to **general protocol C,** to yield ***cis*-X28,** which was isolated after FCC (isohexane/EtOAc) (18 mg, 0.05 mmol, 82%).

**R_{f}**=0.45 (isohexanes:EtOAc, 6:1). **HRMS** (ESI)⁺: for C₁₄H₂₆N₂O₄S₂: calc. 350.1407, found: 351.1411. **¹H-NMR** (400 MHz, CDCl₃): δ (ppm) = 5.55 - 5.20 (br-s, 2H), 4.42 - 3.72 (br-m, 2H), 3.54 - 3.08 (br-m, 2H), 2.88 (br-dd, J= 13.4, 7.9 Hz, 2H), 1.45 (s, 18H). **¹³C-NMR** (101 MHz, CDCl₃): δ (ppm) = 156.0, 80.2, 52.7, 40.8, 28.3.

### (trans)-di-tert-butyl 1,2-dithiane-4,5-diyl)dicarbamate (trans-X28)

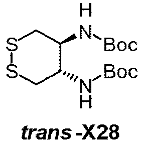

Step 1: Compound **trans-X27** (320 mg, 1.00 mmol) was treated with MsCI (0.18 mL, 2.3 mmol, 2.3 equiv.) in pyridine (10 mL, 0.1 M) at 0°C to give *(trans)- S, S'-*(2,3-bis((*tert-*butoxycarbonyl)amino)butane-1,4-diyl) dimesylate (312 mg, 0.66 mmol, 66%) as a colourless solid after precipitation of the reaction mixture from cold water and filtration of the solids.

Step 2: The dimesyl species (0.28 g, 0.59 mmol) was treated according to **general protocol B.** to give (*trans*)-S,S'-(2,3-bis((*tert*-butoxycarbonyl)amino)butane-1,4-diyl) diethanethioate (0.23 g, 0.53 mmol, 90%) as a colourless solid after purification by FCC (isohexanes/EtOAc).

Step 3: The material obtained in step 2 (0.23 g, 0.52 mmol) was treated according to **general protocol C,** to yield ***trans-X28,*** which was precipitated from the methanolic solution and isolated by filtration (0.18 g, 0.51 mmol, 57% over 3 steps).

***R_{f}*** = 0.29 (isohexane:EtOAc, 9:1). **HRMS** (EI): for C₁₄H₂₆N₂O₄S₂: calc.350.1334, found: 350.1331. **¹H-NMR** (400 MHz, CDCl₃): δ (ppm) = 5.04 (s, 2H), 3.68 (s, 2H), 3.12 (s, 2H), 2.85 (s, 2H),.1.43 (s, 18H). **¹³C-NMR** (101 MHz, CDCl₃): δ (ppm) = 156.0, 80.2, 55.4, 40.5, 28.5.

### (trans)-octahydro-[1,2]dithiino[4,5-b]pyrazine dihydrochloride (trans-X29)

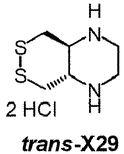

Step 1: Deprotection of ***trans-X28*** (170 mg, 0.49 mmol) was achieved according to **general protocol D** to give *(trans)-1,2-dithiane-4,5-diamine* dihydrochloride (106 mg, 0.48 mmol, 98%) as a colourless solid.

Step 2: (*trans*)-1,2-dithian-4,5-diamine dihydrochloride (50.1 mg, 0.22 mmol) was dissolved in MeOH (0.01 M) and KOH (0.5 mL, 1 M solution in MeOH) was. To the mixture was added a 0.88 M solution of glyoxal (280 µL, 9:1 MeOH/H₂O) and the mixture was stirred at r.t. for 1 h, before a solution of NaCNBH₃ (672 µL, 1 M in solution in MeOH) was added and the mixture was further stirred at r.t. for 1 h. The mixture was filtered over Kieselgur and washed with MeOH (20 mL), before being concentrated under reduced pressure. The residue was taken into MeOH (0.5 mL) and then added dropwise to a solution of HCI in Et₂O (0.01 M, 12 mL), the solid precipitated was filtered off and dried under reduced pressure to give ***trans-X29*** as a colourless powder (50.3, 0.20 mmol, 90%).

**HRMS** (ESI): C₆H₁₃N₂O₂S₂: calc. 177.05147, found 177.05160. **¹H-NMR** (400 MHz, D₂O): δ (ppm) = 3.96 - 3.83 (m, 1H), 3.86 - 3.75 (m, 1H), 3.55 (t, *J* = 10.8 Hz, 1H), 3.39 - 3.24 (m, 2H). **¹³C-NMR** (101 MHz, D₂O): δ (ppm) = 56.6, 40.9, 35.6.

### Compounds of the invention: Fluorogenic Probes

### (4-chloro-2-(6-chloro-4-oxo-3,4-dihydroquinazolin-2-yl)phenyl-(trans)-hexahydro-[1,2]dithiino[4,5-b]pyridine-1(2H)-carboxylate) (P1) (racemic)

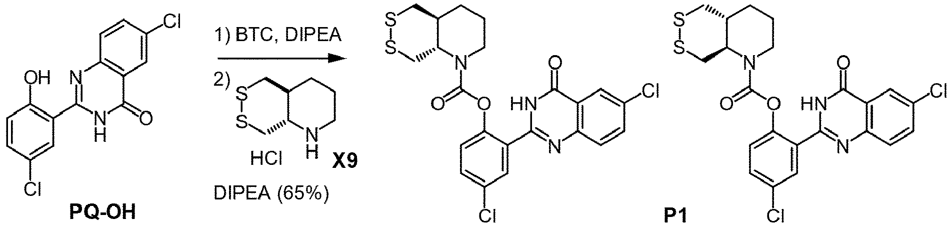

6-chloro-2-(5-chloro-2-hydroxyphenyl)quinazolin-4(3*H*)-one **(PQ-OH)** (70.0 mg, 0.228 mmol) (Felber, J. et al. ChemRxiv 2020. https://doi.org/10.26434/chemrxiv.13483155.v1) was coupled to compound **X9** (57.9 mg, 0.273 mmol) according to **general protocol E.** Purification by FCC (isohexane/EtOAc) gave compound **P1** (75.0 mg, 0.148 mmol, 65%) as a colourless solid.

**R*_{f}***= 0.44 (isohexane:EtOAc, 2:1). **HRMS** (ESI⁺): for C₂₂H₂₀Cl₂N₃O₃S₂: calc. 508.03176, found 508.03187. Individual rotamers were observed by NMR spectroscopy at 298 K (ratio ca. 1:1) and time-averaged spectra were obtained at 373 K. **¹H-NMR** (400 MHz, tetrachlorethane-*d₂*, 373 K): δ (ppm) = 8.23 (t, *J* = 1.5 Hz, 1H), 7.95 (d, *J* = 2.6 Hz, 1H), 7.76 - 7.67 (m, 1H), 7.49 (dd, *J* = 8.7 Hz, *J* = 2.6 Hz, 1H), 7.15 (d, *J* = 8.7 Hz, 1H), 3.77 (ddd, J= 13.6 Hz, *J*= 6.8 Hz, *J*= 3.3 Hz, 1H), 3.72 (ddd, *J=* 11.0 Hz, *J=* 10.5 Hz, *J=* 3.2 Hz, 1H), 3.38 (ddd, *J*= 14.0 Hz, *J=* 10.2 Hz, *J*= 5.5 Hz, 1H), 3.11 (dd, *J=* 12.9 Hz, *J=* 10.3 Hz, 1H), 3.03 (dd, *J*= 13.0 Hz, *J=* 3.3 Hz, 1H), 2.87 (dd, *J*= 13.6 Hz, *J=* 10.7 Hz, 1H), 2.75 (dd, *J*= 13.5 Hz, *J=* 3.0 Hz, 1H), 2.07 (ddd, *J=* 13.5 Hz, *J=* 10.7 Hz, 1H), 1.86 (ddd, *J*= 11.3 Hz, *J=* 7.6 Hz, 1H), 1.70 (ddd, *J=* 12.4 Hz, *J=* 3.4 Hz, 1H), 1.66 - 1.55 (m, 1H), 1.39 - 1.22 (m, 1H). **¹³C-NMR** (101 MHz, tetrachlorethane-*d₂*, 373 K): δ (ppm) = 160.4, 153.3, 149.0, 147.1, 135.3, 133.2, 132.3, 131.9, 130.3, 129.5, 128.0, 125.8, 125.3, 121.9, 63.1, 40.7, 40.2, 39.5, 36.2, 26.9, 23.1.

### (4-chloro-2-(6-chloro-4-oxo-3,4-dihydroquinazolin-2-yl)phenyl-(cis)-hexahydro-[1,2]dithiino[4,5-b]pyridine-1(2H)-carboxylate) (P2) (racemic)

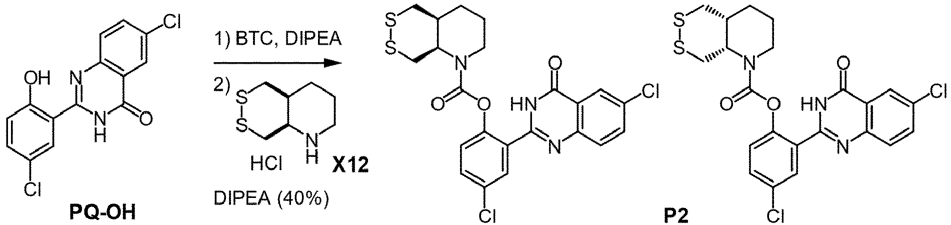

**PQ-OH** (51.6 mg, 0.168 mmol) was coupled to compound X12 (29.6 mg, 0.140 mmol) according to **general protocol E.** Purification by FCC (isohexane/EtOAc) gave compound **P2** (28.4 mg, 0.056 mmol, 40%) as a colourless solid.

R*_{f}* = 0.40 (isohexane:EtOAc, 2:1). **HRMS** (ESI⁺): C₂₂H₂₀Cl₂N₃O₃S₂: calc. 508.03176, found 508.03186. Individual rotamers were observed by NMR spectroscopy at 298 K (ratio ca. 1:1) and time-averaged spectra were obtained at 373 K. **¹H-NMR** (400 MHz, tetrachlorethane-*d₂*, 373 K): δ (ppm) = 9.72 (s, 1H), 8.23 (t, *J=* 1.5 Hz, 1H), 7.99 (d, *J=* 2.6 Hz, 1H), 7.73 (m, 2H), 7.50 (dd, *J=* 8.7 Hz, *J=* 2.6 Hz, 1H), 7.17 (d, *J=* 8.7 Hz, 1H), 4.48 (d, *J=* 11.7 Hz, 1H), 4.07 (d, *J*= 12.5 Hz, 1H), 3.53-3.33 (m, 2H), 3.18-2.88 (m, 1H), 2.77 (dd, *J*= 14.0 Hz, *J*= 3.0 Hz, 1H), 2.43 (d, *J=* 12.7 Hz, 1H), 2.28 (dd, *J*= 12.7 Hz, *J*= 4.0 Hz, 1H), 2.25 - 2.17 (m, 1H), 1.81 (d, *J=* 13.6 Hz, 1H), 1.70 - 1.54 (m, 1H), 1.47 (d, *J=* 13.1 Hz, 1H). **¹³C-NMR** (101 MHz, tetrachlorethane-*d₂*, 373 K): δ (ppm) = 160.2, 154.9, 149.1, 147.7, 147.5, 135.3, 133.5, 132.4, 132.3, 130.5, 129.9, 128.0, 126.1, 125.4, 122.4, 54.6, 42.2, 41.3, 36.0, 29.7, 25.3, 23.1.

### (3'-methoxy-3-oxo-3H-spiro[isobenzofuran-1,9'-xanthen]-6'-yl-(trans)-hexahydro-[1,2]dithiino[4,5-b]pyridine-1(2H)-carboxylate) (P3) (racemic)

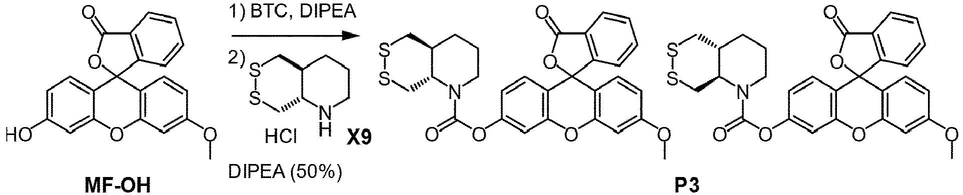

3'-hydroxy-6'-methoxy-3*H*-spiro[isobenzofuran-1,9'-xanthen]-3-one **(MF-OH)** (38.1 mg, 0.11 mmol) was coupled to compound **X9** (21.2 mg, 0.10 mmol) according to **general protocol E.** Purification by FCC (isohexane/EtOAc) gave compound **P3** (27.1 mg, 0.05 mmol, 50%) as a colourless solid.

***R_{f}*** = 0.58 (isohexane:EtOAc, 1:1). **HRMS** (ESI⁺) C₂₉H₂₆NO₆S₂: calc. 548.11961, found 548.11973. **¹H-NMR** (500 MHz, CDCl₃): δ (ppm) = 8.02 (d, *J=* 7.1 Hz, 1H), 7.67 (tt, *J=* 7.5, 1.4 Hz, 1H), 7.62 (t, *J=* 7.3 Hz, 1H), 7.15 (d, *J=* 7.4 Hz, 1H), 7.09 (dd, *J=* 3.7, 1.7 Hz, 1H), 6.84 - 6.77 (m, 2H), 6.76 (d, *J* = 2.5 Hz, 1H), 6.70 (d, *J* = 8.8 Hz, 1H), 6.62 (dd, *J* = 8.8, 2.5 Hz, 1H), 3.94 - 3.84 (m, 1H), 3.84 (s, 3H), 3.80 (dd, *J=* 10.1, 3.4 Hz, 1H), 3.35 (d, *J=* 15.2 Hz, 1H), 3.20-3.06 (m, 2H), 2.94 (dd, *J=* 13.5, 10.8 Hz, 1H), 2.81 (dd, *J=* 13.5, 2.7 Hz, 1H), 2.23 -2.09 (m, 1H), 1.96 (dt, *J=* 19.0, 8.5 Hz, 1H), 1.84-1.72 (m, 1H), 1.72-1.60 (m, 1H), 1.48 - 1.36 (m, 1H). **¹³C-NMR** (101 MHz, CDCl₃): δ (ppm) = 169.5, 161.5, 153.4, 153.2, 152.6, 152.4, 152.0, 135.2, 130.0, 129.2, 129.1, 126.6, 125.2, 124.2, 117.6, 116.4, 112.0, 111.0, 110.4, 101.0, 82.7, 63.1, 55.7, 41.5, 41.1, 40.7, 37.0, 34.3, 29.2, 27.8, 27.2, 26.0, 24.0, 23.1.

### (3'-methoxy-3-oxo-3H-spiro[isobenzofuran-1,9'-xanthen]-6'-yl-cis-hexahydro-[1,2]dithiino[4,5-b]pyridine-1(2H)-carboxylate) (P4) (racemic)

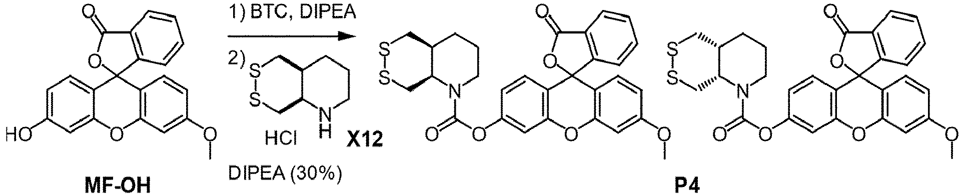

**MF-OH** (25.1 mg, 0.07 mmol) was coupled to compound **X12** (14.0 mg, 0.07 mmol) according to **general protocol E.** Purification by FCC (isohexane/EtOAc) gave compound **P4** (11.0 mg, 0.02 mmol, 30%) as a colourless solid.

**R*_{f}*** = 0.66 (isohexane:EtOAc, 1:1). **HRMS** (ESI⁺) C₂₉H₂₆NO₆S₂: calc. 548.11961, found 548.11996. **¹H-NMR** (500 MHz, CDCl₃): δ (ppm) = 8.02 (d, *J=* 7.4 Hz, 1H), 7.67 (t, *J=* 7.3 Hz, 1H), 7.62 (t, *J=* 7.3 Hz, 1H), ), 7.16 (d, *J=* 7.5 H, 1H), 7.12 - 7.01 (m, 1H), 6.82 - 6.77 (m, 2H), 6.76 (d, *J* = 2.4 Hz, 1H), 6.70 (d, *J* = 8.8 Hz, 1H), 6.62 (dd, *J* = 8.8, 2.4 Hz, 1H), 4.56 (t, *J=* 10.9 Hz, 1H), 4.14 (t, *J=* 12.5 Hz, 1H), 3.84 (s, 3H), 3.62-3.42 (m, 2H), 2.97 (dt, *J=* 44.8, 12.0 Hz, 1H), 2.83 (t, *J=* 15.1 Hz, 1H), 2.51 (ddt, *J=* 10.9, 7.0, 4.1 Hz, 1H), 2.39-2.21 (m, 2H), 1.86 (d, *J=* 13.4 Hz, 1H), 1.74-1.59 (m, 1H), 1.52 (d, *J=* 11.9 Hz, 1H), 1.36-1.21 (m, 1H). **¹³C-NMR** (101 MHz, CDCl₃): δ (ppm) = 169.5, 161.5, 153.2, 152.7, 152.4, 152.0, 135.2, 130.0, 129.2, 129.0, 126.6, 125.2, 124.2, 117.6, 116.3, 112.0, 111.0, 110.4, 101.0, 82.7, 55.7, 54.1, 53.3, 42.3, 42.2, 40.7, 40.2, 36.2, 35.9, 30.1, 29.4, 25.5, 25.0, 23.6.

### 4-chloro-2-(6-chloro-4-oxo-3,4-dihydroquinazolin-2-yl)phenyl trans-hexahydro-[1,2]dithiino[4,5-b]pyrazine-1(2H)-carboxylate (P5) (racemic)

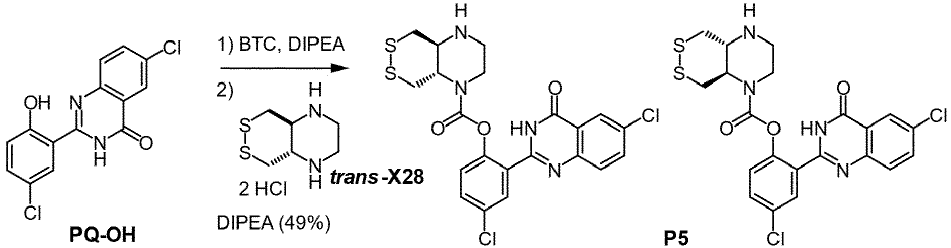

**PQ-OH** (67.9 mg, 0.221 mmol) was coupled to compound ***trans-X28*** (50 mg, 0.201 mmol) according to **general protocol E.** Purification by FCC (DCM/MeOH) gave compound **P5** (50.4 mg, 0.099 mmol, 49%) as a colourless solid. Further purification was achieved by preparative HPLC (H₂O/MeCN, 0.1% FA) to yield **P5** as a colourless solid.

**R*_{f}*** = 0.29 (DCM:MeOH, 9:1). **HPLC** RT = 5.80 min (LRMS: m/z 508.9). **HRMS** (ESI): C₂₁H₁₉Cl₂N₄O₃S₂: [M+H] calc. 509.02701, found 509.02701. **¹H-NMR** (400 MHz, MeOD-*d₄*): δ (ppm) = 8.22 (d, *J=* 2.4 Hz, 1H), 7.91 - 7.85 (m, 2H), 7.77 (d, *J=* 8.7 Hz, 1H), 7.65 (dd, *J* = 8.8, 2.6 Hz, 1H), 7.41 (d, *J=* 8.8 Hz, 1H), 3.89 (d, *J=* 15.1 Hz, 2H), 3.61 - 3.44 (m, 1H), 3.14 (td, *J* = 9.8, 4.3 Hz, 1H), 3.09-2.95 (m, 4H), 2.96-2.84 (m, 2H). **¹³C-NMR** (101 MHz, MeOD-*d₄*): δ (ppm) = 154.3, 136.3, 134.2, 133.0, 132.4, 131.0, 130.3, 126.5, 126.2, 65.0, 57.1, 44.9, 40.7, 38.2, 38.1.

### 4-chloro-2-(6-chloro-4-oxo-3,4-dihydroquinazolin-2-yl)phenyl (trans)-4-methylhexahydro-[1,2]dithiino[4,5-b]pyrazine-1(2H)-carboxylate (P6) (racemic)

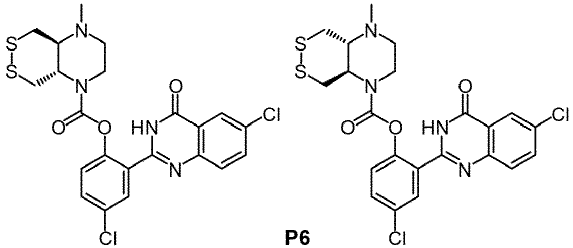

**P5** (9.4 mg 0.018 mmol) was dissolved in MeOH (5 mM) and a solution of formaldehyde (0.88 M, 10:1 diluted from 37% aq. solution: 209 µL, 0.184 mmol) was added. After 5 min of stirring at r.t., a solution of NaCNBH₃ (0.1 M solution in MeOH: 184 µL, 0.018 mmol) was added. The mixture was stirred at r.t. for 10 min, before being purified by preparative HPLC (H₂O/MeCN, 0.1% FA) to yield **P6** as a colourless solid (5.0 mg, 53%).

**R*_{f}*** = 0.40 (DCM:MeOH, 9:1). **HRMS** (ESI⁺): C₂₂H₂₁Cl₂N₄O₃S₂: calc. 523.04266, found 523.04279. **¹H-NMR** (400 MHz, MeOD-*d₄*): δ (ppm) = 8.40 (s, 1H), 8.10 (d, *J* = 2.4 Hz, 1H), 7.76 (dd, *J* = 8.7, 2.5 Hz, 1H), 7.70 (d, *J* = 2.6 Hz, 1H), 7.65 (d, *J* = 8.7 Hz, 1H), 7.53 (dd, *J* = 8.8, 2.6 Hz, 1H), 7.29 (d, *J*= 8.8 Hz, 1H), 3.74 (dd, *J=* 12.6, 5.1 Hz, 1H), 3.08 (d, *J=* 10.7 Hz, 1H), 3.07-3.02 (m, 2H), 2.94-2.84 (m, 1H), 2.82 (d, *J=* 13.2 Hz, 1H), 2.75 (t, *J* = 10.6 Hz, 1H), 2.74 - 2.65 (m, 1H), 2.47 - 2.40 (m, 1H), 2.05 (s, 3H).

### 4-chloro-2-(6-chloro-4-oxo-3,4-dihydroquinazolin-2-yl)phenyl (trans)-4-acetylhexahydro-[1,2]dithiino[4,5-b]pyrazine-1(2H)-carboxylate (P7) (racemic)

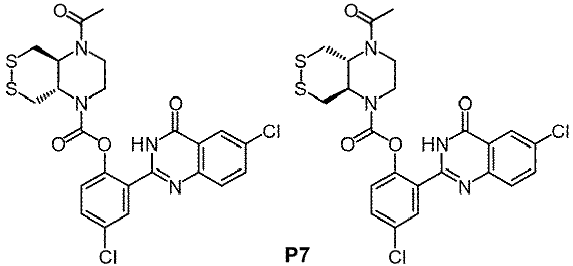

A solution of pyridine (49 µL, 1 M in anhydrous DCM) was added to a solution P5 (6.0 mg 11.8 µmol) in anhydrous DCM (0.01 M), followed by addition of a solution AcCI (29 µL, 1 M in anhydrous DCM). The mixture was stirred at r.t. for 30 min, was then diluted with DCM (5 mL), before being poured into aq. NH₄Cl solution (10 mL). The organic layer was washed with sat. aq. NH₄Cl (2x5 mL), the aq. layer was extracted with DCM (2x5 mL). The combined organic layers were dried over Na₂SO₄, filtered and concentrated under reduced pressure to yield P7 as a colourless solid (6.2 mg, 11.2 µmol, 95%).

**R*_{f}*** = 0.33 (EtOAc). **HRMS** (ESI⁺): C₂₃H₂₁Cl₂N₄O₄S₂: calc. 551.03758, found 551.03821. **¹H-NMR** (400 MHz, CDCl₃): 10.16 (s, 1H), 8.24 (d, *J*= 2.3 Hz, 1H), 7.97 (s, 1H), 7.76 (dd, *J=* 8.7, 2.2 Hz, 1H), 7.72 (d, *J=* 8.7 Hz, 1H), 7.53 (dd, *J=* 8.7, 2.5 Hz, 1H), 7.26 - 7.03 (m, 1H), 4.73 - 4.32 (m, 1H), 4.23 - 3.87 (m, 2H), 3.76 (s, 2H), 3.69 - 3.42 (m, 3H), 3.28 (d, *J=* 12.4 Hz, 1H), 3.14 (d, *J=* 13.7 Hz, 1H), 2.10 (s, 3H).

### 1-(4-chloro-2-(6-chloro-4-oxo-3,4-dihydroquinazolin-2-yl)phenyl) 4-methyl (trans)-hexahydro-[1,2]dithiino[4,5-b]pyrazine-1,4-dicarboxylate (P8) (racemic)

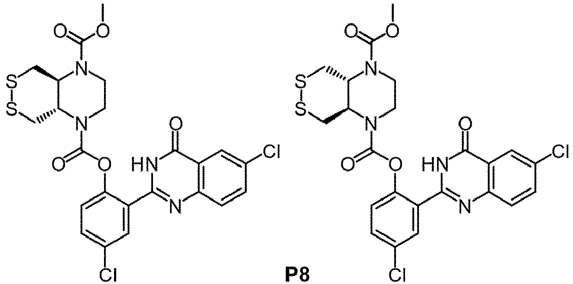

A solution of pyridine (49 µL, 1 M in anhydrous DCM) was added to a solution **P5** (6.0 mg 11.8 µmol) in anhydrous DCM (0.01 M), followed by addition of a solution methyl chloroformate (29 µL, 1 M in anhydrous DCM). The mixture was stirred at r.t. for 30 min, was then diluted with DCM (5 mL), before being poured into aq. NH₄Cl solution (10 mL). The organic layer was washed with sat. aq. NH₄Cl (2x5 mL), the aq. layer was extracted with DCM (2x5 mL). The combined organic layers were dried over Na₂SO₄, filtered and concentrated under reduced pressure to yield **P8** as a colourless solid (6.1 mg, 10.7 µmol, 91%).

**R*_{f}* =** 0.52 (isohexane:EtOAc, 1:2). **HRMS** (ESI⁺): C₂₃H₂₁Cl₂N₄O₅S₂: calc. 567.03249, found 567.03323. **¹H-NMR** (400 MHz, CDCl₃): δ (ppm) = 9.99 (s, 1H), 8.26 (d, *J=* 2.1 Hz, 1H), 7.97 (s, 1H), 7.75 (dd, *J=* 8.7, 2.3 Hz, 1H), 7.72 (d, *J=* 8.7 Hz, 1H), 7.53 (dd, *J=* 8.7, 2.6 Hz, 1H), 7.17 (d, *J=* 8.7 Hz, 1H), 4.11 - 4.01 (m, 1H), 4.02 - 3.84 (m, 2H), 3.77 (d, *J=* 18.4 Hz, 2H), 3.71 (s, 3H), 3.62 - 3.47 (m, 2H), 3.34 (s, 1H), 3.23 (dd, *J*= 13.4, 2.5 Hz, 1H), 3.16 (d, *J=* 12.2 Hz, 1H).

### 4-chloro-2-(6-chloro-4-oxo-3,4-dihydroquinazolin-2-yl)phenyl (trans)-4-(methylsulfonyl) hexahydro-[1,2]dithiino[4,5-b]pyrazine-1(2H)-carboxylate (P9) (racemic)

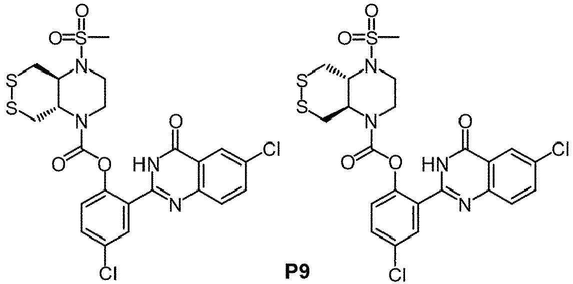

A solution of pyridine (49 µL, 1 M in anhydrous DCM) was added to a solution **P5** (5.0 mg 9.8 µmol) in anhydrous DCM (0.01 M), followed by addition of a solution methane sulfonyl chloride (29 µL, 1 M in anhydrous DCM). The mixture was stirred at r.t. for 30 min, was then diluted with DCM (5 mL), before being poured into aq. NH₄Cl solution (10 mL). The organic layer was washed with sat. aq. NH₄Cl (2x5 mL), the aq. layer was extracted with DCM (2x5 mL). The combined organic layers were dried over Na₂SO₄, filtered and concentrated under reduced pressure to yield **P9** as a colourless solid (3.5 mg, 10.7 µmol, 61%).

R*_{f}* = 0.49 (isohexane:EtOAc, 1:1). **HRMS** (ESI⁺): C₂₂H₂₁Cl₂N₄O₅S₂: calc. 587.00456, found 587.00505.

### Reference compound X19

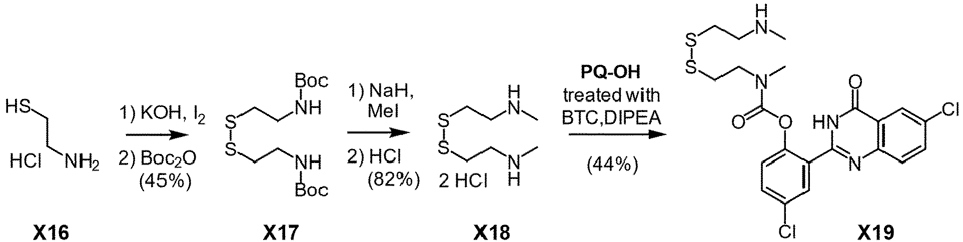

### di-tert-butyl (disulfanediylbis(ethane-2,1-diyl))dicarbamate (X17)

Cysteamine hydrochloride **X16** (1.00 g, 8.8 mmol) was dissolved in MeOH (30 mL) and the free base cysteamine was generated by addition of KOH (1.16 g, 17.6 mmol). Addition of methanolic I₂ until a slight colour persisted permanently, followed by evaporation of all volatiles gave 2-(2-aminoethyldisulfanyl)ethanamine (cystamine) that was N-Boc protected according to **general protocol A** gave **X17** (0.70 g, 2.0 mmol, 45%) as colourless crystalline needles after recrystallization from MeOH, that was directly used for the further steps.

### 2,2'-disulfanediylbis(N-methylethan-1-amine) (X18)

Step 1: **X17** (0.78 g, 2.21 mmol) was dissolved in anhydrous DMF (0.02 M) and solid NaH (2.2 eq) was added at 0 °C, followed by addition of Mel (2.3 eq) in one portion. The mixture was stirred for 0.5 h, then allowed to warm to r.t. and stirred for a further 1 h. The mixture was diluted with EtOAc and washed with sat. aq. NaCl (2x). The combined aq. layers were extracted with EtOAc (3x50 mL) and the combined organic layers were dried over Na₂SO₄. filtered and concentrated and purified by FCC to afford di-*tert*-butyl (disulfanediylbis(ethane-2,1-diyl))bis(methylcarbamate) as a colourless solid.

Step 2: Deprotection of the material obtained in step 1 was achieved according to **general protocol C** and the resulting diamine dihydrochloride **X18** (0.46 mg, 1.82 mmol, 82%) was obtained as a colourless solid.

**¹H-NMR** (400 MHz, DMSO-d₆): δ (ppm) = 9.27 (s, 4H), 3.19 (d, *J*= 7.4 Hz, 4H), 3.09 (dd, *J=* 8.1, 5.7 Hz, 4H), 2.56 (s, 6H). **¹³C-NMR** (101 MHz, DMSO-d₆): δ (ppm) = 47.0, 32.4, 32.3.

### 4-chloro-2-(6-chloro-4-oxo-3,4-dihydroquinazolin-2-yl)phenyl methyl(2-((2-(methyl amino)ethyl)disulfaneyl)ethyl)carbamate (X19)

**PQ-OH** (333 mg, 1.08 mmol) was coupled to **X18** (715 mg, 2.82 mmol) according to **general protocol E.** Purification by FCC (DCM/MeOH) gave **X19** as a colourless powder (245 mg, 0.477 mmol, 44%).

**R*_{f}*** = 0.22 (DCM:MeOH, 9:1). **HRMS** (ESI⁺) for C₂₁H₂₃Cl₂N₄O₃S₂: calc. 513.05831, found 513.05886. **¹H-NMR** (400 MHz, tetrachlorethane-*d₂*, 373 K): δ (ppm) = 8.20 (t, *J* = 1.5 Hz, 1H), 7.87 (d, *J*= 2.6 Hz, 1H), 7.71 (d, *J*= 1.5 Hz, 2H), 7.48 (dd, *J*= 8.7, 2.6 Hz, 1H), 7.24 (d, *J*= 8.7 Hz, 1H), 3.62 (s, 2H), 3.23 (d, *J=* 6.2 Hz, 2H), 3.18 (d, *J*= 6.1 Hz, 2H), 3.03 (s, 3H), 2.88 (s, 2H), 2.66 (s, 3H). **¹³C-NMR** (101 MHz, DMSO-d₆): δ (ppm) = 160.8, 153.1, 150.6, 147.9, 147.2, 134.9, 131.5, 131.4, 130.1, 129.6, 129.4, 128.5, 125.2, 125.0, 122.4, 47.7, 47.0, 35.1, 34.6, 32.4, 32.3.

### 5-(3-(azetidine-1-yl)propoxy-1H-indole-2-carboxylic acid (X21)

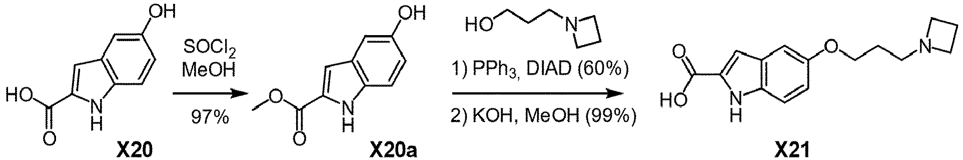

Step 1: 5-hydroxy-1*H*-indole-2-carboxylic acid **X20** (1.27 g, 7.15 mmol) was dissolved in MeOH (14 mL) and cooled to 0 °C. SOCI2 (0.78 mL, 1.28 g, 10.7 mmol) was added dropwise and the resulting mixture was heated to 75 °C for 2 h, then cooled to 25 °C and diluted with EtOAc (100 mL). The organic layer was washed with sat. aq. NaHCO₃ and sat. aq. NaCl and the combined aq. layers were extracted with EtOAc (3×50 mL). The combined organic layers were dried over Na₂SO₄, filtered, concentrated under reduced pressure and purified by FCC (isohexane/EtOAc) to give methyl 5-hydroxy-1*H*-indole-2-carboxylate **X20a** as a colourless powder (1.32 g, 6.9 mmol, 97%).

Step 2: PPh₃ (125 mg, 0.52 mmol) was dissolved in THF (0.25 M) and cooled to 0 °C. DIAD (94 µL, 0.48 mmol) was added dropwise and the mixture was stirred at r.t. for 30 min. A solution of 3-(azetidin-1-yl)propan-1-ol (0.5 M) in THFand a solution of methyl 5-hydroxy-1*H-*indole-2-carboxylate **X20a** (0.5 M) in THF were added subsequently. The mixture was allowed to warm to r.t. for 1 h, was then further stirred for 15 h, before being concentrated under reduced pressure and purified using FCC to give methyl 5-(3-(azetidin-1-yl)propoxy)-1*H-*indole-2-carboxylate (76 mg, 0.27 mmol, 62%) as a colourless oil.

Step 3: Methyl 5-(3-(azetidin-1-yl)propoxy)-1*H*-indole-2-carboxylate (222 mg, 0.77 mmol) was dissolved in MeOH:H₂O (3:1, 40 mL) and solid KOH (61 mg, 0.92 mmol) was added. The resulting solution was heated to 75 °C for 15h, was cooled to r.t. and a solution of HCI (0.74 mL, 1.25 M in MeOH) was added. The resulting heterogenous mixture was concentrated under reduced pressure to obtain compound **X21** (303 mg, 99%. 68% w/w calc. purity) contaminated with KCI. An analytically pure sample was separately obtained from RP chromatography (H₂O/MeCN) to gave **X21** as a colourless powder.

**HRMS** (ESI⁻) for C₁₃H₁₃NO₂Cl: calc. 275.13902, found 275.13910. **¹H-NMR** (400 MHz, DMSO-*d₆*): δ (ppm) = 11.14 (d, *J=* 2.2 Hz, 1H), 7.25 (d, *J=* 8.8 Hz, 1H), 7.08 (d, *J=* 2.4 Hz, 1H), 6.76 (d, *J*= 2.1 Hz, 2H), 6.74 (d, *J*= 2.4 Hz, 1H), 4.02 (t, *J*= 6.8 Hz, 2H), 3.63 (t, *J* = 7.5 Hz, 4H), 2.91 (t, *J*= 6.5 Hz, 2H), 2.20 (p, *J* = 7.5 Hz, 2H), 1.86 (p, *J* = 6.7 Hz, 2H). **¹³C-NMR** (101 MHz, DMSO-*d₆*): δ (ppm) = 165.1, 152.6, 134.1, 131.7, 127.6, 114.3, 112.9, 104.3, 102.6, 65.3, 53.2, 53.0, 25.4, 16.2.

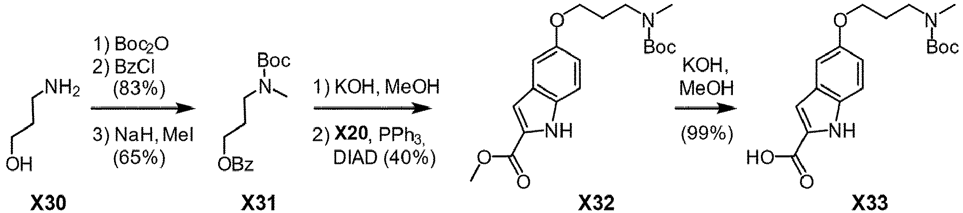

### 3-((tert-butoxycarbonyl)(methyl)amino)propyl benzoate (X31)

Step 1: To a solution of Boc₂O (6.4 g, 28.3 mmol) in DCM (1 M) was added dropwise at 0 °C a solution of 3-amino-1-propanol **X30** (2.0 mL, 26.6 mmol) in anhydrous DCM (3 M). The reaction mixture was allowed to warm to r.t. and further stirred for 15 h, before a solution of BzCI (3.7 mL, 32 mmol) in DCM (3 M) was added and the resulting mixture was further stirred for 4 h, before being poured into a mixture of sat. aq. NH₄Cl/H₂O (1:1, 100 mL). The organic layer was seperated and the aq. layer was extracted with DCM (3x50 mL), the combined organic layer were dried over Na₂SO₄, filtered, concentrated under reduced and purified by FCC (isohexane/EtOAc) to yield 3-((*tert*-butoxycarbonyl)amino)propyl benzoate as a colourless oil (6.2 g, 22.2 mmol, 83%).

Step 2: To the material obtained in step 1 (1.87 g, 6.69 mmol) in anhydrous DMF (0.02 M) at 0 °C was added iodomethane (0.5 mL, 8.03 mmol), directly followed by addition of solid NaH (60%, 0.295 g, 7.36 mmol), the mixture was then allowed to warm to r.t. and was further stirred for 2 h, before being poured into a mixture of sat. aq. NaCl/H₂O (1:1, 100 mL). The organic layer was separated and the aq. layer was extracted with DCM (3x50 mL), the combined organic layer were dried over Na₂SO₄, filtered, concentrated under reduced pressure and purified by FCC (isohexane/EtOAc) to yield **X32** as a colourless oil (1.28 g, 4.36 mmol, 65%).

**R*_{f}*** = 0.47 (isohexane:EtOAc, 3:1). **HRMS** (ESI⁺) for C₁₆H₂₄NNaO₄: calc 316.15193, found 316.15250. **¹H-NMR** (400 MHz, CDCl₃): δ (ppm) = 8.04 (d, *J=* 7.1 Hz, 2H), 7.55 (t, *J=* 7.4 Hz, 1H), 7.43 (t, *J* = 7.6 Hz, 2H), 4.34 (t, *J* = 6.3 Hz, 2H), 3.39 (t, *J* = 6.9 Hz, 2H), 2.88 (s, 3H), 1.99 (p, *J=* 6.5 Hz, 2H), 1.43 (s, 9H). **¹³C-NMR** (101 MHz, CDCl₃): δ (ppm) = 166.6, 155.8, 133.1, 130.3, 129.7, 128.5, 79.6, 62.8, 46.2, 33.9, 28.5, 27.5.

### methyl 5-(3-((tert-butoxycarbonyl)(methyl)amino)propoxy)-1H-indole-2-carboxylate (X32)

Step 1: To a solution of compound **X31** (1.06 g, 3.61 mmol) in MeOH (11 mL) at 0 °C was added a freshly prepared solution of KOH in MeOH (1 M, 5.4 mL, 5.4 mmol), the reaction mixture was allowed to warm to r.t. and was further stirred for 3 h, before being concentrated under reduced pressure. The residue was taken into a mixture of sat. aq. NHaCl/H₂O (1:1, 50 mL) and extracted with EtOAc (3x50 mL) at pH<5 to yield intermediate *tert-butyl* (3-hydroxypropyl)(methyl)carbamate as a colourless oil (0.68 g, 3.59 mmol, 99%).

Step 2: PPh₃ (911 mg, 3.47 mmol) was dissolved in THF (0.25 M) and cooled to 0 °C. DIAD (701 µL, 3.57 mmol) was added dropwise and the mixture was stirred at r.t. for 30 min. A solution of the material obtained in step 1 containing *tert-butyl* (3-hydroxypropyl) (methyl)carbamate (632 mg, 3.31 mmol) in THF and a solution of the 5-methyl-1*H*-indole-2-carboxylate **X20a** (0.5 M) in THF were added subsequently. The mixture was allowed to warm to r.t. for 1 h, was then further stirred for 15 h, before being poured into a mixture of sat. aq. NaCl/H₂O (1:1, 50 mL). The organic layer was seperated and the aq. layer was extracted with EtOAc (3x50 mL), the combined organic layers were dried over Na₂SO₄, filtered, concentrated under reduced pressure and purified by FCC (isohexane/EtOAc) to yield **X32** as a colourless solid (485 mg, 1.34 mmol, 40%).

**R*_{f}*** = 0.56 (isohexane:EtOAc, 2:1). **HRMS** (ESI⁺) for C₁₉H₂₇N₂NaO₅: calc 385.17339, found 385.17382. **¹H-NMR** (400 MHz, CDCl₃): δ (ppm) = 8.94 (s, 1H), 7.31 (d, *J*= 9.0 Hz, 1H), 7.14 - 7.10 (m, 1H), 7.05 (s, 1H), 6.99 (dd, *J*= 8.9, 2.3 Hz, 1H), 4.00 (t, *J*= 6.1 Hz, 2H), 3.93 (s, 3H), 3.43 (t, *J*= 6.8 Hz, 2H), 2.88 (s, 3H), 2.02 (s, 2H), 1.43 (s, 9H). **¹³C-NMR** (101 MHz, CDCl₃): δ (ppm) = 162.5, 156.5, 156.0, 154.0, 132.4, 127.9, 127.6, 117.5, 112.9, 108.4, 103.5, 79.5, 65.6, 52.1, 46.2, 34.6, 29.7, 28.1.

### 5-(3-((tert-butoxycarbonyl)(methyl)amino)propoxy)-1H-indole-2-carboxylic acid (X33)

To a solution of compound **X32** (403 mg, 1.11 mmol) in MeOH/H₂O (2:1, 60 mL) at r.t. was added solid KOH (780 mg, 11.1 mmol), the reaction mixture was heated to 80 °C and stirred for 2 h, before being cooled to r.t. and concentrated under reduced pressure. The residue was taken into a mixture of sat. aq. NH₄Cl/H₂O (1:1, 50 mL) and extracted with EtOAc (3x) at pH<5 to yield **X33** as a colourless oil (384 mg, 1.1 mmol, 99%).

**R*_{f}* =** 0.28 (DCM:MeOH, 9:1). **¹H-NMR** (400 MHz, CDCl₃) δ (ppm) =40% 8.92 (s, 1H), 7.20 (s, 1H), 7.01 (d, *J* = 9.9 Hz, 2H), 6.39 (s, 1H), 3.98 (s, 2H), 3.42 (s, 2H), 2.88 (s, 3H), 2.02 (s, 2H), 1.43 (s, 9H).

### Compounds of the invention: Therapeutic proagents

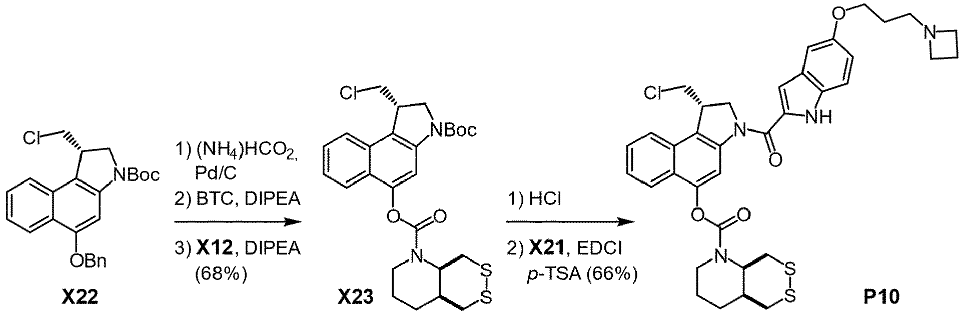

### (S)-3-(Boc)-1-(chloromethyl)-2,3-dihydro-1H-benzo[e]indol-5-yl (cis)-hexahydro-[1,2]dithiino[4,5-b]pyridine-1(2H)-carboxylate (X23) (racemic)

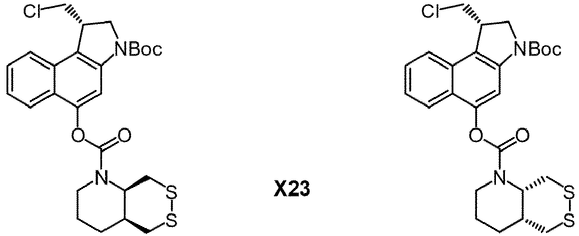

Step 1: Following Tietze *et al* (Tietze, L. F. et al. ChemMedChem 2008, 3, 1946-1955. https://doi.org/10.1002/cmdc.200800250), to commercial (S)-3-(Boc)-5-(benzyloxy)-1-(chloromethyl)-1,2-dihydro-3*H*-benzo[*e*]indole **X22** (591 mg, 1.39 mmol) were added dry THF (0.05 M), Pd/C (297 mg, 10% on charcoal), and aq. NH₄HCO₂ (2.8 mL of a 4 M aq. solution). The mixture was stirred for 80 min, filtered over Kieselgur and washed with EtOAc (20 mL) and the filtrates concentrated under reduced pressure to obtain (S)-3-(Boc)-1-(chloromethyl)-5-hydroxy-1,2-dihydro-3*H*-benzo[e]indole as a colourless solid (460 mg, 1.38 mmol, 99%).

Step 2: (*S*)-3-(Boc)-1-(chloromethyl)-5-hydroxy-1,2-dihydro-3*H*-benzo[*e*]indole (269 mg, 0.68 mmol) was coupled to **X12** (144 mg, 0.68 mmol) according to **general protocol E.** Purification by FCC (isohexane/EtOAc) gave **X23** as a colourless, crystalline solid (245 mg, 0.46 mmol, 67%).

**R*_{f}***= 0.50 (isohexane:EtOAc, 3:1). **HRMS** (ESI⁺) for C₂₆H₃₅N₃O₃ClS₂: calc. 552.17520, found 552.17543. **¹H-NMR** (400 MHz, CDCl₃): δ (ppm) = 8.05 (s, 1H), 7.82 - 7.72 (m, 1H), 7.70 (d, *J* = 8.4 Hz, 1H), 7.50 (t, *J* = 7.6 Hz, 1H), 7.42 - 7.32 (m, 1H), 4.67 (dd, *J* = 76.6, 12.0 Hz, 1H), 4.31 (dd, *J=* 34.5, 12.9 Hz, 1H), 4.14 (t, *J=* 10.4 Hz, 2H), 4.02 (t, *J=* 10.0 Hz, 1H), 3.93 (d, *J=* 11.4 Hz, 1H), 3.72 - 3.60 (m, 1H), 3.59 - 3.42 (m, 2H), 3.25 - 3.07 (m, 1H), 2.95 (dd, *J* = 23.6, 12.1 Hz, 1H), 2.89 - 2.79 (m, 1H), 2.74 - 2.49 (m, 1H), 2.47 - 2.26 (m, 2H), 1.88 (d, *J=* 16.1 Hz, 1H), 1.72 (s, 1H), 1.25 (s, 9H). **¹³C-NMR** (101 MHz, CDCl₃): δ (ppm) = 151.4, 147.3, 140.1, 129.2, 126.5, 123.3, 123.2, 121.5, 121.3, 108.3, 62.1, 53.1, 52.2, 51.9, 45.2, 41.3, 41.1, 40.0, 39.6, 39.1, 36.0, 35.3, 34.8, 30.9, 29.2, 28.7, 28.3, 27.4, 24.5, 24.0, 22.5, 21.7, 20.2, 19.8, 16.5, 13.1.

### (S)-3-(5-(3-(azetidin-1-yl)propoxy)-1H-indole-2-carbonyl)-1-(chloromethyl)-2,3-dihydro-1H-benzo[e]indol-5-yl (cis)-hexahydro-[1,2]dithiino[4,5-b]pyridine-1(2H)-carboxylate (P10) (racemic)

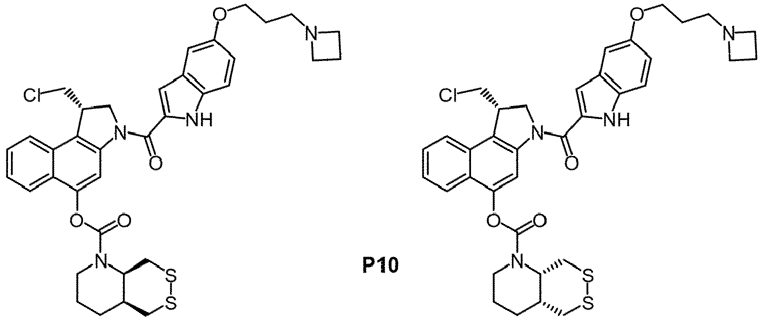

Step 1: Compound X23 (160 mg, 0.30 mmol) was treated according to **general procedure D** to give (*S*)-1-(chloromethyl)-2,3-dihydro-1*H*-benzo[*e*]indole-5-yl (*cis*)-hexahydro-[1,2]dithiino [4,5-*b*]pyridine a colourless solid.

Step 2: The material obtained in step 1 was dissolved in DMF (0.03 M). Similarly to Tercel *et al.* (Tercel, M. et al. Angew. Chem., Int. Ed. 2011, 50, 2606-2609. https://doi.org/10.1002/anie.201004456), **X21** (133 mg, 68% w/w, 0.33 mmol), EDCI (228 mg, 0.33 mmol) and p-TSA (52 mg, 0.30 mmol) were added. The solution was stirred at r.t. for 15 h, concentrated under reduced pressure, further dried by azeotroping with n-heptane, and purified by FCC (DCM/MeOH) to afford **P10** (137 mg, 0.20 mmol, 66%) as a colourless solid. Purification for *in vivo* application was achieved by preparative HPLC (H₂O/MeCN, 0.1% FA) to afford the compound **P10** as a colourless solid.

**R*_{f}***=0.48 (DCM:MeOH, 5:1). **HRMS** (ESI⁺) for C₃₆H₄₀N₄O₄ClS₂: calc 691.21740, found 691.21717. **¹H-NMR** (400 MHz, CDCl₃): δ (ppm) = 9.85 - 9.53 (m, 1H), 8.35 (s, 1H), 7.82 (t, *J* = 9.3 Hz, 1H), 7.76 (dd, *J* = 8.2, 4.3 Hz, 1H), 7.60 - 7.50 (m, 1H), 7.44 (t, *J* = 7.3 Hz, 1H), 7.35 (dd, *J* = 8.8, 2.9 Hz, 1H), 7.09 (d, *J* = 2.4 Hz, 1H), 7.00 (d, *J* = 2.0 Hz, 1H), 6.93 (d, *J=* 9.2 Hz, 1H), 4.78 (d, *J=* 10.8 Hz, 1H), 4.72-4.61 (m, 1H), 4.58 (d, *J=* 12.3 Hz, 1H), 4.39 (d, *J=* 24.8 Hz, 2H), 4.15 (d, *J=* 9.8 Hz, 1H), 4.08 (t, *J=* 5.6 Hz, 2H), 3.96 (d, *J=* 11.4 Hz, 1H), 3.90-3.78 (m, 2H), 3.64 (t, *J=* 5.4 Hz, 1H), 3.50 (t, *J=* 11.0 Hz, 2H), 3.33 (t, *J=* 7.5 Hz, 2H), 3.20 (d, *J*= 7.1 Hz, 1H), 3.02-2.89 (m, 1H), 2.86-2.78 (m, 2H), 2.76-2.68 (m, 1H), 2.54 (dd, *J=* 13.1, 3.8 Hz, 1H), 2.45-2.35 (m, 2H), 2.23 (d, *J* = 6.9 Hz, 4H). **¹³C-NMR** (101 MHz, CDCl₃): δ (ppm) = 153.1, 141.3, 131.6, 130.5, 128.8, 128.2, 127.8, 125.9, 125.3, 122.8, 122.6, 116.7, 113.1, 111.3, 106.2, 103.6, 64.6, 63.3, 55.1, 54.3, 53.0, 52.9, 45.8, 43.2, 42.3, 42.1, 40.7, 33.8, 31.9, 29.7, 29.4, 24.8, 23.5, 22.7.

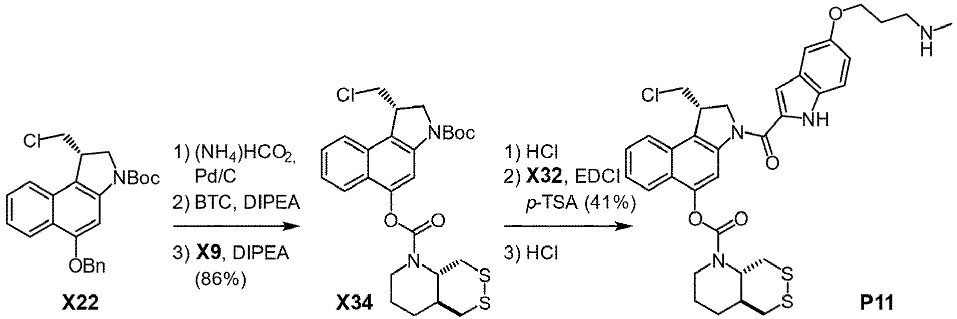

### (S)-3-(tert-butoxycarbony))-1-(chloromethyl)-2,3-dihydro-1H-benzo[e]indol-5-yl (trans)-hexahydro-[1,2]dithiino[4,5-b]pyridine-1(2H)-carboxylate (X34) (racemic)

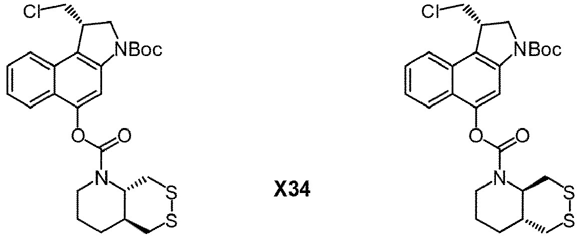

Step 1: (*S*)-3-(Boc)-1-(chloromethyl)-5-hydroxy-1,2-dihydro-3*H*-benzo[*e*]indole (115.1 mg, 0.34 mmol, 97%) was prepared from X22 (148.0 mg, 0.35 mmol) similar to the procedure described above

Step 2: The material obtained in step 1 was coupled to **X9** (88.9 mg, 0.42 mmol) according to **general protocol E.** Purification by FCC (isohexane/EtOAc) gave **X34** as a colourless, crystalline solid (162 mg, 0.30 mmol, 86%).

**R*_{f}***= 0.44 (isohexane:EtOAc, 3:1). **HRMS** (ESI⁺) for C₂₆H₃₅ClN₃O₃S₂: calc. 552.17520, found 552.17594. **¹H-NMR** (500 MHz, CDCl₃): δ (ppm) = 8.06 (s, 1H), 7.78 (d, *J=* 8.4 Hz, 1H), 7.70 (d, *J=* 8.4 Hz, 1H), 7.51 (t, *J=* 7.3 Hz, 1H), 7.38 (t, *J=* 7.6 Hz, 1H), 4.26 (d, *J=* 10.8 Hz, 1H), 4.13 (t, *J=* 10.2 Hz, 1H), 4.01 (td, *J*= 11.1, 10.1, 5.0 Hz, 2H), 3.96 - 3.82 (m, 2H), 3.46 (t, *J=* 10.7 Hz, 1H), 3.21 (d, *J=* 5.9 Hz, 2H), 3.03 - 2.91 (m, 1H), 2.83 (dd, *J=* 13.5, 2.6 Hz, 1H), 2.28 - 2.14 (m, 1H), 2.12 - 1.99 (m, 1H), 1.93 - 1.78 (m, 1H), 1.77 - 1.67 (m, 1H), 1.58 (s, 9H). **¹³C-NMR** (126 MHz, CDCl₃): δ (ppm) = 154.0, 153.2, 152.5, 148.4, 141.5, 131.4, 130.3, 128.1, 127.7, 124.5, 122.6, 122.5, 120.4, 109.4, 81.9, 63.8, 54.7, 47.0, 43.4, 41.2, 40.8, 39.1, 37.2, 28.5, 26.9, 23.3.

### (S)-3-(5-(3-((tert-butoxycarbonyl)(methyl)amino)propoxy)-1H-indole-2-carbonyl)-1-(chloromethyl)-2,3-dihydro-1H-benzo[e]indol-5-yl (trans)-hexahydro-[1,2]dithiino[4,5-b]pyridine-1(2H)-carboxylate (X35) (racemic)

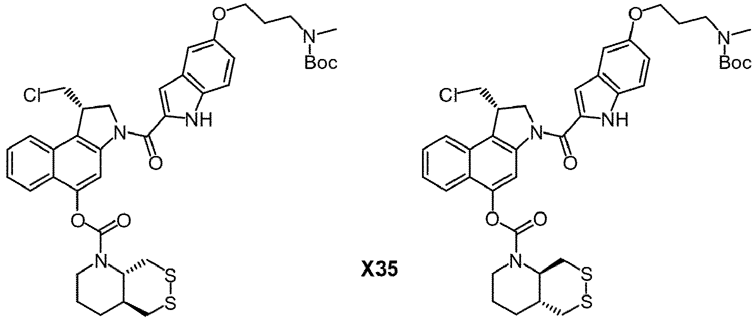

Step 1: Compound **X34** (22.0 mg, 0.04 mmol) was treated according to **general procedure D** to give (S)-1-(chloromethyl)-2,3-dihydro-1*H*-benzo[e*]*indole-5-yl (*trans*)-hexahydro-[1,2]dithiino [4,5-*b*]pyridine a colourless solid.

Step 2: The material obtained in step 1 was dissolved in DMF (0.03 M). Similarly to Tercel *et al.* (Tercel, M. et al. Angew. Chem., Int. Ed. 2011, 50, 2606-2609. https://doi.org/10.1002/anie.201004456), **X21** (23.2 mg, 0.05 mmol), EDCI (31 mg, 0.16 mmol) and *p*-TSA (7 mg, 0.04 mmol) were added. The solution was stirred at r.t. for 15 h, before being poured into a mixture of sat. aq. NaCl/H₂O (1:1, 20 mL). The organic layer was seperated and the aq. layer was extracted with EtOAc (3x15 mL), the combined organic layers were dried over Na₂SO₄, filtered, concentrated under reduced pressure and purified by FCC (isohexane/EtOAc) to yield **X34** (12.6 mg, 0.02 mmol, 50%) as a colourless solid.

**R***_{f}*= 0.59 (isohexane:EtOAc, 1:1). **HRMS** (ESI⁺) for C₃₉H₄₆N₄NaO₆S₂Cl: calc 787.23613, found 787.23632. **¹H-NMR** (400 MHz, CDCl₃): δ (ppm) = 9.47 (s, 1H), 8.40 (s, 1H), 7.85 (d, *J =* 8.4 Hz, 1H), 7.76 (dd, *J* = 8.2, 2.2 Hz, 1H), 7.55 (t, *J =* 7.6 Hz, 1H), 7.49 - 7.43 (m, 1H), 7.36 - 7.27 (m, 1H), 7.11 (s, 1H), 7.05 - 7.00 (m, 1H), 6.97 (ddd, *J =* 8.9, 3.8, 2.4 Hz, 1H),4.82 (d, *J =* 10.7 Hz, 1H), 4.67 (t, *J =* 9.6 Hz, 1H), 4.15 (dd, *J =* 11.4, 8.2 Hz, 1H), 4.08 - 3.97 (m, 4H), 3.97 - 3.88 (m, 2H), 3.54 - 3.48 (m, 1H), 3.45 (t, *J* = 7.0 Hz, 3H), 3.22 (d, *J* = 6.8 Hz, 2H), 3.03 - 2.92 (m, 1H), 2.90 (s, 3H), 2.83 (dd, *J* = 13.4, 2.3 Hz, 1H), 2.27 - 2.14 (m, 1H), 2.06 (dt, *J =* 13.6, 3.6 Hz, 3H), 1.85 (d, *J* = 6.3 Hz, 1H), 1.78 - 1.68 (m, 1H), 1.64 (s, 3H), 1.45 (s, 9H). **¹³C-NMR** (101 MHz, CDCl₃): δ (ppm) = 160.6, 156.0, 154.0, 148.2, 147.0, 141.5, 131.5, 130.4, 129.9, 128.4, 127.9, 127.2, 125.4, 125.0, 123.5, 122.9, 122.7, 122.5, 122.1, 117.4, 112.9, 111.4, 106.2, 103.4, 79.5, 65.6, 63.2, 55.2, 46.2, 46.0, 43.5, 41.1, 40.7, 39.8, 37.1, 34.7, 29.8, 28.6, 28.2, 27.7, 27.3, 23.3.

### (S)-1-(chloromethyl)-3-(5-(3-(methylamino)propoxy)-1H-indole-2-carbonyl)-2,3-dihydro-1H-benzo[e]indol-5-yl (trans)-hexahydro-[1,2]dithiino[4,5-b]pyridine-1(2H)-carboxylate (P11) (racemic)

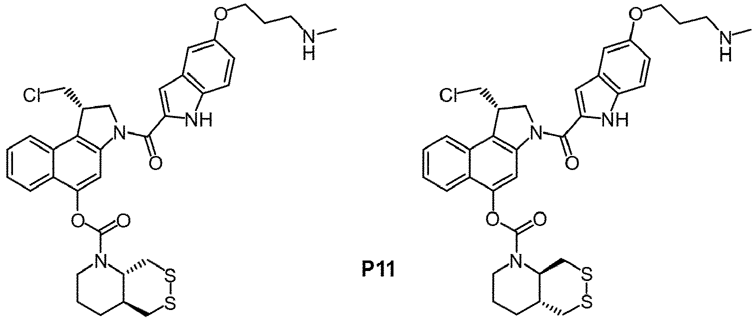

**X35** (5.1 mg, 0.007 mmol) was treated according to **general procedure D** and the residue was purified by preparative HPLC (H₂O/MeCN, 0.1% FA) to afford the compound **P11** as a colourless solid.

R*_{f}*= 0.15 (DCM:MeOH, 5:1). **¹H-NMR** (600 MHz, DMSO-*d₆*): δ (ppm) = 11.65 (s, 1H), 8.49 (s, 2H), 8.21 (s, 1H), 8.04 (d, *J* = 8.3 Hz, 1H), 7.86 - 7.79 (m, 1H), 7.63 (t, *J* = 7.7 Hz, 1H), 7.57 - 7.51 (m, 1H), 7.42 (d, *J* = 8.9 Hz, 1H), 7.18 (d, *J* = 1.9 Hz, 1H), 7.17 - 7.10 (m, 1H), 6.95 (dd, *J* = 8.9, 2.3 Hz, 1H), 4.88 (t, *J* = 10.0 Hz, 1H), 4.61 (d, *J* = 10.8 Hz, 1H), 4.42 (s, 1H), 4.13 - 4.06 (m, 3H), 4.00 (dd, *J* = 11.2, 6.8 Hz, 1H), 3.30 (t, *J* = 11.3 Hz, 1H), 3.08 (dt, *J* = 14.5, 7.6 Hz, 3H), 3.01 (d, *J* = 11.1 Hz, 1H), 2.95 (d, *J* = 12.5 Hz, 1H), 2.63 - 2.59 (m, 3H), 2.50 (dd, *J = 3.9,* 2.0 Hz, 2H), 2.38 (s, 1H), 2.21 (q, *J* = 11.1 Hz, 1H), 2.08 (p, *J* = 6.3 Hz, 2H), 1.94 (s, 1H), 1.81 (s, 1H), *1.69* (t, *J =* 10.8 Hz, 1H), 1.45 (p, *J* = 12.3, 10.8 Hz, 1H). **¹³C-NMR**(151 MHz, DMSO-*d₆*): δ (ppm) = 160.2, 153.2, 152.8, 147.2, 141.5, 139.3, 131.8, 130.8, 129.5, 127.7, 127.5, 125.2, 124.3, 123.5, 122.3, 122.2, 115.9, 113.3, 110.6, 109.5, 105.5, 103.3, 67.8, 65.0, 62.5, 54.9, 47.7, 46.0, 41.2, 33.7, 32.7, 26.3, 25.6, 21.9.

### 5. Biological Testing

### 5.1 Chemical reductant assay (see Figure 1)

This assay (see Figure 1) examined the stability of disulfide-based reduction-triggered compounds of the invention when exposed to monothiol reductants, primarily the major physiological cellular monothiol GSH, and dithiol reductants like the disulfide reductant dithiothreitol (DTT), as evaluated in a cell-free assay, to determine whether these compounds can be useful for selective release.

Assay Conditions: A black 96-well plate with black bottom was charged with solutions of fluorophore-releasing reduction-triggered probes that are compounds of the invention (**P1, P2**, **P3, P4** and **P5**) or that are not compounds of the invention and reveal the performance of prior art systems (**X19**) (80 µL; 25 µM in aq. TE buffer (pH 7.4) and 1.25%vol DMSO). Solutions of challenge species chosen from the monothiol glutathione (GSH, 0.1-50 mM) and the dithiol reductant dithiothreitol (DTT, 0.1-50 mM) or phosphine reductant tris-(2-carboxyethyl) phosphine (TCEP); (20 µL; 1 mM in aq. TE buffer (pH 7.4) were added and the reaction mixtures were incubated at 37 °C for 6 h while measuring fluorescence intensity over time [F(t)] using a BMG Labtech FLUOstar Omega platereader (λ_{exc}= 355 nm, λₑₘ = 530 nm for **PQ-OH**-releasing probes **X19, P1** and **P2;** λ_{exc} = 485 nm, λₑₘ = 530 nm for **MF-OH**-releasing probes **P3** and **P4.**

Data Representation: F^{max} is the maximum possible fluorescence increase corresponding to complete reduction-triggered cargo release, i.e. the fluorescence intensity increase from time zero to signal plateau under TCEP incubation **PQ-OH**-releasing probes. F(t)/F^{max}(t) thus gives the timecourse measurements of fluorescence intensity, as a fraction of the maximum possible fluorescence signal. F^{sat} is the endpoint fluorescence intensity increase for each condition, ie. F^{sat} = (F(6 h)). F^{sat}/F^{max} thus gives the assay endpoint fluorescence intensity increase, as a fraction of the maximum possible fluorescence signal. In the GSH timecourse measurements shown in Figure 1, all datasets annotated with concentrations only (0.01-10 mM) refer to GSH challenges, while datasets for TCEP positive control (100 µM) and no-reductant control (blank) are shown for comparison. The dose-response plots show F^{sat}/F^{max} for the different ratios between probe and GSH or probe and DTT concentrations, with a logarithmic horizontal scale.

Experimental Results and Discussion: In general, Figure 1 illustrates that **P1, P2 , P3** or **P4** (molecules of the invention) do not release cargo when challenged with non-enzymatic monothiol reductants as are abundant in all cells (note cellular GSH levels are around 5-10 mM), reflecting their desirably high resistance to ordinary cellular reducing conditions. As far as we know, they are the first disulfide-based proagent designs to display this key property, that is, they can respond to need (A) described above (suitable for responding to disulfide reductive activity). On the other hand, **P5** (compound of the invention) does release cargo very efficiently under those conditions, meaning that **P5** may therefore be utilized for rapid and robust intracellular release of appended cargoes, i.e. for the situation that the delivery into the cell is the process that gives the desired selectivity, thus responding to the need (E) described above (efficient activation).

Successful activation of all compounds of the invention by dithiol reductant DTT was also shown. DTT chemically mimics the active site of intracellular redox effector proteins (e.g. thioredoxin, to which it has similar reduction potential; Cheng, Z. et al. Biochemistry 2007, 46, 7875-7885. https://doi.org/10.1021/bi700442r; Houk, J. et al. J. Am. Chem. Soc. 1987, 109, 6825-6836. https://doi.org/10.1021/ja00256a040) which is relevant to the present invention.

TCEP is a non-physiological chemical reductant that rapidly and quantitatively chemically reduces all disulfides. The kinetics of signal generation during incubation with excess TCEP are therefore indicative of the speed of cargo release expected from probes after they have been reduced, and the fluorescence intensity established with excess TCEP is the maximum obtainable fluorescence from the assay. It is seen that compounds of the invention rapidly and quantitatively release their cargo upon reductive triggering and can therefore be useful as proagents for reduction-triggered release. Non-reducing control experiments (blank) shows that there is no assay interference by fluorescence increase independent of reduction, i.e. there is no non-reduction-based cargo release occurs with the compounds of the invention.

Taken together, this data shows that compounds of the invention **P1, P2, P3** and **P4** may be activatable specifically by vicinal dithiol type systems (such as DTT) and can rapidly release their cargo after such activation, while these compounds of the invention will be resistant to monothiols under physiological cellular conditions as well as resistant to other spontaneous degradation processes such as hydrolysis. Hence the compounds of the invention can offer the novel capacity to be specifically activated by altered states of metabolic activity and/or by specific cellular reductase enzymes, in that they can resist nonenzymatic activation by physiological cellular thiol levels. In comparison, prior art disulfide-based proagents can offer no stability against non-enzymatic reduction by monothiol species as are present in all cells at high concentrations. Compound of the invention **P5** has an unusually strong and fast response to both monothiol (GSH) and dithiol reductants (DTT) and may therefore serve as a platform for intracellular activation and release of cargos.

### 5.2 Enzymatic activation assay (see Figure 2)

This assay (see Figure 2) examined the resistance of disulfide-based reduction-triggered compounds to the major cellular enzymatic systems that maintain redox homeostasis and are capable of reducing disulfides, evaluated in a cell-free assay, to see if these compounds can be useful for selective enzyme-triggered release.

Assay conditions: A black 96-well plate with black bottom was charged with solutions of fluorophore-releasing reduction-triggered probes that are compounds of the invention (**P1, P2**) or that are not compounds of the invention and reveal the performance of prior art systems (**X19**) (50 µL; 40 µM in aq. TE buffer (pH 7.4) and 4 µL DMSO). Separately, mastermixes (30 µL in aq. TE buffer (pH 7.4)) containing mixtures of the proteins human recombinant thioredoxin 1 (Trx1, 0.166-16.66 µM) or thioredoxin 2 (Trx2, 3.3 µM), or human recombinant glutaredoxin 1 ("Grx1", 3.3 µM) or glutaredoxin 2 (Grx2, 3.3 µM) and/or oxidoreductases human recombinant thioredoxin reductase 1 (TrxR1, 0.07 µM), the thioredoxin reductase Secys498Cys mutant (TrxR1 - U498C, 0.07 µM), thioredoxin reductase 2 (TrxR2, 0.07 µM) or human recombinant glutathione reductase (GR, 0.07 µM), and/or GSH (3.3 µM) were prepared. Note that TrxR1 (U498C) has decreased reducing power compared to TrxR1 due to the mutation of the key vicinal selenothiol/thiol into a vicinal dithiol, but it still reduces Trx1. The appropriate mastermix was added into the probe solution and the assay was started by addition of *β*-NADPH (20 µL: 1 mM in aq. TE buffer (pH 7.4), which serves as the electron donor that reduces GR and TrxR. These mixtures are intended to mimic the biological TrxR/Trx and GR/Grx redox systems, that maintain cellular redox homeostasis and maintain glutathione redox buffer status, respectively. The reaction mixtures were incubated at 37 °C for 6 h. Timecourse measurements of fluorescence intensity were conducted. Note that in the summary table, "Trx1" indicates thioredoxin 1 that is applied in its oxidised form, which should not be capable of reduction unless enzymatically reduced by either TrxR1 or TrxR1 (U498C). Similarly Grx1 is applied in an oxidised form that can only be reduced by *in situ* recovered GSH, as in the GR/GSH/Grx mixture.

Data Representation: (F(t)-F^{NADPH}(t)) is the fluorescence intensity of the test solution at time t, from which is subtracted a background fluorescence level also at time t from a solution with NADPH (which itself is fluorescent, and is present in 10-fold higher concentration than the probe) and with the same protein mixture but without the probe. F^{max}(t) is the maximum possible fluorescence intensity value at time t, determined by incubation with TCEP (100 µM)) similarly as for the chemical reductant assay. (F(t)-F^{NADPH}(t))/F^{max}(t) thus gives the timecourse of fluorescence signal, as a fraction of the maximum possible fluorescence signal.

The timecourse plots show fluorescence timecourses with the NADPH/TrxR1/Trx1 system, where legend entries are as follows: "TrxR1" indicates that no Trx1 is added (NADPH/TrxR1 only), a concentration of "Trx1" indicates that the given concentration of Trx1 is included as well as NADPH/TrxR1, and "TCEP" indicates the 100 µM TCEP control. In the enzyme system summary table, enzymatic processing of the various probes is summarised as "no release" (-) indicating less than 5% cargo release in the first 2 h, or "release" (+) indicating more than 30% cargo release in the first 2 h.

Experimental Results and Discussion: In general, Figure 2 illustrates that **P1** or **P2** (molecules of the invention) release cargo only when challenged by the complete NADPH/TrxR/Trx or NADPH/GR/GSH/Grx redox cascades (that can adapt to and thereby serve as a marker of metabolic deregulation in pathologies, and not where components of the cascade are missing. **P1** and **P2** are not processed by TrxR1 or TrxR2, but are processed when additional high levels of Trx1 and Trx2 are added (see dose-response). However, they are not processed by TrxR1(U498C)/Trx1, indicating that the compounds of the invention can serve as proagents that are selectively triggered only by Trx1 and Trx2 and only when the full enzymatic system (native TrxR + Trx combination) is in place to maintain the Trx in its active state. They also resist the oxidoreductase GR, as well as high GSH levels generated by the mixture of GR/GSH, and are only processed by high levels of Grx1 and Grx2 generated by the full mixture of GR/GSH/Grx. The novel reduction-sensing motifs incorporated in molecules of the invention can therefore indeed be processed by endogenous redox effector proteins with high specificity and/or only under certain conditions.

In contrast, probe **X19** based on a prior art disulfide trigger releases cargo when challenged by various components of the cascades. It is rapidly degraded when exposed to GR/GSH and TrxR and TrxR1(U498C), with enhanced reaction kinetics when exposed to GR/GSH/Grx and TrxR/Trx. This indicates that the prior art triggers are cleavable by GSH, Grx, Trx, and TrxR, offering no specificity for any enzyme/protein and no stability against enzymatic and non-enzymatic reductions that occur with high turnover in all cells.

Taken together, this shows that compounds of the invention can have the novel feature of performing as selective diagnostics for activity of specific oxidoreductases or its redox effector protein within their system context (e.g. thioredoxin reductase in the TrxR+Trx system), whereas prior art compounds do not allow this; and more generally, compounds of the invention may be useful for selective cargo release triggered by activity of specific enzymes or triggered by unusual redox metabolic conditions.

### 5.3 Cellular reduction assay (see Figure 3)

Assay Conditions: Cells were cultured under standard conditions (Gao, L. et al. Cell Chemical Biology 2021, 28, 1-14. https://doi.org/10.1016/j.chembiol.2020.11.007), left to adhere for 24 h, then compounds were added to 100 µM with 1%vol DMSO. Fluorescence timecourse measurements were acquired as in the chemoreductant assay, with all conditions in triplicates.

Data Representation: F(t)-F(0) is the fluorescence intensity signal increase as compared to time zero signal (fluorescence intensity immediately following compound addition, before probe activation can occur), in arbitrary units although with the same detection settings for all probes releasing the same cargo. This corresponds to reduction-triggered cargo release. "*ctrf*" indicates cells not treated with probes, as a negative control.

Experimental Results and Discussion: Figure 3 illustrates that **P1, P2** or **P4** (molecules of the invention) do not release cargo inside cells under ordinary conditions (there is no signal increase over time), while **P5** (molecule of the invention) and prior art disulfide probe **X19** substantially release cargo inside cells cultured under ordinary conditions. The match between the curve shapes of each probe across the three cell lines argues that the results observed here for two human and one mouse cell line are generalisable across all standard cells kept under ordinary conditions. Thus compounds of the invention either have the possibility of being useful by giving selective cargo release in pathologically growing cells or cells under abnormal conditions (**P1, P2, P4**), or offer novel opportunities of cellular delivery of appended cargoes with optimal response kinetics (**P5**).

### 5.4 in vitro cell viability assay (see Figure 4)

Assay Conditions: Cells were cultured under standard conditions (Gao, L. et al. Cell Chemical Biology 2021, 28, 1-14. https://doi.org/10.1016/j.chembiol.2020.11.007), left to adhere for 24 h, then **P10** was added from DMSO stock solution and adjusted to 1%vol DMSO. Cells were incubated under standard conditions for 48 h, then 0.20 volume equivalents of a solution of resazurin in PBS (0.15 mg/mL) were added and incubated for 3 hours, then fluorescence (ex 544 nm, em 590 nm) was detected on a fluorescence platereader. Cell viability (%) is calculated relative to a control experiment with no **P10** added, from the fluorescence values.

Experimental Results and Discussion: Figure 4 illustrates that **P10** is significantly less toxic to cells grown under ordinary conditions, than its released cargo CBI-OH would be (literature IC₅₀ for CBI-OH is approx. 40 pM; Jin, W. et al. J Am Chem Soc 2007, 129, 15391-15397. https://doi.org/10.1021/ja075398e). This indicates that in cells under normal growth conditions **P10** only releases a small amount of the of active DNA-alkylating agent. Therefore, if the stabilised disulfide system in **P10** is activated under increasingly reductive conditions (such as by overexpression or overactivation of enzymatic reductants in redox-dysregulated tissues), its toxicity in those tissues may be substantially and selectively increased, compared to in tissues with normal conditions.

### 5.5 in vivo mouse tumour model assay (see Figure 5)

Assay Conditions and Data Representation: Immunocompetent wildtype Balb/c mice were inoculated with 4T1 Balb/c mouse breast cancer cell line orthotopically in the mammary fat pad (2×10⁵ cells) and tumour growth was monitored by caliper measurement. The day when all tumours had reached ca. 150 mm³ was defined as Day 0; on this day the mice were randomised by tumour size into groups of minimum size 5 animals (mean tumour size ca. 200 mm³), and treated. Treatments were chosen from 0.9% NaCl ("untreated"), the vascular disrupting agent combretastatin A-4 phosphate (CA4P) at 30 mg/kg administered in 100 µL NaCl ("VDA"), compound of the invention **P10** at 4 mg/kg administered in 30 µL DMSO ("**P10**"), or a combination regime involving treatment with VDA and then 6 hours later with **P10** ("VDA+**P10**"). All treatment were administered intraperitoneally (i.p.). CA4P was re-applied to groups "VDA" and "VDA+SS-CBI" once at day 7; no **P10** treatment was re-applied to either "**P10**" or "VDA+**P10**" groups. Tumor volumes were measured as assay readout. Experimental Results and Discussion: Figure 5 shows that a single treatment with therapeutic compound of the invention **P10** significantly slows primary tumor progression in this aggressive immunocompetent tumor model; and that a preferred combination treatment type according to the invention, involving the administration of a vascular disrupting agent (VDA) designed to enhance tumor hypoxia shortly before administration of **P10,** still more significantly slows tumour growth in accordance with the design for bioreduction-based compound activation.

## Claims

**1.** A compound having the formula (I)
A-L-B (I)
wherein
A is represented by 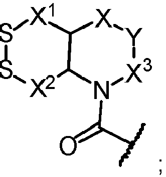
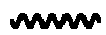 denotes the attachment point of A to L;
L is a bond or a self-immolative spacer;
B is represented by 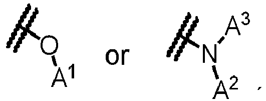
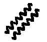 denotes the attachment point of B to L;
A¹ is selected such that A¹-OH is a therapeutic, diagnostic or theranostic agent which contains an -OH group that is attached to a 5- or 6-membered aromatic or heteroaromatic ring;
A² and A³ are independently selected such that A²-NH-A³ is a therapeutic, diagnostic or theranostic agent which contains an -NH₂ or -NH- moiety;
X is selected from -N(R^{a})-, -N(R^{b})- and -CR^{c}₂-;
X¹ is -(CR^{d}₂)ₘ-;
X² is -(CR^{e}₂)ₙ-;
X³ is -CR^{f}₂-;
Y is -(CR⁹₂)ₚ-;
W is independently selected from -OH, -N(R^{h})(Rⁱ), and a heterocyclic group selected from azetidinyl, pyrrolidinyl, piperidinyl or morpholino, wherein the heterocyclic group is attached to the -C₁₋₄-alkyl or -(C₂₋₄-alkylene)- group via the N atom;
R^{a} is selected from -H, -C₁₋₄-alkyl, -C(O)-C₁₋₄-alkyl, -C(O)-O-C₁₋₄-alkyl, -C(O)-N(C₁₋₄-alkyl)₂, -S(O)₂-C₁₋₄-alkyl, and -(C₂₋₄-alkylene)-O-(C₁₋₄-alkyl), wherein -C₁₋₄-alkyl or -(C₂₋₄-alkylene)- can be optionally substituted by W;
R^{b} is an acyl group of a monopeptide selected from -proteinogenic amino acids attached via a carboxy group;
R^{c} groups are independently selected from -H and -C₁₋₄-alkyl;
R^{d} groups are independently selected from -H and -C₁₋₄-alkyl;
R^{e} groups are independently selected from -H and -C₁₋₄-alkyl;
R^{f} groups are independently selected from -H and -C₁₋₄-alkyl;
R^{g} groups are independently selected from -H and -C₁₋₄-alkyl;
R^{h} is independently selected from -H, -C₁₋₄-alkyl and -CH₂CH₂OH;
Rⁱ is independently selected from -H, -C₁₋₄-alkyl and -CH₂CH₂OH;
m is 0, 1 or 2;
n is 1 or 2, provided that m+n is 2 or 3;
p is 0, 1, or 2, provided that when X = -N(R^{a})- or -N(R^{b})- then p = 1 or 2;
or any pharmaceutically acceptable salt, solvate or ester thereof.

**2.** The compound according to claim 1, wherein L is a bond.

**3.** The compound according to claim 1, wherein L is a self-immolative spacer selected from 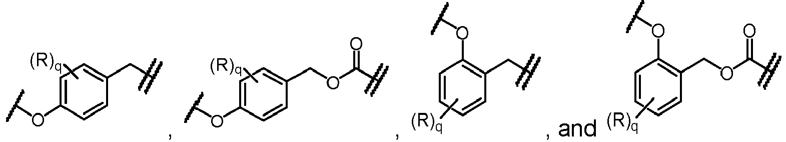 wherein
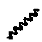 denotes the attachment point to A;
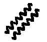 denotes the attachment point to B;
R is independently selected from halogen, -O(R^{r}), -N(R^{s})(R^{t}), -NO₂, -CN, and a heterocyclic group selected from azetidinyl, pyrrolidinyl, piperidinyl or morpholino,
wherein the heterocyclic group is attached to the phenyl ring *via* the N atom;
q is 0, 1, 2, 3 or 4;
R^{r} is independently selected from -H, -C₁₋₄-alkyl and -(C₂₋₄-alkylene)-O-(C₁₋₄-alkyl), wherein -C₁₋₄-alkyl or -(C₂₋₄-alkylene)- can be optionally substituted by W;
R^{s} is independently selected from -H, -C(O)-C₁₋₄-alkyl and -C₁₋₄-alkyl; and
R^{t} is independently selected from -H, -C(O)-C₁₋₄-alkyl and -C₁₋₄-alkyl.

**4.** The compound according to claim 3, wherein L is a self-immolative spacer selected from 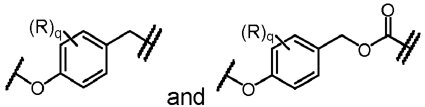 wherein R and q are as defined in claim 3.

**5.** The compound according to any one of claims 1 to 4, wherein X¹ and X² are -CH₂-.

**6.** The compound according to any one of claims 1 to 5, wherein A¹-OH or A²-NH-A³ is selected from a diagnostically acceptable dye, a therapeutically acceptable DNA-alkylating agent, a therapeutically acceptable tubulin-inhibiting agent, and a therapeutically acceptable DNA-intercalating agent.

**7.** The compound according to any one of claims 1 to 6, wherein A¹-OH or A²-NH-A³ is selected from 10-hydroxycamptothecin, 10-hydroxybelotecan, 10-hydroxygimatecan, 10-hydroxy-CKD-602, 10-hydroxy-BNP-1350, 10-hydroxy-sinotecan, topotecan, 7-ethyl-10-hydroxy-camptothecin (SN-38), 10-hydroxy-20-acetoxy-camptothecin, pyrrolobenzodiazepine, methotrexate, duocarmycin, CC-1065, doxorubicin, epirubicin, daunorubicin, pirarubicin, carminomycin, doxorubicin-*N*, *O*-acetal, 4-(bis(2-chloroethyl)amino)phenol, 4-(bis(2-bromoethyl)amino)phenol, 4-(bis(2-mesylethyl)amino)phenol, 4-((2-chloroethyl-2'-mesylethyl)amino)phenol, 5-hydroxy-seco-cyclopropabenzaindoles, 5-hydroxy-seco-(2-methyl-cyclopropa)benzaindoles, 5-hydroxy-seco-cyclopropamethoxybenzaindoles, 5-amino-seco-cyclopropabenzaindoles, etoposide, teniposide, GL331, NPF, TOP53, NK611, tubulysin A, tubulysin B, tubulysin C, tubulysin G, tubulysin I, monomethyl auristatin E, monomethyl auristatin F, dolastatin 10, dolastatin 15, symplostastin 1, symplostastin 3, narciclasine, pancratistatin, 2-epi-narciclasine, narciprimine, calicheamicin α1, calicheamicin β1, calicheamicin γ1, calicheamicin δ1, calicheamicin ε, calicheamicin θ, calicheamicin T, diclofenac, aceclofenac, mefenamic acid, clonixin, piroxicam, meloxicam, tenoxicam, lornoxicam, baricitinib, filgotinib, tofacitinib, upadacitinib, ruxolitinib, peficitinib, decemotinib, solcitinib, itacitinib, fostamatinib, SHR0302, leuco-methylene blue, leuco-methyl methylene blue, leuco-dimethyl methylene blue, leuco-toluidine blue, leuco-Azure A, leuco-Azure B, leuco-Azure C, leuco-Thionin, leuco-methylene violet, leuco-new methylene blue, leuco-Nile blue A, leuco-brilliant cresyl blue, firefly luciferin (D-Luciferin), umbelliferone, 4-trifluoromethylumbelliferone, 6,8-difluoro-4-methylumbelliferone, 7-hydroxycoumarin-3-carboxylic acid, 6,8-difluoro-7-hydroxy-5-methylcoumarin (DiFMU), 7-amino-4-methylcoumarin, 7-amino-4-chloromethylcoumarin, 3-*O*-methylfluorescein, 3-*O*-ethyl-5-carboxyfluorescein, 2,7-difluoro-3-*O*-methylfluorescein, 3-*N*-acetyl-rhodamine, 3-*N*-acetyl-dimethylsilarhodamine, 2,7-dibromo-3-*N*-acetyl-dimethylcarborhodamine, 3-*N*-acetyl-6-carboxyrhodamine, 2,7-difluoro-3-*N*-acetylrhodol, 3-*O*-(*N*,*N*-dimethyl-2-aminoethyl)-6-carboxyfluorescein, 2,7-dichloro-3-*O*-(*N*,*N*-dimethyl-2-aminoethyl)fluorescein, blackberry quencher (BBQ), black hole quencher 3 (BHQ3), 2-(2-hydroxyphenyl)quinazolin-4-one, 6-chloro-2-(5-chloro-2-hydroxyphenyl)quinazolin-4-one, and 6-bromo-2-(5-bromo-2-hydroxyphenyl)quinazolin-4-one.

**8.** A pharmaceutical or diagnostic composition comprising the compound according to any one of claims 1 to 7, or a pharmaceutically acceptable salt, solvate, or ester thereof, and optionally a pharmaceutically acceptable carrier or excipient.

**9.** The pharmaceutical or diagnostic composition according to claim 8, further comprising a second pharmaceutically active agent selected from a vascular disrupting agent, a cytotoxic chemotherapeutic agent and an immunomodulator.

**10.** The pharmaceutical or diagnostic composition according to claim 9, wherein the second pharmaceutically active agent is selected from combretastatin A-4 (CA4), 3'-aminocombretastatin A-4, BNC105, ABT-751, ZD6126, combretastatin A-1, or prodrugs of the same (which includes but is not limited to combretastatin A-4 phosphate (CA4P), 3'-aminocombretastatin A-4 3'-serinamide (ombrabulin), combretastatin A-1 bisphosphate (CA1P), and BNC105 phosphate (BNC105P)), and pharmaceutically acceptable salts, solvates or esters of the same.

**11.** A compound according to any one of claims 1 to 7, or a pharmaceutically acceptable salt, solvate, or ester thereof, for use in medicine.

**12.** A compound according to any one of claims 1 to 7, or a pharmaceutically acceptable salt, solvate, or ester thereof, for use in the treatment, amelioration, prevention or diagnosis of a disorder selected from a neoplastic disorder; atherosclerosis; an autoimmune disorder; an inflammatory disease; a chronic inflammatory autoimmune disease; ischaemia; and reperfusion injury.

**13.** The compound for use according to claim 12, wherein the neoplastic disorder is cancer which is preferably selected from acoustic neuroma, adenocarcinoma, angiosarcoma, basal cell carcinoma, bile duct carcinoma, bladder carcinoma, breast cancer, bronchogenic carcinoma, cervical cancer, chondrosarcoma, chordoma, choriocarcinoma, craniopharyngioma, cystadenocarcinoma, embryonal carcinoma, endotheliosarcoma, ependymoma, epithelial carcinoma, Ewing's tumor, fibrosarcoma, hemangioblastoma, leiomyosarcoma, liposarcoma, Merkel cell carcinoma, melanoma, mesothelioma, myelodysplastic syndrome, myxosarcoma, oligodendroglioma, osteogenic sarcoma, ovarian cancer, pancreatic cancer, papillary adenocarcinomas, papillary carcinoma, pinealoma, prostate cancer, renal cell carcinoma, retinoblastoma, rhabdomyosarcoma, sebaceous gland carcinoma, seminoma, squamous cell carcinoma, sweat gland carcinoma, synovioma, testicular tumor, Wilms' tumor, adrenocortical carcinoma, urothelial carcinoma, gallbladder cancer, parathyroid cancer, Kaposi sarcoma, colon carcinoma, gastrointestinal stromal tumor, anal cancer, rectal cancer, small intestine cancer, brain tumor, glioma, glioblastoma, astrocytoma, neuroblastoma, medullary carcinoma, medulloblastoma, meningioma, leukemias including acute myeloid leukemia, multiple myeloma, acute lymphoblastic leukemia, liver cancer including hepatoma and hepatocellular carcinoma, lung carcinoma, non-small-cell lung cancer, small cell lung carcinoma, lymphangioendotheliosarcoma, lymphangiosarcoma, primary CNS lymphoma, non-Hodgkin lymphoma, and classical Hodgkin's lymphoma, preferably colon cancer, rectal cancer, small intestine cancer, brain tumor, leukemia, liver cancer, lung cancer, lymphoma, basal cell carcinoma, breast cancer, cervical cancer, melanoma, ovarian cancer, pancreatic cancer, and squamous cell carcinoma.

**14.** Use of a compound according to any one of claims 1 to 7, or a pharmaceutically acceptable salt, solvate, or ester thereof, for the manufacture of a medicament for the treatment, amelioration, prevention or diagnosis of a disorder selected from a neoplastic disorder; atherosclerosis; an autoimmune disorder; an inflammatory disease; a chronic inflammatory autoimmune disease; ischaemia; and reperfusion injury.

**15.** Use of a compound according to claim 14, or a pharmaceutically acceptable salt, solvate, or ester thereof, wherein the neoplastic disorder is cancer which is preferably selected from acoustic neuroma, adenocarcinoma, angiosarcoma, basal cell carcinoma, bile duct carcinoma, bladder carcinoma, breast cancer, bronchogenic carcinoma, cervical cancer, chondrosarcoma, chordoma, choriocarcinoma, craniopharyngioma, cystadenocarcinoma, embryonal carcinoma, endotheliosarcoma, ependymoma, epithelial carcinoma, Ewing's tumor, fibrosarcoma, hemangioblastoma, leiomyosarcoma, liposarcoma, Merkel cell carcinoma, melanoma, mesothelioma, myelodysplastic syndrome, myxosarcoma, oligodendroglioma, osteogenic sarcoma, ovarian cancer, pancreatic cancer, papillary adenocarcinomas, papillary carcinoma, pinealoma, prostate cancer, renal cell carcinoma, retinoblastoma, rhabdomyosarcoma, sebaceous gland carcinoma, seminoma, squamous cell carcinoma, sweat gland carcinoma, synovioma, testicular tumor, Wilms' tumor, adrenocortical carcinoma, urothelial carcinoma, gallbladder cancer, parathyroid cancer, Kaposi sarcoma, colon carcinoma, gastrointestinal stromal tumor, anal cancer, rectal cancer, small intestine cancer, brain tumor, glioma, glioblastoma, astrocytoma, neuroblastoma, medullary carcinoma, medulloblastoma, meningioma, leukemias including acute myeloid leukemia, multiple myeloma, acute lymphoblastic leukemia, liver cancer including hepatoma and hepatocellular carcinoma, lung carcinoma, non-small-cell lung cancer, small cell lung carcinoma, lymphangioendotheliosarcoma, lymphangiosarcoma, primary CNS lymphoma, non-Hodgkin lymphoma, and classical Hodgkin's lymphoma, preferably colon cancer, rectal cancer, small intestine cancer, brain tumor, leukemia, liver cancer, lung cancer, lymphoma, basal cell carcinoma, breast cancer, cervical cancer, melanoma, ovarian cancer, pancreatic cancer, and squamous cell carcinoma.

**16.** A method of treating, ameliorating, preventing or diagnosing a disorder selected from a neoplastic disorder; atherosclerosis; an autoimmune disorder; an inflammatory disease; a chronic inflammatory autoimmune disease; ischaemia; and reperfusion injury, wherein an effective amount of a compound according to any one of claims 1 to 7, or a pharmaceutically acceptable salt, solvate, or ester thereof, is administered to a patient in need thereof.

**17.** A method according to claim 16, wherein the neoplastic disorder is cancer which is preferably is selected from acoustic neuroma, adenocarcinoma, angiosarcoma, basal cell carcinoma, bile duct carcinoma, bladder carcinoma, breast cancer, bronchogenic carcinoma, cervical cancer, chondrosarcoma, chordoma, choriocarcinoma, craniopharyngioma, cystadenocarcinoma, embryonal carcinoma, endotheliosarcoma, ependymoma, epithelial carcinoma, Ewing's tumor, fibrosarcoma, hemangioblastoma, leiomyosarcoma, liposarcoma, Merkel cell carcinoma, melanoma, mesothelioma, myelodysplastic syndrome, myxosarcoma, oligodendroglioma, osteogenic sarcoma, ovarian cancer, pancreatic cancer, papillary adenocarcinomas, papillary carcinoma, pinealoma, prostate cancer, renal cell carcinoma, retinoblastoma, rhabdomyosarcoma, sebaceous gland carcinoma, seminoma, squamous cell carcinoma, sweat gland carcinoma, synovioma, testicular tumor, Wilms' tumor, adrenocortical carcinoma, urothelial carcinoma, gallbladder cancer, parathyroid cancer, Kaposi sarcoma, colon carcinoma, gastrointestinal stromal tumor, anal cancer, rectal cancer, small intestine cancer, brain tumor, glioma, glioblastoma, astrocytoma, neuroblastoma, medullary carcinoma, medulloblastoma, meningioma, leukemias including acute myeloid leukemia, multiple myeloma, acute lymphoblastic leukemia, liver cancer including hepatoma and hepatocellular carcinoma, lung carcinoma, non-small-cell lung cancer, small cell lung carcinoma, lymphangioendotheliosarcoma, lymphangiosarcoma, primary CNS lymphoma, non-Hodgkin lymphoma, and classical Hodgkin's lymphoma, preferably colon cancer, rectal cancer, small intestine cancer, brain tumor, leukemia, liver cancer, lung cancer, lymphoma, basal cell carcinoma, breast cancer, cervical cancer, melanoma, ovarian cancer, pancreatic cancer, and squamous cell carcinoma.

A method of predicting the suitability of a compound having the formula (I) as defined in any one of claims 1 to 7, or a pharmaceutically acceptable salt, solvate, or ester thereof, for treating a patient who is suffering from a disorder selected from a neoplastic disorder; atherosclerosis; an autoimmune disorder; an inflammatory disease; a chronic inflammatory autoimmune disease; ischaemia; and reperfusion injury, wherein the method comprises:
obtaining a sample from the patient;
contacting the sample with a compound having the formula (I) as defined in any one of claims 1 to 7, or a pharmaceutically acceptable salt, solvate, or ester thereof, wherein A¹ is selected such that A¹-OH is a diagnostic or theranostic agent or A² and A³ are independently selected such that A²-NH-A³ is a diagnostic or theranostic agent; and
detecting the presence or absence of A¹-OH or A²-NH-A³.
